# EUROPEAN PATENT APPLICATION

(11) **EP 2 784 164 A1**
(43) Date of publication of application: **01.10.2014**
(21) Application number: 13460019.6
(22) Date of filing: 29.03.2013
(51) Int. Cl.: C12Q 1/68

(54) **A method for detecting a genetic predisposition to large intestine cancer (CRC)**

(71) Applicant: Centrum Badan DNA Sp. z o.o., 61-612 Poznan (PL)
(72) Inventor: Wojciechowicz, Tatiana, 60-682 Poznan (PL); Bociag, Piotr, 62-082 Kozieglowy (PL); Odwazna, Joanna, 64-000 Koscian (PL); Wróblewska-Kabba, Sylwia, 60-688 Poznan (PL); Lacna, Katarzyna, 60-681 Poznan (PL); Sikorski, Adam, 89-652 Lag (PL); Wojciechowicz, Jacek, 60-682 Poznan (PL)
(74) Representative: Kossowska, Janina

(57) **Abstract**

The present invention relates to a method for detecting genetic predisposition to colorectal cancer using DNA microarray by testing patient's biological sample towards occurance of mutations in genes MLH1, MSH2, MSH6, NOD2, CDK2A, CHEK2, CYP1B1.

The invention relates also to a kit for carrying out the method according to the invention and a set of primers for use in the amplification of genetic material by PCR method and a set of oligonucleotide sequences for use as probes for detecting genetic predisposition to colorectal cancer.

## Description

The present invention relates to a method for detecting genetic predisposition to colorectal cancer, a kit for use in this method and a set of primers for PCR amplification of genomic DNA obtained from a patient as well as to oligonucleotide sequences for use as probes.

Colorectal cancer is one of the most common neoplastic diseases in the world. The highest incidence is observed in developed countries and in developing countries the incidence keeps on increasing. It is the second most common among tumors: in women after breast cancer and in men after lung cancer (Jemal A. et al. CA Cancer J Clin 61 (2): 69-90, 2011). Data from the National Cancer Registry show that there are 14,000 new cases in Poland every year and number of deaths is close to 10,000 people each year (Wojciechowska U., Didkowska J., Zatoński W. Centrum Onkologii - Instytut, Biuletyn 2008). The high prevalence and high percentage of patients dying because of this reason is not only an important medical issue but also a social and economic problem. Taking into account epidemiological estimates it can be concluded that in the coming decades the number of cases and deaths from this type of cancer will continue to grow and the most vulnerable group are people over 65 years of age. Because of a long incubation period of malignant tumors, it is considered that they are primarily a group of diseases in the elderly (Didkowska J., Wojciechowska U., Zatoński W. Centrum Onkologii - Instytut, Warszawa 2009). In the Polish population there have been more and more cases of malignant tumors at a very young age, which often occur in conjunction with carrier state of mutations in genes that predispose to the disease (Panasiuk A., Krawczuk-Rybak M., Muszyńska-Ros 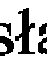 an K., Przegl 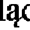 d Pediatryczny vol. 39, 2: 126-131, 2009).

It is estimated that about 30% of all cases result from high, genetically determined predisposition. The remaining 70% are the so-called sporadic tumors that result from environmental factors, which most often include harmful factors related to lifestyle, such as improper diet, fiber diet, with a low content of D and E vitamins as well as calcium and selenium ions and rich in red meat and fat. A group which is particularly vulnerable are people who often consume alcohol, those suffering from chronic constipation and/or diarrhea, overweight and who exercise too little (Gil et al., Wspó 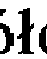 czesna Onkologia vol. 14, 3: 211-216, 2010).

Familial cases can be divided into two basic groups:
FAP - inherited in an autosomal dominant manner and it is a syndrome in which occurence of polyps throughout the colon is observed. In extreme cases, the entire intestine is covered with polyps. Polyps are not malignant, however if they are not treated, at least one or more of them transforms into an invasive cancer form. The gene which conditions the disease is an APC gene (Bulow S , Bjork J, Christensen IJ, Gut 53: 381-6, 2004).
HNPCC - it is also inherited in an autosomal dominant manner; it is characterized by occurence of a few foci, usually one or more, however transformation into a malignant tumor happens very quickly.

Hereditary colorectal cancer not associated with polyposis (hereditary non-polyposis colorectal cancer, HNPCC) described by Lynch (Lynch HT et al., Dis Colon Rectum 31:372-7, 1988) accounts for about 5% of all cases of colorectal cancer. Among newly diagnosed colorectal cancers annually, about 5,000 people in Poland may have their genetically determined form (Kladny J. et al., Postepy Nauk Medycznych 7: 456-459, 2008). In this group, genetic diagnosis is by far the most effective, locating mutations is successful in even more than half of the respondents depending on used techniques. At the moment, there are clear recommendations, both in Europe and the U.S., to conduct genetic tests on all patients with newly detected colorectal cancer (Evaluation of Genomic Applications in Practice and Prevention (EGAPP), Working Group Genetics In Medicine vol. 11, Nr 1, 2009).

Development of HNPCC is associated with presence of mutations in one or more genes such as MSH2, MLH1, MSH6. These genes are associated with repair of damaged DNA strands. Mutations in the first two of them are the most frequent causes of Lynch syndrome. Their participation in HNPCC is 50-60% and 30-40%, respectively (Vassen HF et al., J Med Genet 44 (6): 353-362, 2007).

Indications for the test are:
- early onset age (average of about 45 years of age),
- more frequent right location of the tumor,
- two and more cases of CRC among first degree relatives,
- multiple synchronous and metasynchronous CRC foci,
- occurence of disease in subsequent generations (vertical transmission),
- increased incidence of endometrial, small intestine and urinary tract cancers in relatives.

In 1991 the Amsterdam criteria were established (Vasen HF et al. Dis Colon Rectum 34(5): 424-5, 1991), according to which families with hereditary nonpolyposis colorectal cancer are characterized by the following features:
- colon and/or rectum cancer (histologically confirmed) occurred in at least three relatives wherein one of the patients should be first degree relative to at least two other patients;
- one case of colon and/or rectum cancer was diagnosed before the age of 50;
- cancers occured in at least two successive generations;
- familial adenomatous polyposis of the colon was excluded in patients (Familia Adenomatous Polyposis).

The criteria were expanded after adopting principles of recognition according to BE-THESDA (Rodriguez-Bigas MA, J Natl Cancer Inst 89(23): 1758-1762, 1997), according to which hereditary predisposition to colon cancer is present in:
- families meet Amsterdam criteria I of 1991;
- patients who were diagnosed with colorectal and/or rectum cancer with one first degree relative who had the same cancer or cancer of the spectrum of HNPCC/Lynch syndrome before age 45 or if a relative was diagnosed with adenomas in colon and/or rectum before age 40;
- patients with colon and/or rectum cancer or endometrial cancer was diagnosed before age 45;
- patients with cancer of the ascending colon and/or rectum diagnosed before age 45, histologically undifferentiated cancer or solid/cribriforme type of cancer, or with goblet cells;
- patients with colorectal adenomas diagnosed before age 40.

Despite the increase in incidence and mortality from cancer up to today fighting the disease is based primarily on alleviating its effects. In view of increasingly younger age of incidence of cancer, prevention and early diagnosis of cancer play an essential role. Early detection of cancer at an early stage of the disease determines a patient's life.

Due to growing advancement in knowledge on contribution of genetic mutations in conditioning of the disease and development of molecular biology techniques, it is possible to identify specific mutations, carrier-state of which significantly increases the risk of disease even at a very young age. Identification of carriers of mutations in genes that predispose to the disease allows implementing appropriate prevention at an early age, long before the onset of the disease, and thus it increases the chance of "winning" the fight against cancer.

Detection of marker mutations for HNPCC is very important clinically because it allows ruling out about 50% of family members from the high-risk group and it also facilitates taking the decision on radicalness of surgical procedures, e.g. colectomy, instead of a classical partial resection and prophylactic hysterectomy and ovariectomy, when these procedures are performed in patients diagnosed as mutation carriers.

Detection of mutations should lead to colonoscopy - the most effective method for detecting colorectal cancer. With appropriately early qualification for colonoscopy it is possible to identify colorectal cancer at its early stage. This is absolutely the most important element of improving the results of surgical treatment.

The test is very important for family members of persons with hereditary nonpolyposis colorectal cancer. This allows for, in the case of detection of mutations, precise definition of persons at risk of developing colorectal cancer and persons for whom this risk is not increased. Mandatory recommendation for relatives of people with genetically determined colon cancer is colonoscopy examination every two years.

Constitutional mutations in other DNA repair genes account for about 10% of detected changes in HNPCC. MSH6 gene together with MLH1 and MSH2 genes are involved in DNA repair process. Clinical picture of cancer in families with constitutional mutation in MSH6 gene is usually only close to HNPCC and does not meet clinical pedigree criteria, such as the Amsterdam criteria. The following was found for carriers of mutations in this gene:
1. increased risk of colon, endometrial and ovarian cancer, upper respiratory tract, stomach and breast cancer;
2. frequency of parenteral tumors increased in comparison with HNPCC;
3. later age at cancer diagnosis (mean age of onset about age 56 for colorectal cancer, about age 54 for endometrial cancer, about age 49 for ovarian cancer);
4. more frequent left-sided location of colorectal cancers.

Genetic test result does not show the current state of health. Determining the presence of a genetic mutation is not equivalent to the existence of a disease at a given time, it only shows an increased predisposition to the disease, a possibility of its development at a much earlier age, and what is related thereto, the need to implement appropriate prophylaxis. There is also a risk of transmission of a mutation to offspring. However, absence of a mutation is not equivalent to the fact that cancer is not developing or will never develop in such a subject. People who have been excluded as carriers are vulnerable to adverse effects of environmental factors and to a possibility of developing cancer at an older age.

Genetic test result is immutable, so there is no need to repeat it. Due to the fact that the examined changes are the result of inheritance from a parent, it does not matter at which moment of life the test is carried out. The test result does not indicate occurrence of a disease, it only indicates a possibility of its occurrence.

In connection with difficulties that may occur and in connection with often erroneous interpretation of a genetic test result it is indicated to visit a specialized genetic clinic with every genetic test result. Due to the fact that genetic tests are tests of a particular type and their results can have a huge impact on the personal life of patients and their families, only persons of legal age, after signing an informed consent for genetic studies can undergo a test for genetic predispositions to tumors.

Due to the high incidence of colorectal cancer (2. place among malignant tumors in Poland), there is a need to develop a reliable method for determining predisposition to colorectal cancer.

The present invention meets this need and provides a new method for detecting genetic predisposition to colorectal cancer.

The method according to the present invention consists in identifying 206 genetic mutations/polymorphic sites conditioning familial form of colorectal cancer in a sample taken from a subject. The test allows to diagnose genetic predisposition towards familial nonpolyposis colorectal cancer HNPCC. This is the first comprehensive genetic test of this kind which detects most of key mutations responsible for hereditary form of colorectal cancer and it is conducted using DNA microarray technology.

The DNA microarray technology is one of the most preferable methods developed in order to:
- analyse gene expression (detecting mRNA transcribed into more stable DNA);
- detect genetic material;
- analyse genomic DNA (so called genotyping).

A DNA microarray is a glass or plastic slide with appropriately prepared surface on which molecular probes were immobilized (usually these are chemically synthesized under laboratory conditions oligonucleotides complementary to a specific DNA fragment of the tested sample). The tested genetic material is attached to the probes specifically on the basis of complementarity (hybridization). Positions and fields that the probes occupy on a slide are defined in advance and they are in a matrix system (the fields form a regular arrangement of rows and columns). Each probe is specific for sequences of the analyzed genetic material for which it was designed in order to allow visualization (readout on a monitor connected to a microarray scanner) of signals with specific positions on the microarray, providing information on the analyzed changes in the analyzed genetic material. There are many types of microarrays offered by many manufacturers (including Affymetrix, Illumina, Roche Perlan, Bio, Genorama), which are related to differences in methodology, length of the probes, method of visualization of the signal, etc.

In the case of present invention the most preferable microarray platform turned out to be APEX (Arrayed Primer Extension) using microarray scanner Genorama QuattroImager (Berik J., Kurg A. Metspalu A., Estonian Bioct 2008: EP1980841). This platform is used only for genotyping - analysis of changes in genomic DNA (US Patent Application No. 09/741,960).

Analysis of genomic information from APEX microarray comprises the isolation of genetic material, amplification of selected regions of genomic DNA using designed primers, fragmentation of obtained amplification products and deactivation of unincorporated nucleotides, denaturation of obtained fragments to one strand form, hybridization of the fragmented amplification products with previously designed immobilized oligonucleotide probes and APEX reaction as well as visualization of the analyzed changes.

### Detailed description of the invention

The present invention relates to a method of carrying out a first complex genetic test to detect most of the key mutations responsible for hereditary colorectal cancer. This test is intended for patients with cancer, their families and members of families in which colorectal, endometrial, gastric or breast cancer occurs in close members of one family.

Inventors of the present invention have combined results obtained in previous genetic studies of colorectal cancer with a new DNA microarray technique, using a combination of primers and probes specific for selected locations in the human genome.

An advantage of this new method of the invention is the ability to simultaneously test more than 100 mutations. This method requires a relatively short time to conduct and is very economical. It would also open the possibility of introducing new mutations to the test, in case new mutations or SNPs (*Single Nucleotide Polymorphism*) having an impact on occurrence of colorectal cancer are disclosed.

The present invention relates to a method for detecting key mutations leading to of colorectal cancer and for determining predisposition to prostate cancer, comprising:
(a) obtaining a biological sample of genomic DNA from a patient;
(b) isolating genomic DNA from a sample obtained from a patient;
(c) amplification of regions containing the analyzed mutation sites and polymorphic sites;
(d) purifying and concentrating the amplification products;
(e) fragmentation and functional inactivation of unincorporated nucleotides;
(f) hybridization of fragmented DNA obtained from a patient with oligonucleotide probes immobilized at specific points on the slide designed according to the nucleotide sequence of MLH1, MSH2, MSH6, NOD2, CDK2A, CHEK2 and CYP1B1 genes for sense and antisense directions (SE and AS) of sequence flanking the site of the genetic change;
(g) adding, on the principle of complementarity, a single ddNTP to the end of each probe on the DNA array;
(h) excitation of a slide with at least four lengths of light successively and reading signals by means of a DNA microarray scanner.

In the present disclosure "obtaining a sample of genomic DNA from a patient" means taking a sample of peripheral blood or swab of inner cheek surface.

In some embodiments obtained samples are stored short term at 4°C, in other embodiments samples are stored long term at -20°C or -70°C. Repeated freezing and thawing degrades DNA.

Methods of extracting gDNA from a sample are preferably carried out using genomic DNA isolation kits NucleoSpin Dx Blood and NucleoSpin Tissue (Marcherey-Nagel, Germany).

Obtained gDNA serves as a template for amplification of 43 regions of 7 analyzed genes: MLH1, MSH2, MSH6, NOD2, CDK2A, CHEK2 and CYP1B1, including 206 analyzed mutation sites and polymorphic sites.

The term "mutation" as used herein means, in accordance with its generally accepted meaning, any genetic change such as substitution, insertion or deletion, which can lead to malignancies.

The term "amplification" means multiplication in vitro of genetic material comprising selected regions of genomic DNA.

According to the present invention, amplification is preferably conducted by means of Multiplex PCR method using pairs of oligonucleotide primers and amplification kit APEX Template Preparation (Genorama, Estonia) to amplify sequence fragments of interest. It is essential in this step to design appropriate primers, without which the amplification step would be impossible.

Compared with conventional PCR part of dTTP fraction in PCR reaction mixture is replaced with 20% dUTP. Incorporation of dUTP allows subsequent fragmentation of the material with uracil-N-glycosylase (UNG), which allows efficient hybridization of short fragments of a product to oligonucleotide probes because obtained PCR products have a length from 100 to 1000 bp, preventing effective hybridization.

As used herein, "purification and concentration" means that products are pooled, concentrated and purified, preferably using GeneJet PCR kit (Genorama, Estonia), which consists of GeneJet columns for purification of PCR products, binding buffer, washing buffer and elution buffer.

Fragmentation and functional inactivation of unincorporated nucleotides is preferably conducted in a single incubation step at 37°C using uracil-N-glycosylase (UNG) (Genorama, Estonia) and alkaline phosphatase (SAP or FastAP) (Genorama, Estonia). Uracil-N-glycosylase (UNG) recognizes uracil in dsDNA and ssDNA and hydrolyzes the bond between uracil and deoxyribose moiety. Samples are heated in order to inactivate unincorporated nucleotides and to conduct fragmentation of DNA in sites with uracil. Alkaline phosphatase inactivates nucleotides by removal of 5' phosphate moieties. Fragmentation of long PCR products is carried out in order to facilitate proper hybridization with oligonucleotides (probes) immobilized on a glass slide (APEX microarray).

Fragmentation is followed by denaturation of obtained fragments. Denaturation of DNA is separation of naturally occurring DNA double-strand at 95°C into a single strand form capable of hybridizing with complementary strand, including a nucleotide probe.

Oligonucleotides used as probes for microarrays are designed according to nucleotide sequence of genes: MLH1, MSH2, MSH6, NOD2, CDK2A, CHEK2 and CYP1B1 for sense and antisense directions (SE and AS) and comprise changes predisposing to colorectal cancer are shown in Table 1:

**Table 1**

| **No** | **GENE/LOCUS** | **Analyzed change** | **Position on microarray** | **Designation of primers and mutations** | | **Name of a gene sequence record from ENSEMBL database** |
|---|---|---|---|---|---|---|
| | | | | **SE** | **AS** | |
| 1 | MLH1/3p21.3 (OMIM) | c.-93G>A | 1.3 | PCR-MLH1sis1 MLH1_93G>A_SE CT | PCR-MLH1sis1 MLH1_-93G>A_AS GA | ENSG00000076242 |
| 2 | MLH1/3p21.3 (OMIM) | c.37delG | 1.5 | PCR-MLH1sis1 MLH1_37delG_SE GA | PCR-MLH1sis1 MLH1_37delG_AS_CG | ENSG00000076242 |
| 3 | MLH1/3p21.3 (OMIM) | c.65G>C | 1.7 | PCR-MLH1sis1 MLH_65G>C_SE GC | PCR-MLH1sis1 MLH_65G>C_AS_CG | ENSG00000076242 |
| 4 | MLH1/3p21.3 (OMIM) | c.66delG | 1.9 | PCR-MLH1sis1 MLHl_66delG_AS GA | PCR-MLH1sis1 MLH1_66delG_AS CG | ENSG00000076242 |
| 5 | MLH1/3p21.3 (OMIM) | c.69A>T | 1.11 | PCR-MLH1sis1 MLH1_69A>T_AS TA | PCR-MLH1sis1 MLH1_69A>T_AS TA | ENSG00000076242 |
| 6 | MLH1/3p21.3 (OMIM) | c.74T>C | 1.13 | PCR-MLH1sis1 MLH1_74T>C_SE TC | PCR-MLH1sis1 MLH1_74T>C_AS AG | ENSG00000076242 |
| 7 | MLH1/3p21.3 (OMIM) | c.83C>T | 1.15 | PCR-MLH1sis1 MLH1_830T_SE CT | PCR-MLH1sis1 MLH1_83C>T_AS GA | ENSG00000076242 |
| 8 | MLH1/3p21.3 (OMIM) | c.85G>T | 1.17 | PCR-MLH1sis1 MLH1_85G>T_SE GT | PCR-MLH1sis1 MLH1_85G>T_AS CA | ENSG00000076242 |
| 9 | MLH1/3p21.3 (OMIM) | c.104T>G | 1.19 | PCR-MLH1sis1 MLH1_104T>G_SE TG | PCR-MLH1sis1 MLH1_104T>G_AS AC | ENSG00000076242 |
| 10 | MLH1/3p21.3 (OMIM) | c.131C>T | 1.21 | PCR-MLH1sis2 MLH1_131C>T_SE CT | PCR-MLH1sis2 MLH1_131C>T_AS GA | ENSG00000076242 |
| 11 | MLH1/3p21.3 (OMIM) | c.137G>T | 1.23 | PCR-MLH1sis2 MLH1_137G>T_SE GT | PCR-MLH1sis2 MLH1_137G>T_AS CA | ENSG00000076242 |
| 12 | MLH1/3p21.3 (OMIM) | c.161G>A | 1.25 | PCR-MLH1sis2 MLH1_161G>A_SE GA | PCR-MLH1sis2 MLH1_161G>A_AS C- | ENSG00000076242 |
| 13 | MLH1/3p21.3 (OMIM) | c.161delG | 1.27 | PCR-MLH1sis2 MLH1_161delG_SE GA | PCR-MLH1sis2 MLH1_161delG_AS CT | ENSG00000076242 |
| 14 | MLH1/3p21.3 (OMIM) | c.184C>A | 1.29 | PCR-MLH1sis2 MLH1_184C>A/T_SE CAT | PCR-MLH1sis2 MLH1_184C>A/T_AS GTA | ENSG00000076242 |
| 15 | MLH1/3p21.3 (OMIM) | c.184C>T | | -II- | -II- | ENSG00000076242 |
| 16 | MLH1/3p21.3 (OMIM) | c.191A>G | 1.31 | PCR-MLH1sis2 MLH1_191A>G_SE AG | PCR-MLH1sis2 MLH1_191A>G_AS TC | ENSG00000076242 |
| 17 | MLH1/3p21.3 (OMIM) | c.194G>A | 1.33 | PCR-MLH1sis2 MLH1_194G>A_SE GA | PCR-MLH1sis2 MLH1_194G>A_AS CT | ENSG00000076242 |
| 18 | MLH1/3p21.3 (OMIM) | c.199G>A | 1.35 | PCR-MLH1sis2 MLH1_199G>A/T_SE GAT | PCR-MLH1sis2 MLH1_199G>A/T_AS CTA | ENSG00000076242 |
| 19 | MLH1/3p21.3 (OMIM) | c.199G>T | | -II- | -II- | ENSG00000076242 |
| 20 | MLH1/3p21.3 (OMIM) | c.200G>A | 1.37 | PCR-MLH1sis2 MLH1_200G>A_SE GA | PCR-MLH1sis2 MLH1_200G>A_AS CT | ENSG00000076242 |
| 21 | MLH1/3p21.3 (OMIM) | c.203T>A | 1.39 | PCR-MLH1sis2 MLH1_203T>A_SE TA | PCR-MLH1sis2 MLH1_203T>A_AS AT | ENSG00000076242 |
| 22 | MLH1/3p21.3 (OMIM) | c.206G>A | 1.41 | PCR-MLH1sis2 MLH1_206G>A_SE GA | PCR-MLH1sis2 MLH1_206G>A_AS CT | ENSG00000076242 |
| 23 | MLH1/3p21.3 (OMIM) | c.229T>C | 1.43 | PCR-MLH1sis3 MLH1_229T>C_SE TC | PCR-MLH1sis3 MLH1_229T>C_ASAG | ENSG00000076242 |
| 24 | MLH1/3p21.3 (OMIM) | c.230G>A | 1.45 | PCR-MLH1sis3 MLH1_230G>A_SE GA | PCR-MLH1sis3 MLH1_230G>A_AS CT | ENSG00000076242 |
| 25 | MLH1/3p21.3 (OMIM) | c.238T>G | 3.1 | PCR-MLH1sis3 MLH1_238T>G_SE TG | PCR-MLH1sis3 MLH1_238T>G_ASAC | ENSG00000076242 |
| 26 | MLH1/3p21.3 (OMIM) | c.250A>G | 3.3 | PCR-MLH1sis3 MLH1_250A>G_SE AG | PCR-MLH1sis3 MLH1_250A>G_AS TC | ENSG00000076242 |
| 27 | MLH1/3p21.3 (OMIM) | c.256C>T | 3.5 | PCR-MLH1sis3 MLH1_256C>T_SE CT | PCR-MLH1sis3 MLH1_256C>T_AS GA | ENSG00000076242 |
| 28 | MLH1/3p21.3 (OMIM) | c.277A>G | 3.7 | PCR-MLH1sis3 MLH1_277A>G_SE AG | PCR-MLH1sis3 MLH1_277A>G_AS TC | ENSG00000076242 |
| 29 | MLH1/3p21.3 (OMIM) | c.298C>T | 3.9 | PCR-MLH1sis3 MLH1_298C>T _SE CT | PCR-MLH1sis3 MLH1_298C>T_AS GA | ENSG00000076242 |
| 30 | MLH1/3p21.3 (OMIM) | c.299G>C | 3.11 | PCR-MLH1sis3 MLH1_299G>C_SE GC | PCR-MLH1sis3 MLH1_299G>C_AS CG | ENSG00000076242 |
| 31 | MLH1/3p21.3 (OMIM) | c.304G>A | 3.13 | PCR-MLH1sis3 MLH1_304G>A_SE GA | PCR-MLH1sis3 MLH1_304G>A_AS CT | ENSG00000076242 |
| 32 | MLHl/3p21.3 (OMIM) | c.306G>T | 3.15 | PCR-MLH1sis3 MLH1_306G>T_SE GT | PCR-MLH1sis3 MLH1_306G>T_AS CA | ENSG00000076242 |
| 33 | MLH1/3p21.3 (OMIM) | c.306+1G>A | 3.17 | PCR-MLH1sis3 MLH1_306+1G>A_SE GA | PCR-MLH1sis3 MLH1_306+1G>A_AS CT | ENSG00000076242 |
| 34 | MLH1/3p21.3 (OMIM) | c.320T>G | 3.19 | PCR-MLH1sis4 MLH1_320T>G_SE TG | PCR-MLH1sis4 MLH1_320T>G_AS AC | ENSG00000076242 |
| 35 | MLHl/3p21.3 (OMIM) | c.332C>T | 3.21 | PCR-MLH1sis4 MLH1_332C>T_SE CT | PCR-MLH1sis4 MLH1_332C>T_AS GA | ENSG00000076242 |
| 36 | MLH1/3p21.3 (OMIM) | c.350C>G | 3.23 | PCR-MLH1sis4 MLH1_350C>G/T_SE CGT | PCR-MLH1sis4 MLH1_350C>G/T_AS GCA | ENSG00000076242 |
| 37 | MLHl/3p21.3 (OMIM) | c.350C>T | | -II- | -II- | ENSG00000076242 |
| 38 | MLH1/3p21.3 (OMIM) | c.382G>C | 3.27 | PCR-MLH1sis5 MLH1_382G>C_SE GC | PCR-MLH1sis5 MLH1_382G>C_AS CG | ENSG00000076242 |
| 39 | MLH1/3p21.3 (OMIM) | c.392C>A | 3.29 | PCR-MLHlsis5 MLH1_392C>A_SE CA | PCR-MLH1sis5 MLH1_392C>A_AS GT | ENSG00000076242 |
| 40 | MLH1/3p21.3 (OMIM) | c.394G>C | 3.31 | PCR-MLH1sis5 MLH1_394G>C_SE GC | PCR-MLH1sis5 MLH1_394G>C_AS CG | ENSG00000076242 |
| 41 | MLH1/3p21.3 (OMIM) | c.453+79A>G | 3.33 | PCR-MLH1sis5 MLH1_453+79A>G_SE AG | PCR-MLH1sis5 MLHI-453+79A>G-AS TC | ENSG00000076242 |
| 42 | MLH1/3p21.3 (OMIM) | c.454-1G>A | 3.35 | PCR-MLH1sis6 MLH1_454-1G>A_SE GA | PCR-MLH1sis6 MLH1_454-1G>A_AS CT | ENSG00000076242 |
| 43 | MLH1/3p21.3 (OMIM) | c.464T>G | 3.37 | PCR-MLH1sis6 MLH1_464T>G_SE TG | PCR-MLHlsis6 MLH1_464T>G_AS AC | ENSG00000076242 |
| 44 | MLH1/3p21.3 (OMIM) | c.479C>T | 3.39 | PCR-MLH1sis6 MLH1_479C>T_SE CT | PCR-MLH1sis6 MLH1_479C>T_AS GA | ENSG00000076242 |
| 45 | MLH1/3p21.3 (OMIM) | c.544A>G | 3.41 | PCR-MLH1sis6 MLH1_544A>G_SE AG | PCR-MLH1sis6 MLH1_544A>G_AS TC | ENSG00000076242 |
| 46 | MLH1/3p21.3 (OMIM) | c.546-2A>G | 3.43 | PCR-MLH1sis7,8 MLH1_546-2A>G_SE AG | PCR-MLH1sis7,8 MLH1_546-2A>G_AS TC | ENSG00000076242 |
| 47 | MLH1/3p21.3 (OMIM) | c.577T>C | 3.47 | PCR-MLH1sis7,8 MLH1_577T>C_SE TC | PCR-MLH1sis7,8 MLH1_577T>C_AS AG | ENSG00000076242 |
| 48 | MLH1/3p21.3 (OMIM) | c.589-2A>G | 5.1 | PCR-MLH1sis7,8 MLH1_589-2A>G_SE AG | PCR-MLH1sis7,8 MLH1_589-2A>G_AS TC | ENSG00000076242 |
| 49 | MLH1/3p21.3 (OMIM) | c.595G>C | 5.3 | PCR-MLH1sis7,8 MLH1_595G>C_SE GC | PCR-MLH1sis7,8 MLH1_595G>C_AS CG | ENSG00000076242 |
| 50 | MLH1/3p21.3 (OMIM) | c.637G>A | 5.5 | PCR-MLH1sis7,8 MLH1_637G>A_SE GA | PCR-MLH1sis7,8 MLH1_637G>A_AS CT | ENSG00000076242 |
| 51 | MLH1/3p21.3 (OMIM) | c.649C>T | 5.7 | PCR-MLH1sis7,8 MLH1_649C>T_SE CT | PCR-MLH1sis7,8 MLH1_649C>T_AS GA | ENSG00000076242 |
| 52 | MLH1/3p21.3 (OMIM) | c.655A>C | 5.9 | PCR-MLH1sis7,8 MLH1_655A>C/G_SE ACG | PCR-MLH1sis7,8 MLH1_655A>C/G_AS TGC | ENSG00000076242 |
| 53 | MLH1/3p21.3 (OMIM) | c.655A>G | | -II- | -II- | ENSG00000076242 |
| 54 | MLH1/3p21.3 (OMIM) | c.676C>T | 5.11 | PCR-MLH1sis7,8 MLH1_676C>T_SE CT | PCR-MLH1sis7,8 MLH1_676C>T_AS GA | ENSG00000076242 |
| 55 | MLH1/3p21.3 (OMIM) | c.677G>A | 5.13 | PCR-MLH1sis7,8 MLH1_677G>A/T_SE GAT | PCR-MLH1sis7,8 MLH1_677G>A/T_AS CTA | ENSG00000076242 |
| 56 | MLH1/3p21.3 (OMIM) | c.677G>T | | -II- | -II- | ENSG00000076242 |
| 57 | MLH1/3p21.3 (OMIM) | c.677+3A>G | 5.15 | PCR-MLH1sis7,8 MLH1_677+3A>G_SE AG | PCR-MLH1sis7,8 MLH1_677+3A>G_AS TC | ENSG00000076242 |
| 58 | MLH1/3p21.3 (OMIM) | c.731G>A | 5.17 | PCR-MLH1sis9 MLH1_731G>A_SE GA | PCR-MLH1sis9 MLH1_731G>A_AS CT | ENSG00000076242 |
| 59 | MLH1/3p21.3 (OMIM) | c.739T>C | 5.19 | PCR-MLH1sis9 MLH1_739T>C_SE TC | PCR-MLH1sis9 MLH1_739T>C_AS AG | ENSG00000076242 |
| 60 | MLH1/3p21.3 (OMIM) | c.778C>T | 5.21 | PCR-MLH1sis9 MLH1_7780T_SE CT | PCR-MLH1sis9 MLH1_7780T_AS GA | ENSG00000076242 |
| 61 | MLH1/3p21.3 (OMIM) | c.790+1G>A | 5.23 | PCR-MLH1sis9 MLH1_790+1G>A_SE GA | PCR-MLH1sis9 MLH1_790+1G>A_AS CT | ENSG00000076242 |
| 62 | MLH1/3p21.3 (OMIM) | c.793C>T | 5.25 | PCR-MLH1sis10 MLH1_793C>T_SE CT | PCR-MLH1sis10 MLH1_793C>T_AS GA | ENSG00000076242 |
| 63 | MLH1/3p21.3 (OMIM) | c.794G>A | 5.27 | PCR-MLH1sis10 MLH1_794G>A_SE GA | PCR-MLH1sis10 MLH1_794G>A_AS CT | ENSG00000076242 |
| 64 | MLH1/3p21.3 (OMIM) | c.803A>G | 5.29 | PCR-MLH1sis10 MLH1_803A>G_SE AG | PCR-MLH1sis10 MLH1_803A>G_AS TC | ENSG00000076242 |
| 65 | MLH1/3p21.3 (OMIM) | c.814T>G | 5.31 | PCR-MLH1sis10 MLH1-814T>G_SE TG | PCR-MLH1sis10 MLH1_814T>G_AS AC | ENSG00000076242 |
| 66 | MLH1/3p21.3 (OMIM) | c.842C>T | 5.33 | PCR-MLH1sis10 MLH1_842C>T_SE CT | PCR-MLH1sis10 MLH1_842C>T_AS GA | ENSG00000076242 |
| 67 | MLH1/3p21.3 (OMIM) | c.875T>C | 5.35 | PCR-MLH1sis10 MLH1_875T>C_SE TC | PCR-MLH1sis10 MLH1_875T>C_AS AG | ENSG00000076242 |
| 68 | MLH1/3p21.3 (OMIM) | c.882C>T | 5.37 | PCR-MLLH1sis10 MLH1_8820T_SE CT | PCR-MLH1sis10 MLH1_8820T_AS GA | ENSG00000076242 |
| 69 | MLH1/3p21.3 (OMIM) | c.883-884delAGGT | 5.39 | PCR-MLH1sis10 MLH1_883delAGgt_SE GA | PCR-MLH1sis10 MLH1_883delAGgt_AS AG | ENSG00000076242 |
| 70 | MLH1/3p21.3 (OMIM) | c.883A>C | 5.41 | PCR-MLH1sis10 MLH1_883A>C_SE AC | PCR-MLH1sis10 MLH1_883A>C_AS TG | ENSG00000076242 |
| 71 | MLH1/3p21.3 (OMIM) | c.884-2A>C | 5.43 | PCR-MLH1sis11 MLH1_884-2A>C_SE AC | PCR-MLHsis11 TG(885) MLH1_884-2A>C_AS | ENSG00000076242 |
| 72 | MLH1/3p21.3 (OMIM) | c.1013A>G | 7.1 | PCR-MLH1sis1 MLH1_1013A>G_SE AG | PCR-MLHsis11 MLH1_1013A>G_AS TC | ENSG00000076242 |
| 73 | MLH1/3p21.3 (OMIM) | c.1038G>C | 7.3 | PCR-MLH1sis1 MLH1_1038G>C_SE GC | PCR-MLHsis11 MLH1_1038G>C_AS CG | ENSG00000076242 |
| 74 | MLH1/3p21.3 (OMIM) | c.1151T>A | 7.7 | PCR-MLH1sis12 MLH1_1151T>A_SE TA | PCR-MLH1sis12 MLH1_1151T>A_AS AT | ENSG00000076242 |
| 75 | MLH1/3p21.3 (OMIM) | c.1166G>A | 7.9 | PCR-MLH1sis12 MLH1_1166G>A_SE GA | PCR-MLH1sis12 MLHI_1 1166G>A_AS CT | ENSG00000076242 |
| 76 | MLH1/3p21.3 (OMIM) | c.1217G>A | 7.11 | PCR-MLH1sis12 MELH1_1217G>A_SE GA | PCR-MLH1sis12 MLH1_1217G>A_AS CT | ENSG00000076242 |
| 77 | MLH1/3p21.3 (OMIM) | c.1252delGA | 7.13 | PCR-MLH1sis12 MLH1_1252delGA_SE GT | PCR-MLH1sis12 MLH1_1252delGA_AS CG | ENSG00000076242 |
| 78 | MLH1/3p21.3 (OMIM) | c.1321G>A | 7.15 | PCR-MLH1sis12 MLH1_1321G>A_SE GA | PCR-MLH1sis12 MLH1_1321G>A_AS CT | ENSG00000076242 |
| 79 | MLH1/3p21.3 (OMIM) | c.1409+1G>C | 7.19 | PCR-MLH1sis12 MLH1_1409+1G>C_SE GC | PCR-MLH1sis12 MLH1_1409+1G>C_AS CG | ENSG00000076242 |
| 80 | MLH1/3p21.3 (OMIM) | c.1421G>A | 7.21 | PCR-MLH1sis13 MLH1_1421G>A_SE GA | PCR-MLH1sis13 MLH1_1421G>A_AS CT | ENSG00000076242 |
| 81 | MLH1/3p21.3 (OMIM) | c.1474G>A | 7.23 | PCR-MLH1sis13 MLH1_1474G>A_SE GA | PCR-MLH1sis13 MLH1_1474G>A_AS CT | ENSG00000076242 |
| 82 | MLH1/3p21.3 (OMIM) | C,1489dupC | 7.25 | PCR-MLH1sis13 MLH1_1489dupC_SE GC | PCR-MLH1sis13 MLH1_1489dupC_AS TG | ENSG00000076242 |
| 83 | MLH1/3p21.3 (OMIM) | c.1490insC | 7.27 | PCR-MLH1sis13 MLH1_1490insC_SE GC | PCR-MLH1sis13 MLH1_1490msC_AS CG | ENSG00000076242 |
| 84 | MLH1/3p21.3 (OMIM) | c.1517T>C | 7.29 | PCR-MLH1sis13 MLH1_1517T>C_SE T- | PCR-MLH1sis13 MLH1_1517T>C_AS AG | ENSG00000076242 |
| 85 | MLH1/3p21.3 (OMIM) | c.1528C>T | 7.31 | PCR-MLH1sis13 MLH1_1528C>T_SE CT | PCR-MLH1sis13 MLH1_1528C>T_AS GA | ENSG00000076242 |
| 86 | MLH1/3p21.3 (OMIM) | c.1558+14G>A | 7.33 | PCR-MLH1sis13 MLH1_1558+14G>A_SE GA | PCR-MLH1sis13 MLH1_1558+14G>A_AS CT | ENSG00000076242 |
| 87 | MLH1/3p21.3 (OMIM) | c.1569G>T | 7.35 | PCR-MLH1sis14 MLH1_1569G>T_SE GT | PCR-MLH1sis14 MLH1_1569G>T_AS CA | ENSG00000076242 |
| 88 | MLH1/3p21.3 (OMIM) | c.1625A>T | 7.37 | PCR-MLH1sis14 MLH1_ 1625A>T_SE AT | PCR-MLH1sis14 MLH1_ 1625A>T-AS TA | ENSG00000076242 |
| 89 | MLH1/3p21.3 (OMIM) | c.1646T>C | 7.39 | PCR-MLH1sis14 MLH1_1646T>C_SE TC | PCR-MLH1sis14 MLH1_1646T>C_AS AG | ENSG00000076242 |
| 90 | MLH1/3p21.3 (OMIM) | c.1649T>C | 7.41 | PCR-MLH1sis14 MLH1_1649T>C_SE TC | PCR-MLH1sis14 MLH1_1649T>C_AS AG | ENSG00000076242 |
| 91 | MLH1/3p21.3 (OMIM) | c.1652A>C | 7.43 | PCR-MLH1sis14 MLH1_1652A>C_SE AC | PCR-MLH1sis14 MLH1_1652A>C_AS TG | ENSG00000076242 |
| 92 | MLHl/3p21.3 (OMIM) | c.1658de1CCA | 7.45 | PCR-MLH1sis14 MLH1_1658de1CCA_SE CA | PCR-MLH1sis14 MLH1_1658delCCA_AS G- | ENSG00000076242 |
| 93 | MLH1/3p21.3 (OMIM) | c.1668-19A>G | 7.47 | PCR-MLH1sis15 MLH1_1668-19A>G_SE AG | PCR-MLH1sis15 MLH1_1668-19A>G_AS TC | ENSG00000076242 |
| 94 | MLH1/3p21.3 (OMIM) | c.1672G>T | 9.1 | PCR-MLH1sis15 MLH1_1672G>T_SE GT | PCR-MLH1sis15 MLH1_1672G>T_AS CA | ENSG00000076242 |
| 95 | MLHl/3p21.3 (OMIM) | c.1693A>T | 9.3 | PCR-MLH1sis15 MLH1_1693A>T_SE AT | PCR-MLH1sis15 MLH1_1693A>T_AS TA | ENSG00000076242 |
| 96 | MLHl/3p21.3 (OMIM) | c.1721T>C | 9.5 | PCR-MLH1sis15 MLH1_1721T>C_SE TC | PCR-MLH1sis15 MLH1_1721T>C_AS AG | ENSG00000076242 |
| 97 | MLH1/3p21.3 (OMIM) | c.17310>A | 9.7 | PCR-MLH1sis15 MLH1_1731G>A_SE GA | PCR-MLH1sis15 MLH1_1731G>A_AS CT | ENSG00000076242 |
| 98 | MLH1/3p21.3 (OMIM) | c.1733A>G | 9.9 | PCR-MM1sis16 MLH1_1733A>G_SE AG | PCR-MLH1sis16 MLH1_1733A>G_AS TC | ENSG00000076242 |
| 99 | MLH1/3p21.3 (OMIM) | c.1744C>G | 9.11 | PCR-MLH1sis16 MLH1_1744C>G_SE CG | PCR-MLH1sis16 MLH1_17440G_AS GC | ENSG00000076242 |
| 100 | MLH1/3p21.3 (OMIM) | c.1756G>C | 9.13 | PCR-MM1sis16 MLH1_1756G>C_SE GC | PCR-MLH1sis16 MLH1_1756G>C_AS CG | ENSG00000076242 |
| 101 | MLH1/3p21.3 (OMIM) | c.1766C>A | 9.15 | PCR-MLH1sis16 MLH1_17660A_SE CA | PCR-MLH1sis16 MLH1_17660A_AS GT | ENSG00000076242 |
| 102 | MLH1/3p21.3 (OMIM) | c.1783delAG | 9.17 | PCR-MLH1sis16 MLH1_1783delAG_SE AT | PCR-MLH1sis16 MLH1_1783delAG_AS CG | ENSG00000076242 |
| 103 | MLH1/3p21.3 (OMIM) | c.1808C>G | 9.19 | PCR-MLH1sis16 MLH1_1808C>G_SE CG | PCR-MLH1sis16 MLH1_18080G_AS GC | ENSG00000076242 |
| 104 | MLH1/3p21.3 (OMIM) | c.1820T>A | 9.21 | PCR-MLH1sis16 MLH1_1820T>A_SE TA | PCR-MLH1sis16 MLH1_1820T>A_AS AT | ENSG00000076242 |
| 105 | MLH1/3p21.3 (OMIM) | c.1846delAAG | 9.23 | PCR-MLH1sis16 MLH1_1846delAAG_SE AG | PCR-MLH1sis16 MLH1_1846delAAG_AS TA | ENSG00000076242 |
| 106 | MLH1/3p21.3 (OMIM) | c.1852_1853del AAinsGC | 9.25 | PCR-MLH1sis16 MLH1 1852 1853delAAinsGC SE AG | PCR-MLH1sis16 MLH1_1852 1853delAAinsGC_AS_TG | ENSG00000076242 |
| 107 | MLH1/3p21.3 (OMIM) | c.1852delAAG | 9.27 | PCR-MM1sis16 MLH1_1852delAAG_SEAG | PCR-MLH1sis16 MLH1_1852delAAG_AS TC | ENSG00000076242 |
| 108 | MLH1/3p21.3 (OMIM) | c.1853A>C | 9.29 | PCR-MM1sis16 MLH1_1853A>C/G_SE ACG | PCR-MLH1sis16 MLH1_1853A>C/G_AS TGC | ENSG00000076242 |
| 109 | MLH1/3p21.3 (OMIM) | c.1853A>G | | -II- | -II- | ENSG00000076242 |
| 110 | MLH1/3p21.3 (OMIM) | c.1865T>A | 9.31 | PCR-MM1sis16 MLH1_1865T>A_SE TA | PCR-MLH1sis16 MLH1_1865T>A_AS AT | ENSG00000076242 |
| 111 | MLH1/3p21.3 (OMIM) | c.1918C>T | 9.33 | PCR-MLH1sis17 MLH1_1918C>T_SE CT | PCR-MLH1sis17 MLH1_19l8C>T_AS GA | ENSG00000076242 |
| 112 | MLH1/3p21.3 (OMIM) | c.1937A>G | 9.35 | PCR-MLH1sis17 MLH1_1937A>G_SE AG | PCR-MLH1sis17 MLH1_1937A>G_AS TC | ENSG00000076242 |
| 113 | MLH1/3p21.3 (OMIM) | c.1942C>T | 9.37 | PCR-MLH1sis17 MLH1_19420T_SE CT | PCR-MLH1sis17 MLH1_1942C>T_AS GA | ENSG00000076242 |
| 114 | MLH1/3p21.3 (OMIM) | c.1943C>T | 9.39 | PCR-MLH1sis17 MLH1_1943C>T_SE CT | PCR-MLH1sis17 MLH1_1943C>T_AS GA | ENSG00000076242 |
| 115 | MLH1/3p21.3 (OMIM) | c.1958T>G | 9.41 | PCR-MLH1sis17 MLH1_1958T>G_SE TG | PCR-MLH1sis17 MLH1_1958T>G_AS AC | ENSG00000076242 |
| 116 | MLH1/3p21.3 (OMIM) | c.1959G>T | 9.43 | PCR-MLH1sis17 MLH1_1959G>T_SE GT | PCR-MLH1sis17 MLH1_1959G>T_AS CA | ENSG00000076242 |
| 117 | MLH1/3p21.3 (OMIM) | c.1961C>T | 9.45 | PCR-MLH1sis17 MLH1_1961C>T_SE CT | PCR-MLH1sis17 MLH1_1961C>T_AS GA | ENSG00000076242 |
| 118 | MLH1/3p21.3 (OMIM) | c.1963A>G | 9.47 | PCR-MLH1sis17 MLH1_1963A>G_SE AG | PCR-MLH1sis17 MLH1_1963A>G_AS TC | ENSG00000076242 |
| 119 | MLH1/3p21.3 (OMIM) | c.1964T>C | 11.1 | PCR-MLH1sis17 MLH1_1964T>C_SE TC | PCR-MLH1sis17 MLH1_1964T>C_AS AG | ENSG00000076242 |
| 120 | MLH1/3p21.3 (OMIM) | c.1976G>A | 11.3 | PCR-MLH1sis17 MLH1_1976G>A/C_SE GAC | PCR-MLH1sis17 MLH1_1976G>A/C_AS CTG | ENSG00000076242 |
| 121 | MLH1/3p21.3 (OMIM) | c.1976G>C | | -II- | -II- | ENSG00000076242 |
| 122 | MLH1/3p21.3 (OMIM) | c.1984A>C | 11.5 | PCR-MLH1sis17 MLH1_1984A>C_SE AC | PCR-MLH1sis17 MLH1_1984A>C_AS TG | ENSG00000076242 |
| 123 | MLH1/3p21.3 (OMIM) | c.1989G>T | 11.7 | PCR-MLH1sis17 MLH1_1989G>T_SE GT | PCR-MLH1sis17 MLH1_1989G>T_AS CA | ENSG00000076242 |
| 124 | MLH1/3p21.3 (OMIM) | c.2027T>G | 11.9 | PCR-MLH1sis18 MLH1_2027T>G_SE TG | PCR-MLH1sis18 MLH1_2027T>G_AS AC | ENSG00000076242 |
| 125 | MLHl/3p21.3 (OMIM) | c.2040C>A | 11.11 | PCR-MLH1sis18 MLH1_2040C>A_SE CA | PCR-MLH1sis18 MLH1_2040C>A_AS GT | ENSG00000076242 |
| 126 | MLH1/3p21.3 (OMIM) | c.2041G>A | 11.13 | PCR-MLH1sis18 MLH1_2041G>A_SE GA | PCR-MLH1sis18 MLH1_2041G>A_AS CT | ENSG00000076242 |
| 127 | MLH1/3p21.3 (OMIM) | c.2059C>T | 11.15 | PCR-MLH1sis18 MLH1_2059C>T_SE CT | PCR-MLH1sis18 MLH1_2059C>T_AS GA | ENSG00000076242 |
| 128 | MLH1/3p21.3 (OMIM) | c.2066A>G | 11.17 | PCR-MLH1sis18 MLH1_2066A>G_SE AG | PCR-MLH1sis18 MLH1_2066A>G_AS TC | ENSG00000076242 |
| 129 | MLH1/3p21.3 (OMIM) | c.2103G>C | 11.19 | PCR-MLH1sis18 MLH1_2103G>C_SE GC | PCR-MLH1sis18 MLH1_2103G<C_AS CG | ENSG00000076242 |
| 130 | MLH1/3p21.3 (OMIM) | c.2103+1G>A | 11.21 | PCR-MLH1sis18 MLH1_2103+1G>A_SE GA | PCR-MLH1sis18 MLH1_2103+1G>A_AS CT | ENSG00000076242 |
| 131 | MLH1/3p21.3 (OMIM) | c.2146G>A | 11.23 | PCR-MLH1sis19 MLH1_2146G>A_SE GA | PCR-MLH1sis19 MLH1_2146G>A_AS CT | ENSG00000076242 |
| 132 | MLH1/3p21.3 (OMIM) | c.2152C>T | 11.25 | PCR-MLH1sis19 MLH1_2152C>T_SE CT | PCR-MLH1sis19 MLH1_2152C>T_AS GA | ENSG00000076242 |
| 133 | MLH1/3p21.3 (OMIM) | c.2223del11 | 11.27 | PCR-MLH1sis19 MLH1_2223del11_SE GA | PCR-MLH1sis19 MLH1_2223del11_AS TA | ENSG00000076242 |
| 134 | MLH1/3p21.3 (OMIM) | c.2252A>G | 11.29 | PCR-MLH1sis19 MLH1_2252A>G_SE AG | PCR-MLH1sis19 MLH1_2252A>G_AS TC | ENSG00000076242 |
| 135 | MSH2/1p22-p21 (OMIM) | c.-118T>C | 11.31 | PCR-MSH2sis1 MSH2_-118T>C_SE TC | PCR-MSH2sis1 MSH2_-118T>C_AS AG | ENSG00000076242 |
| 136 | MSH2/1p22-p21 (OMIM) | c.4G>A | 11.33 | PCR-MSH2sis1 MSH2_4G>A_SE GA | PCR-MSH2sis1 MSH2_4G>A_AS CT | ENST00000233146 |
| 137 | MSH2/1p22-p21 (OMIM) | c.226C>T | 11.35 | PCR-MSH2sis2 MSH2_226C>T_SE CT | PCR-MSH2sis2 MSH2_226C>T_AS GA | ENST00000233146 |
| 138 | MSH2/1p22-p21 (OMIM) | c.339G>A | 11.37 | PCR-MSH2sis2 MSH2_339G>A_SE GA | PCR-MSH2sis2 MSH2_339G>A_AS CT | ENST00000233146 |
| 139 | MSH2/1p22-p21 (OMIM) | c.380A>G | 11.39 | PCR-MSH2sis3 MSH2_380A>G_SE AG | PCR-MSH2sis3 MSH2_380A>G_AS TC | ENST00000233146 |
| 140 | MSH2/1p22-p21 (OMIM) | c.435T>G | 11.41 | PCR-MSH2sis3 MSH2_435T>G_SE TG | PCR-MSH2sis3 MSH2_435T>G_AS AC | ENST00000233146 |
| 141 | MSH2/1p22-p21 (OMIM) | c.499G>C | 11.45 | PCR-MSH2sis3 MSH2_499G>C_SE GC | PCR-MSH2sis3 MSH2_499G>C_AS CG | ENST00000233146 |
| 142 | MSH2/1p22-p21 (OMIM) | c.505A>G | 11.47 | PCR-MSH2sis3 MSH2_505A>G_SE AG | PCR-MSH2sis3 MSH2_505A>G_AS TC | ENST00000233146 |
| 143 | MSH2/1p22-p21 (OMIM) | c.518T>C | 13.1 | PCR-MSH2sis3 MSH2_518T>C_SE TC | PCR-MSH2sis3 MSH2_518T>C_AS AG | ENST00000233146 |
| 144 | MSH2/1p22-p21 (OMIM) | c.560T>C | 13.3 | PCR-MSH2sis3 MSH2_560T>C_SE TC | PCR-MSH2sis3 MSH2_560T>C_AS AG | ENST00000233146 |
| 145 | MSH2/1p22-p21 (OMIM) | c.593A>G | 13.5 | PCR-MSH2sis3 MSH2_593A>G_SE AG | PCR-MSH2sis3 MSH2_593A>G_AS TC | ENST00000233146 |
| 146 | MSH2/1p22-p21 (OMIM) | c.595T>C | 13.7 | PCR-MSH2sis3 MSH2_595T>C_SE TC | PCR-MSH2sis3 MSH2_595T>C_AS AG | ENST00000233146 |
| 147 | MSH2/1p22-p21 (OMIM) | c.687delA | 13.9 | PCR-MSH2sis4 MSH2_687delA_SE AG | PCR-MSH2sis4 MSH2_687delA_AS TC | ENST00000233146 |
| 148 | MSH2/1p22-p21 (OMIM) | c.806C>T | 13.11 | PCR-MSH2sis5 MSH2_806C>T_SE CT | PCR-MSH2sis5 MSH2_806C>T_AS GA | ENST00000233146 |
| 149 | MSH2/1p22-p21 (OMIM) | c.862C>T | 13.13 | PCR-MSH2sis5 MSH2_862C>T_SE CT | PCR-MSH2sis5 MSH2_862C>T_AS GA | ENST00000233146 |
| 150 | MSH2/1p22-p21 (OMIM) | c.892C>T | 13.15 | PCR-MSH2sis5 MSH2_892C>T_SE CT | PCR-MSH2sis5 MSH2_892C>T_AS GA | ENST00000233146 |
| 151 | MSH2/1p22-p21 (OMIM) | c.942+3A>T | 13.17 | PCR-MSH2sis5 MSH2_942+3A>T_SE AT | Ignore | ENST00000233146 |
| 152 | MSH2/1p22-p21 (OMIM) | c.965G>A | 13.19 | PCR-MSH2sis6 MSH2_965G>A_SE GA | PCR-MSH2sis6 MSH2_965G>A_AS CT | ENST00000233146 |
| 153 | MSH2/1p22-p21 (OMIM) | c.984C>T | 13.21 | PCR-MSH2sis6 MSH2_984C>T_SE CT | PCR-MSH2sis6 MSH2_984C>T_AS GA | ENST00000233146 |
| 154 | MSH2/1p22-p21 (OMIM) | c.998G>A | 13.23 | PCR-MSH2sis6 MSH2_998G>A_SE GA | PCR-MSH2sis6 MSH2_998G>A_AS CT | ENST00000233146 |
| 155 | MSH2/1p22-p21 (OMIM) | c.1077-10T>C | 13.27 | PCR-MSH2sis7 MSH2_1077-10T>C_SE TC | PCR-MSH2sis7 MSH2_1077-10T>C_AS AG | ENST00000233146 |
| 156 | MSH2/1p22-p21 (OMIM) | c.1077A>T | 13.29 | PCR-MSH2sis7 MSH2_1077A>T_SE AT | PCR-MSH2sis7 MSH2_1077A>T_AS TA | ENST00000233146 |
| 157 | MSH2/1p22 p21 (OMIM) | c.1147C>T | 13.31 | PCR-MSH2sis7 MSH2_1147C>T_SE CT | PCR-MSH2sis7 MSH2_1147C>T_AS GA | ENST00000233146 |
| 158 | MSH2/1p22-p21 (OMIM) | c.1165C>T | 13.33 | PCR-MSH2sis7 MSH2_1165C>T_SE CT | PCR-MSH2sis7 MSH2_1165C>T_AS GA | ENST00000233146 |
| 159 | MSH2/1p22-p21 (OMIM) | c.1168C>T | 13.35 | PCR-MSH2sis7 MSH2_1168C>T_SE CT | PCR-MSH2sis7 MSH2_1168C>T_AS GA | ENST00000233146 |
| 160 | MSH2/1p22-p21 (OMIM) | c.1216C>T | 13.37 | PCR-MSH2sis7 MSH2_1216C>T_SE CT | PCR-MSH2sis7 MSH2_1216C>T_AS GA | ENST00000233146 |
| 161 | MSH2/1p22-p21 (OMIM) | c.1226delAG | 13.39 | PCR-MSH2sis7 MSH2_1226delAG_SE AG | PCR-MSH2sis7 MSH2_1226delAG_AS CG | ENST00000233146 |
| 162 | MSH2/1p22-p21 (OMIM) | c.1255C>A | 13.41 | PCR-MSH2sis7 MSH2_255C>A_SE CA | PCR-MSH2sis7 MSH2_1255C>A_AS GT | ENST00000233146 |
| 163 | MSH2/1p22-p21 (OMIM) | c.1373T>G | 13.43 | PCR-MSH2sis8 MSH2_1373T>G_SE TG | PCR-MSH2sis8 MSH2_1373T>G_AS AC | ENST00000233146 |
| 164 | MSH2/1p22-p21 (OMIM) | c.1571G>C | 13.47 | PCR-MSH2sis10 MSH2_1571G>C_SE GC | PCR-MSH2sis10 MSH2_1571G>C_AS CG | ENST00000233146 |
| 165 | MSH2/1p22-p21 (OMIM) | c.1654A>C | 15.1 | PCR-MSH2sis10 MSH2_1654A>C_SE AC | PCR-MSH2sis10 MSH2_1654A>C_AS TG | ENST00000233146 |
| 166 | MSH2/1p22-p21 (OMIM) | c.1661+12G>A | 15.3 | PCR-MSH2sis10 MSH2_1661+12G>A_SE GA | PCR-MSH2sis10 MSH2_1661+12G>A_AS CT | ENST00000233146 |
| 167 | MSH2/1p22-p21 (OMIM) | c.1666T>C | 15.5 | PCR-MSH2sis11 MSH2_1666T>C_SE TC | PCR-MSH2sis11 MSH2_1666T>C_AS AG | ENST00000233146 |
| 168 | MSH2/1p22-p21 (OMIM) | c.1737A>G | 15.7 | PCR-MSH2sis11 MSH2_1737A>G_SE AG | PCR-MSH2sis11 MSH2_1737A>G_AS TC | ENST00000233146 |
| 169 | MSH2/1p22-p21 (OMIM) | c.1738G>T | 15.9 | PCR-MSH2sis11 MSH2_1738G>T_SE GT | PCR-MSH2sis11 MSH2_1738G>T_AS CA | ENST00000233146 |
| 170 | MSH2/1p22-p21 (OMIM) | c.1786delAAT | 15.11 | PCR-MSH2sis12 MSH2_1786delAAT_SE AG | PCR-MSH2sis12 MSH2_1786delAAT_AS AG | ENST00000233146 |
| 171 | MSH2/1p22-p21 (OMIM) | c.1787A>G | 15.13 | PCR-MSH2sis12 MSH2_1787A>G_SE AG | PCR-MSH2sis12 MSH2_1787A>G_AS TC | ENST00000233146 |
| 172 | MSH2/1p22-p21 (OMIM) | c.1799C>T | 15.15 | PCR-MSH2sis12 MSH2_1799C>T_SE CT | PCR-MSH2sis12 MSH2_1799C>T_AS GA | ENST00000233146 |
| 173 | MSH2/1p22-p21 (OMIM) | c.1807G>A | 15.17 | PCR-MSH2sis12 MSH2_1807G>A_SE A- | PCR-MSH2sis12 MSH2_1807G>A_AS CT | ENST00000233146 |
| 174 | MSH2/1p22-p21 (OMIM) | c.1861C>T | 15.19 | PCR-MSH2sis12 MSH2_1861C>T_SE CT | PCR-MSH2sis12 MSH2_1861C>T_AS GA | ENST00000233146 |
| 175 | MSH2/1p22-p21 (OMIM) | c.1865C>T | 15.21 | PCR-MSH2sis12 MSH2_1865C>T_SE CT | PCR-MSH2sis12 MSH2_1865C>T_AS GA | ENST00000233146 |
| 176 | MSH2/1p22-p21 (OMIM) | c.1906G>C | 15.23 | PCR-MSH2sis12 MSH2_1906G>C_SE GC | PCR-MSH2sis12 MSH2_1906G>C_AS CG | ENST00000233146 |
| 177 | MSH2/1p22-p21 (OMIM) | c.2006-6T>C | 15.25 | PCR-MSH2sis13 MSH2_2006-6T>C_SE TC | PCR-MSH2sis13 MSH2_2006-6T>C_AS AG | ENST00000233146 |
| 178 | MSH2/1p22-p21 (OMIM) | c.2064G>A | 15.29 | PCR-MSH2sis13 MSH2_2064G>A_SE GA | PCR-MSH2sis13 MSH2_2064G>A_AS CT | ENST00000233146 |
| 179 | MSH2/1p22-p21 (OMIM) | c.2089T>C | 15.31 | PCR-MSH2sis13 MSH2_2089T>C_SE TC | PCR-MSH2sis13 MSH2_2089T>C_AS AG | ENST00000233146 |
| 180 | MSH2/1p22-p21 (OMIM) | c.2090G>T | 15.33 | PCR-MSH2sis13 MSH2_2090G>T_SE GT | PCR-MSH2sis13 MSH2_2090G>T_AS CA | ENST00000233146 |
| 181 | MSH2/1p22-p21 (OMIM) | c.2131C>T | 15.35 | PCR-MSH2sis13 MSH2_2131C>T_SE CT | PCR-MSH2sis13 MSH2_2131C>T_AS GA | ENST00000233146 |
| 182 | MSH2/1p22-p21 (OMIM) | c.2168C>T | 15.37 | PCR-MSH2sis13 MSH2_2168C>T_SE CT | PCR-MSH2sis13 MSH2_2168C>T_AS GA | ENST00000233146 |
| 183 | MSH2/1p22-p21 (OMIM) | c.2245G>A | 15.39 | PCR-MSH2sis14 MSH2_2245G>A_SE GA | PCR-MSH2sis14 MSH2_2245G>A_AS CT | ENST00000233146 |
| 184 | MSH2/1p22-p21 (OMIM) | c.2251G>A | 15.41 | PCR-MSH2sis14 MSH2_2251G>A_SE GA | PCR-MSH2sis14 MSH2_2251G>A_AS CT | ENST00000233146 |
| 185 | MSH2/1p22-p21 (OMIM) | c.2500G>A | 15.43 | PCR-MSH2sis15 MSH2_2500G>A_SE GA | PCR-MSH2sis15 MSH2_2500G>A_AS CT | ENST00000233146 |
| 186 | MSH2/1p22-p21 (OMIM) | c.2633delAG | 15.45 | PCR-MSH2sis15 MSH2_2633delAG_SE AG | PCR-MSH2sis15 MSH2_2633delAG_AS CT | ENST00000233146 |
| 187 | MSH2/1p22-p21 (OMIM) | c.2714C>G | 15.47 | PCR-MSH2sis16 MSH2_2714C>G_SE CG | PCR-MSH2sis16 MSH2_2714C>G_AS GC | ENST00000233146 |
| 188 | MSH6/2p16 (OMIM) | c.276A>G | 17.5 | PCR-MSH6sis2 MSH6_276A>G_SE AG | PCR-MSH6sis2 MSH6_276A>G_AS TC | ENST00000234420 |
| 189 | MSH6/2p16 (OMIM) | c.431G>T | 17.7 | PCR-MSH6sis2 MSH6_431G>T_SE GT | PCR-MSH6sis2 MSH6_431G>T_AS CA | ENST00000234420 |
| 190 | MSH6/2p16 (OMIM) | c.457+52T>A | 17.9 | PCR-MSH6sis2 MSH6_457+52T>A_SE_TA | PCR-MSH6sis2 MSH6_457+52T>A_AS AT | ENST00000234420 |
| 191 | MSH6/2p16 (OMIM) | c.467C>G | 17.11 | PCR-MSH6sis3 MSH6_467C>G_SE CG | PCR-MSH6sis3 MSH6_467C>G_AS GC | ENST00000234420 |
| 192 | MSH6/2p16 (OMIM) | c.540T>C | 17.13 | PCR-MSH6sis3 MSH6_540T>C_SE TC | PCR-MSH6sis3 MSH6_540T>C_AS AG | ENST00000234420 |
| 193 | MSH6/2p16 (OMIM) | c.642C>T | 17.15 | PCR-MSH6sis4A MSH6_642C>T_SE CT | PCR-MSH6sis4A MSH6_642C>T_AS GA | ENST00000234420 |
| 194 | MSH6/2p16 (OMIM) | c.11.86C>G | 17.19 | PCR-MSH6sis4A MSH6_1186C>G_SE CG | PCR-MSH6sis4A MSH6_1186C>G_AS GC | ENST00000234420 |
| 195 | MSH6/2p16 (OMIM) | c.1508C>G | 17.21 | PCR-MSH6sis4B MSH6_1508C>G_SE CG | PCR-MSH6sis4B MSH6_1508C>G_AS GC | ENST00000234420 |
| 196 | MSH6/2p16 (OMIM) | c.1784delT | 17.23 | PCR-MSH6sis4B MSH6_1784delT_SE TA | PCR-MSH6sis4B MSH6_1784delT_AS AC | ENST00000234420 |
| 197 | MSH6/2p16 (OMIM) | c.1787delT | 17.25 | PCR-MSH6sis4B MSH6_1787delT_SE TG | PCR-MSH6sis4B MSH6_1787delT_AS AT | ENST00000234420 |
| 198 | MSH6/2p16 (OMIM) | c.3261delC | 17.27 | PCR-MSH6sis5 MSH6_3261_SE CT | PCR-MSH6sis5 MSH6_3261_AS GT | ENST00000234420 |
| 199 | MSH6/2p16 (OMIM) | c.3438+14A>T | 17.33 | PCR-MSH6sis5 MSH6_3438+14A>T_SE AT | PCR-MSH6sis5 MSH6_3438+14A>T_AS TA | ENST00000234420 |
| 200 | MSH6/2p16 (OMIM) | c.3514dupA | 17.35 | PCR-MSH6sis6 MSH6_3514dupA_SE GA | PCR-MSH6sis6 MSH6_3514dupA_AS AT | ENST00000234420 |
| 201 | MSH6/2p16 (OMIM) | c.3838C>T | 17.37 | PCR-MSH6sis8,9,10 MSH6_3838C>T_SE CT | PCR-MSH6sis8,9,10 MSH6_3838C>T_AS GA | ENST00000234420 |
| 202 | NOD2/16q12 (OMIM) | c.3020insC | 17.39 | PCR-NOD2_1 NOD2_3020insC_SE TC | PCR-NOD2_1 NOD2_3020insC_AS CG | ENSG00000167207 |
| 203 | CDKN2A/9p21.3 (OMIM) | p.A148T | 17.41 | PCR-CDKN2A_1 CDKN2A_A148T_SE GA | PCR-CDKN2A_1 CDKN2A_A148T_AS CT | ENST00000304494 |
| 204 | CYP1B1/2p22.2 | p.A119S | 17.43 | PCR-CYP1B1_1 CYP1B1_A119S_SE GT | PCR-CYP1B1_1 CYP1B1_A119S_AS CA | ENSG00000138061 |
| 205 | CHEK2/22q12.1 (OMIM) | c.1100delC | 17.45 | PCR-CHEK2_2 CHEK2_1100delC_SE CT | PCR-CHEK2_2 CHEK2_1100delC_AS GT | ENST00000328354 |
| 206 | CHEK2/22q12.1 (OMIM) | c.IVS2+1G>A | 17.47 | PCR-CHEK2_1 CHEK2_IVS2+1G>A_SE GA | PCR-CHEK2_1 CHEK2_IVS2+1G>A_AS CT | ENST00000328354 |

Preferably, microarray used in the method according to the invention is APEX microarray.

Probes in APEX microarray are immobilized on a glass slide at their 5' terminal.

APEX oligonucleotides (probes) are designed so as to flank the tested genetic change.

As used herein, the term "APEX microarray" means a glass slide measuring 24 x 60 x 0.15 mm coated with aminosilane with a linker (SAL), on which fields with immobilized oligonucleotide probes are arranged in a matrix system at specific positions.

Microarray slides (Genorama, Estonia) stored at 4°C, are washed before use in distilled water at 95°C, to wash out buffer which is blocking the probes and to minimize background level before the APEX reaction.

Fragmented PCR products are denatured in a heating block at 95°C (10 min.) and then added to the APEX Reaction Mix (Genorama, Estonia) containing fluorescently labeled dideoxsynucleotides (ddNTPs) (preferably FI-12-ddUTP, Cy3-ddCTP, Teras Red-ddATP, Cy5-ddGTP) and ThermoSequenase polymerase. Thus prepared mixture is applied on a microarray slide disposed in a heating block at a temperature of 58°C. Microarray is then covered with a glass coverslip LifterSlip (Genorama, Estonia) and incubated (58°C for 20 minutes). Hybrydyzation step of patient's fragmented DNA with microarray probes follows.

Hybridization consists in attaching fragmented amplification products with a probe immobilized on a slide in a complementary specific way.

Oligonucleotide probes specific for analyzed sites are designed so that each probe sequence ends just before the analyzed genetic change and its length is determined based on the melting point of a oligonucleotide single strand. Probes specific in terms of complementarity with analyzed DNA fragment hybridize at 58°C with fragmented amplification products, allowing APEX reaction. It occurs at the same temperature, and consists in extending of oligonucleotides (or probes) arranged in line and immobilized on a glass slide by one fluorescently labeled nucleotide, using thermostable polymerase (an enzyme capable of binding nucleotides in accordance with the principle of complementarity in direction of 5'-> 3' after providing appropriate reaction conditions), while at the same time causing termination of extending of the oligonucleotide. Probes must be designed so that the covalently attached, fluorescently labeled dideoxynucleotide (terminal nucleotide) signals the presence or absence of mutations. Extension is carried out on the basis of complementarity with nucleotide in patient's DNA sequences, therefore specifically. Upon excitation of appropriately fluorescently labeled terminal nucleotides with appropriate wavelength of laser light, signal emitted by fluorochromes provides information on presence of specific nucleotide and thereby of a mutation or absence of a genetic change. For each mutation site the two types of probes are designed in most cases - sense probe (SE; designed on the basis of gDNA sense sequence) and antisense (AS; complementary to the sense strand, designed on the basis of gDNA antisense sequence). Probes are located on the microarray, each in duplicate. Because of that a slide comprises 4 fields of probes for each mutation site - two fields with SE probes and two fields AS probes.

APEX reaction is terminated by placing the microarray slides in 0.3% Alcanox solution for 3 minutes. The slides are then washed and a drop of Atlas Antifade Reagent (Genorama, Estonia) is applied on the microarray, what prevents fading of fluorochromes.

Readout of signals is carried out using a microarray scanner, preferably Genorama Quattrolmager (Genorama, Estonia) equipped with 4 lasers with different wavelength ranges. Slides are excited in turns with four different lengths of laser light, causing excitation of fluorescently labeled nucleotides by the phenomenon of internal reflection.

Use of an CCD (Charge Coupled Device) image sensor allows registration of light signals from each of fluorochromes. Results are archived and analyzed using Genorama Genotyping Software (Genorama Estonia).

Subsequently verification of positive and doubtful signals is performed by DNA sequencing by means of chain termination method (in this case using BigDye Terminator kit, Applied Biosystems, USA and ABI Prism 3100-Avant apparatus, Applied Biosystems, USA).

Using an appropriate compilation of specific, designed for the need of this diagnostic test, primers and oligonucleotide probes, enables innovative diagnostic of genetic predispositions using DNA microarray technology.

The present invention also relates to a kit for carrying out the method according to the invention which comprises:
a) DNA microarrays with immobilized oligonucleotides - type SE and AS probes presented in SEQ ID NOS: 1-398;
b) PCR primers shown in SEQ ID NOS: 399-796 for carrying out amplification of DNA template fragments comprising tested changes;
c) PCR reagents;
d) APEX mixture;
e) slip covers;
f) washing reagent for slides;
g) protocol.

PCR reagents preferably comprise polymerase, reaction buffor, dNTPs (including 20% UTP), MgCl₂.

APEX mixture preferably comprises ThermoSequenase polymerase, fluorescently labeled dideoxynucleotides: (FL-12-ddUTP; Cy3-ddCTP, Texas Red-ddATP, Cy5-ddGTP).

The kit of the present invention includes primers designed for amplification of sequence fragments derived from the patient's DNA template.

DNA template fragments of interest are fragments comprising regions with analyzed mutation sites or polymorphic sites in the above mentioned genes. Analyzed sites have been selected on the basis of available literature. So far one particular gene, which is responsible for triggering colorectal cancer has not been known.

In studies devoted to the search for genes associated with occurrence of colorectal cancer, much attention is paid to changes occurring in MSH6 gene (Barrow E. et al., Clin. Genet. 74: 233-242, 2008).

Other tumor suppressor genes, which play a key role in DNA repair and maintaining genomic stability, in which mutations are reflected in the occurrence of colorectal cancer include MLH1 and MSH2 genes (Aaltonen L et al., Science 260: 812-816, 1993; Wijnen J., New Eng J. Med. 339: 511-518, 1998).

The relationship of c.3020ins mutation in NOD2 gene with colorectal cancer is reported by Kurzawski G et al. (Kurzawski G et al. Cancer Res 1, 64(5): 1604-6, 2004).

The next gene considered in the case of predisposition to colorectal cancer is CDKN2A (D 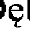 bniak T., Hereditary Cancer in Clinical Practice 5: 97-116, 2007).

Another gene which is relevant in determining predisposition to colorectal cancer is CHEK2 gene (Suchy J. at al. Int J Cancer 126(12): 3005-9, 2010)

Yet another gene considered in the case of colorectal cancer in the Polish population is CYP1B1 gene (Trubicka J at al. MC Cancer 10: 420, 2010).

All the latest reports regarding published results of research relating to genetic predisposition to colorectal cancer, can be traced through OMIM (www.ncbi.nlm.nih.gov/omim) and InSiGHT (http://www.insight-group.org/mutations) databases, in which the relevant reports are updated on a regular basis.

The present invention relates also to a primer set for use in PCR amplification of genetic material. Primers according to the invention include nucleotide sequences presented as SEQ ID NOS: 399-796.

The scope of present invention also includes oligonucleotide sequences presented as SEQ ID NOS: 1-398 for use as probes for detecting genetic predisposition to colorectal cancer. The probes are designed to include SE and AS probes and may be used in other methods for hybridization of tested material.

The following example is provided for better understanding of the present invention, the scope of which is set forth in the appended claims.

### Example

The study was conducted on 21 patients with Lynch syndrome. All patients underwent an innovative test of genetic predisposition to development of colorectal cancer based on APEX microarray technology. Suitability of the tested family members with Lynch syndrome was assessed using the so-called Amsterdam criteria. All patients and their families are covered by a ministerial care program for families with high-risk of developing malignant tumors. Analyzed patients constituted a validation group of genetic predisposition test for colorectal cancer. Peripheral blood sample was taken from each patient and subjected to genetic test.

Individual stages of the analysis are shown on an example of one of the patients from the validation group who had colorectal cancer. The filed innovative method of diagnostics of genetic predispositions, including analysis of selected 206 sites in the human genome associated with genetic predisposition to colorectal cancer, revealed presence of 4 genetic changes in the MSH2 gene (c.1077-10T>C in a heterozygous configuration c.965G>A in heterozygous configuration, c.1661+12G>A in homozygous configuration and c.2006-6T>C in homozygous configuration).

Peripheral blood of a patient was analyzed. Patient's genomic DNA was isolated from 200 µl of peripheral blood supplemented with EDTA. Isolation of gDNA was conducted using NucleoSpin Blood Dx kit (Marcherey-Nagel, Germany). The obtained genetic material, suspended in 100 µl of elution buffer, was used as a template for amplification of 43 regions of 6 analyzed genes (MLH1, MSH2, MSH6, NOD2, CDK2A, CHEK2), comprising 206 analyzed mutation sites and polymorphic sites. Amplification was conducted by means of multiplex PCR method using 43 primer pairs and APEX Template Preparation amplification kit (Genorama, Estonia). In comparison with conventional PCR reaction, 20% fraction of dTTP in reaction mixture was replaced with dUTP. Amplification products were purified and concentrated using GeneJet PCR kit (Genorama, Estonia). Protection against fragmentation and functional inactivation of unincorporated nucleotides was achieved by carrying out a single stage of incubation using uracil N-glycosylase (Genorama, Estonia) and shrimp alkaline phosphatase (Genorama, Estonia).

In accordance with the description concerning the diagnostic test of genetic predisposition based on APEX microarray, oligonucleotides used as specific probes of the microarray, designed according to nucleotide sequences of MLH1, MSH2, MSH6, NOD2, CHEK2 genes for sense and antisense directions (SE and AS), enabled analysis of changes in genetic material of a patient. There were two types of probes, SE and AS for one analyzed mutation site, each in duplicate. Microarray slides (Genorama, Estonia) stored at 4°C, were labeled and washed in distilled water at 95°C before use to remove buffer blocking the probes and to minimize background level before the APEX reaction *(Arrayed Primer Extension).* PCR products fragmented in an earlier step were denatured in a heating block at 95°C (10 min.) and then added to the APEX reaction mixture (Genorama, Estonia) containing fluorescently labeled ddNTPs (F1-12-ddUTP, Cy3-ddCTP, Texas Red-ddATP, Cy5-ddGTP) and ThermoSequenase polymerase. Thus prepared mixture was applied to a microarray slide and placed in a heating block at a temperature of 58°C. The microarray was covered with LifterSlip coverslip (Genorama, Estonia) and incubated (58°C, 20 min.) The APEX reaction was terminated by placing the microarray slides in 0.3% Alcanox solution (3 min.), then the plates were washed three times with MilliQ water (95°C, 1 min.). Drops of Atlas Antifade Reagent (Genorama, Estonia) were applied on the microarray to prevent fading of fluorochromes. Signals readout was conducted using a microarray scanner Genorama QuattroImager (Genorama, Estonia). Owing to usage of CCD (Charge Coupled Device) image sensor it was possible to record light signals of each of fluorochromes. The results archived in TIFF format and analyzed using Genorama Genotyping Software (Genorama, Estonia).

Positive signals from the microarray were verified by DNA sequencing by method of chain termination, using BigDye Terminator v.3.1 Cycle Sequencing Kit (Applied Biosystems, USA) and ABI Prism 3100-Avant (Applied Biosystems, USA) apparatus to confirm changes detected by an alternative test method.

## Claims

1. A method for detecting genetic predisposition to colorectal cancer using DNA microarray, comprising:
(a) obtaining a biological sample from a patient;
(b) isolating genomic DNA from the sample obtained from a patient;
(c) amplification of regions containing analyzed mutation sites and polymorphic sites;
(d) purification and concentration of amplification products;
(e) fragmentation and functional inactivation of unincorporated nucleotides;
(f) hybridization of fragmented DNA obtained from a patient with oligonucleotide probes immobilized at specific points on a slide, designed in accordance with nucleotide sequences of at least MLH1, MSH2, MSH6, NOD2, CDK2A, CHEK2 and CYP1B1 genes for sense and antisense directions (SE and AS) of a sequence flanking genetic change site;
(g) adding, on the principle of complementarity, a single ddNTP to the end of each probe on the DNA array;
(h) excitation of a slide successively with at least four lengths of light and reading signals by means of a DNA microarray scanner.

2. Method according to claim 1, wherein the sample taken from a patient is a blood sample.

3. Method according to claim 1, wherein the sample taken from a patient is a swab from inner cheek surface.

4. Method according to claim 1, wherein amplification is conducted by means of PCR method using primers shown in SEQ ID NOS 399-796.

5. Method according to claim 1, wherein probes having sequences shown in SEQ ID NOS 1-398.

6. Method according to claim 1, wherein the microarray is APEX microarray and microarray analysis is conducted using an APEX scanner.

7. Method according to claim 1, wherein mutations in MLH1 are at least mutations Nos. 1-134 presented in Table 1.

8. Method according to claim 1, wherein mutations in MSH2 gene are at least mutations Nos.: 135-187 presented in Table 1.

9. Method according to claim 1, wherein mutations in MSH6 gene are at least mutations Nos.: 188-201 presented in Table 1.

10. Method according to claim 1, wherein the mutation in NOD2 gene is at least mutation 3020insC (no. 202 in Table 1).

11. Method according to claim 1, wherein the mutation in CDKN2 gene is at least mutation A148T (no. 203 in Table 1), and the mutation in CYP1B1 gene is at least mutation A119S (no. 204 in Table 1).

12. Method according to claim 1, wherein mutations in CHEK2 gene are at least mutations: 1100delC and IVS2+1G>A (nos. 205 and 206 in Table 1).

13. Kit for carrying out the method according to claim 1, which comprises:
a) DNA microarrays with immobilized oligonucleotides - type SE and AS probes presented in SEQ ID NOS: 1-398;
b) PCR primers shown in SEQ ID NOS: 399-796 for carrying out amplification of DNA template fragments comprising tested changes;
c) PCR reagents;
d) APEX mixture;
e) slip covers;
f) washing reagent for slides;
g) protocol.

14. A set of primers shown in SEQ ID NOS: 399-796 for use in the amplification of genetic material by means of PCR method.

15. Oligonucleotide sequences shown as SEQ ID NOS: 1-398 for use as probes for detecting genetic predisposition to colorectal cancer.


SEQUENCE LISTING

<110> Centrum Badan DNA Sp. z o. o.

<120> A method for detecting genetic predisposition to colorectal cancer
(CRC)

<160> 796

<170> BiSSAP 1.2

<210> 1
<211> 23
<212> DNA
<213> Artificial Sequence

<220>
<221> source
<222> 1..23
<223> /mol_type="unassigned DNA"
      /note="Oligonucleotide sense probe designed on
      the basis of MLH1 gene sequence for c.-93G>A site"
      /organism="Artificial Sequence"

<400> 1
gctggatggc gtaagctaca gct                                              23


<210> 2
<211> 23
<212> DNA
<213> Artificial Sequence

<220>
<221> source
<222> 1..23
<223> /mol_type="unassigned DNA"
      /note="Oligonucleotide antisense probe designed on
      the basis of MLH1 gene sequence for c.-93G>A site"
      /organism="Artificial Sequence"

<400> 2
gcctcgtgct cacgttcttc ctt                                              23


<210> 3
<211> 20
<212> DNA
<213> Artificial Sequence

<220>
<221> source
<222> 1..20
<223> /mol_type="unassigned DNA"
      /note="Oligonucleotide sense probe designed on
      the basis of MLH1 gene sequence for c.37delG site"
      /organism="Artificial Sequence"

<400> 3
gggttattcg gcggctggac                                                    20


<210> 4
<211> 22
<212> DNA
<213> Artificial Sequence

<220>
<221> source
<222> 1..22
<223> /mol_type="unassigned DNA"
      /note="Oligonucleotide antisense probe designed on
      the basis of MLH1 gene sequence for c.37delG site"
      /organism="Artificial Sequence"

<400> 4
gcgatgcggt tcaccactgt ct                                                 22


<210> 5
<211> 19
<212> DNA
<213> Artificial Sequence

<220>
<221> source
<222> 1..19
<223> /mol_type="unassigned DNA"
      /note="Oligonucleotide sense probe designed on
      the basis of MLH1 gene sequence for c.65G>C site"
      /organism="Artificial Sequence"

<400> 5
gtgaacggca tcgcggcgg                                                     19


<210> 6
<211> 22
<212> DNA
<213> Artificial Sequence

<220>
<221> source
<222> 1..22
<223> /mol_type="unassigned DNA"
      /note="Oligonucleotide antisense probe designed on
      the basis of MLH1 gene sequence for c.65G>C site"
      /organism="Artificial Sequence"

<400> 6
agctggccgc tggataactt cc                                                 22


<210> 7
<211> 19
<212> DNA

<213> Artificial Sequence

<220>
<221> source
<222> 1..19
<223> /mol_type="unassigned DNA"
      /note="Oligonucleotide sense probe designed on
      the basis of MLH1 gene sequence for c.66delG site"
      /organism="Artificial Sequence"

<400> 7
gaaccacatc gcggcgggg                                                    19


<210> 8
<211> 22
<212> DNA
<213> Artificial Sequence

<220>
<221> source
<222> 1..22
<223> /mol_type="unassigned DNA"
      /note="Oligonucleotide antisense probe designed on
      the basis of MLH1 gene sequence for c.66delG site"
      /organism="Artificial Sequence"

<400> 8
ctggccgctg gataacttcc cc                                                22


<210> 9
<211> 18
<212> DNA
<213> Artificial Sequence

<220>
<221> source
<222> 1..18
<223> /mol_type="unassigned DNA"
      /note="Oligonucleotide sense probe designed on
      the basis of MLH1 gene sequence for c.69A>T site"
      /organism="Artificial Sequence"

<400> 9
cagcatcgcg gcggggga                                                     18


<210> 10
<211> 26
<212> DNA
<213> Artificial Sequence

<220>
<221> source
<222> 1..26
<223> /mol_type="unassigned DNA"
      /note="Oligonucleotide antisense probe designed on

the basis of MLH1 gene sequence for c.69A>T site"
/organism="Artificial Sequence"

<400> 10
atagcattag ctggccgctg gataac 26

<210> 11
<211> 21
<212> DNA
<213> Artificial Sequence

<220>
<221> source
<222> 1..21
<223> /mol_type="unassigned DNA"
/note="Oligonucleotide sense probe designed on
the basis of MLH1 gene sequence for c.74T>C site"
/organism="Artificial Sequence"

<400> 11
gcatcgcggc gggggaagtt a 21

<210> 12
<211> 25
<212> DNA
<213> Artificial Sequence

<220>
<221> source
<222> 1..25
<223> /mol_type="unassigned DNA"
/note="Oligonucleotide antisense probe designed on
the basis of MLH1 gene sequence for c.74T>C site"
/organism="Artificial Sequence"

<400> 12
tttgatagca ttagctggcc gctgg 25

<210> 13
<211> 21
<212> DNA
<213> Artificial Sequence

<220>
<221> source
<222> 1..21
<223> /mol_type="unassigned DNA"
/note="Oligonucleotide sense probe designed on
the basis of MLH1 gene sequence for c.83C>T site"
/organism="Artificial Sequence"

<400> 13
ggggaagtta tccagcggcc a 21

<210> 14
<211> 31
<212> DNA
<213> Artificial Sequence

<220>
<221> source
<222> 1..31
<223> /mol_type="unassigned DNA"
      /note="Oligonucleotide antisense probe designed on
      the basis of MLH1 gene sequence for c.83C>T site"
      /organism="Artificial Sequence"

<400> 14
cagttctcaa tcatctcttt gatagcatta g                                    31


<210> 15
<211> 21
<212> DNA
<213> Artificial Sequence

<220>
<221> source
<222> 1..21
<223> /mol_type="unassigned DNA"
      /note="Oligonucleotide sense probe designed on
      the basis of MLH1 gene sequence for c.85G>T site"
      /organism="Artificial Sequence"

<400> 15
ggggaagtta tccagcggcc a                                               21


<210> 16
<211> 31
<212> DNA
<213> Artificial Sequence

<220>
<221> source
<222> 1..31
<223> /mol_type="unassigned DNA"
      /note="Oligonucleotide antisense probe designed on
      the basis of MLH1 gene sequence for c.85G>T site"
      /organism="Artificial Sequence"

<400> 16
cagttctcaa tcatctcttt gatagcatta g                                    31


<210> 17
<211> 26
<212> DNA
<213> Artificial Sequence

<220>
<221> source

<222> 1..26
<223> /mol_type="unassigned DNA"
      /note="Oligonucleotide sense probe designed on
      the basis of MLH1 gene sequence for c.104T>G site"
      /organism="Artificial Sequence"

<400> 17
gcggccaact aatgctatca aagaga                                          26


<210> 18
<211> 24
<212> DNA
<213> Artificial Sequence

<220>
<221> source
<222> 1..24
<223> /mol_type="unassigned DNA"
      /note="Oligonucleotide antisense probe designed on
      the basis of MLH1 gene sequence for c.104T>G site"
      /organism="Artificial Sequence"

<400> 18
ctccctccgt accagttctc aatc                                            24


<210> 19
<211> 32
<212> DNA
<213> Artificial Sequence

<220>
<221> source
<222> 1..32
<223> /mol_type="unassigned DNA"
      /note="Oligonucleotide sense probe designed on
      the basis of MLH1 gene sequence for c.131C>T site"
      /organism="Artificial Sequence"

<400> 19
agagatgatt gagaactgtt tagatgcaaa at                                   32


<210> 20
<211> 32
<212> DNA
<213> Artificial Sequence

<220>
<221> source
<222> 1..32
<223> /mol_type="unassigned DNA"
      /note="Oligonucleotide antisense probe designed on
      the basis of MLH1 gene sequence for c.131C>T site"
      /organism="Artificial Sequence"

<400> 20

agagatgatt gagaactgtt tagatgcaaa at                                    32

<210> 21
<211> 33
<212> DNA
<213> Artificial Sequence

<220>
<221> source
<222> 1..33
<223> /mol_type="unassigned DNA"
      /note="Oligonucleotide sense probe designed on
      the basis of MLH1 gene sequence for c.137G>T site"
      /organism="Artificial Sequence"

<400> 21
tgattgagaa ctgtttagat gcaaaatcca caa                                   33

<210> 22
<211> 29
<212> DNA
<213> Artificial Sequence

<220>
<221> source
<222> 1..29
<223> /mol_type="unassigned DNA"
      /note="Oligonucleotide antisense probe designed on
      the basis of MLH1 gene sequence for c.137G>T site"
      /organism="Artificial Sequence"

<400> 22
ggcctccctc tttaacaatc acttgaata                                        29

<210> 23
<211> 35
<212> DNA
<213> Artificial Sequence

<220>
<221> source
<222> 1..35
<223> /mol_type="unassigned DNA"
      /note="Oligonucleotide sense probe designed on
      the basis of MLH1 gene sequence for c.161G>A site"
      /organism="Artificial Sequence"

<400> 23
aaaatccaca agtattcaag tgattgttaa agagg                                 35

<210> 24
<211> 27
<212> DNA
<213> Artificial Sequence

<220>
<221> source
<222> 1..27
<223> /mol_type="unassigned DNA"
      /note="Oligonucleotide antisense probe designed on
      the basis of MLH1 gene sequence for c.161G>A site"
      /organism="Artificial Sequence"

<400> 24
tcttggatct caatcaactt caggcct                                          27


<210> 25
<211> 35
<212> DNA
<213> Artificial Sequence

<220>
<221> source
<222> 1..35
<223> /mol_type="unassigned DNA"
      /note="Oligonucleotide sense probe designed on
      the basis of MLH1 gene sequence for c.161delG site"
      /organism="Artificial Sequence"

<400> 25
aaaatccaca agtattcaag tgattgttaa agagg                                 35


<210> 26
<211> 25
<212> DNA
<213> Artificial Sequence

<220>
<221> source
<222> 1..25
<223> /mol_type="unassigned DNA"
      /note="Oligonucleotide antisense probe designed on
      the basis of MLH1 gene sequence for c.161delG site"
      /organism="Artificial Sequence"

<400> 26
ggatctcaat caacttcagg cctcc                                            25


<210> 27
<211> 25
<212> DNA
<213> Artificial Sequence

<220>
<221> source
<222> 1..25
<223> /mol_type="unassigned DNA"
      /note="Oligonucleotide sense probe designed on
      the basis of MLH1 gene sequence for c.184C>A/T site"

/organism="Artificial Sequence"

<400> 27
gggaggcgtg aagttgattc agatc                                          25


<210> 28
<211> 23
<212> DNA
<213> Artificial Sequence

<220>
<221> source
<222> 1..23
<223> /mol_type="unassigned DNA"
      /note="Oligonucleotide antisense probe designed on
      the basis of MLH1 gene sequence for c.184C>A/T site"
      /organism="Artificial Sequence"

<400> 28
cctgatcccg gtgccattgt ctt                                            23


<210> 29
<211> 27
<212> DNA
<213> Artificial Sequence

<220>
<221> source
<222> 1..27
<223> /mol_type="unassigned DNA"
      /note="Oligonucleotide sense probe designed on
      the basis of MLH1 gene sequence for c.191A>G site"
      /organism="Artificial Sequence"

<400> 29
gcctcaagtt gattcagatc caagaca                                        27


<210> 30
<211> 23
<212> DNA
<213> Artificial Sequence

<220>
<221> source
<222> 1..23
<223> /mol_type="unassigned DNA"
      /note="Oligonucleotide antisense probe designed on
      the basis of MLH1 gene sequence for c.191A>G site"
      /organism="Artificial Sequence"

<400> 30
cttctttcct gatcccggtg cca                                            23


<210> 31

<211> 29
<212> DNA
<213> Artificial Sequence

<220>
<221> source
<222> 1..29
<223> /mol_type="unassigned DNA"
      /note="Oligonucleotide sense probe designed on
      the basis of MLH1 gene sequence for c.194G>A site"
      /organism="Artificial Sequence"

<400> 31
cctcaagttg attcagatcc aagacaatg                                         29


<210> 32
<211> 25
<212> DNA
<213> Artificial Sequence

<220>
<221> source
<222> 1..25
<223> /mol_type="unassigned DNA"
      /note="Oligonucleotide antisense probe designed on
      the basis of MLH1 gene sequence for c.194G>A site"
      /organism="Artificial Sequence"

<400> 32
cagatcttct ttcctgatcc cggtg                                             25


<210> 33
<211> 27
<212> DNA
<213> Artificial Sequence

<220>
<221> source
<222> 1..27
<223> /mol_type="unassigned DNA"
      /note="Oligonucleotide sense probe designed on
      the basis of MLH1 gene sequence for c.199G>A/T site"
      /organism="Artificial Sequence"

<400> 33
ttgattcaga tccaagacaa tggcacc                                           27


<210> 34
<211> 30
<212> DNA
<213> Artificial Sequence

<220>
<221> source
<222> 1..30

<223> /mol_type="unassigned DNA"
      /note="Oligonucleotide antisense probe designed on
      the basis of MLH1 gene sequence for c.199G>A/T site"
      /organism="Artificial Sequence"

<400> 34
atacaatatc cacatcttct ttcctgatcc                                        30


<210> 35
<211> 25
<212> DNA
<213> Artificial Sequence

<220>
<221> source
<222> 1..25
<223> /mol_type="unassigned DNA"
      /note="Oligonucleotide sense probe designed on
      the basis of MLH1 gene sequence for c.200G>A site"
      /organism="Artificial Sequence"

<400> 35
attcagatcc aagacaatgg caccg                                             25


<210> 36
<211> 31
<212> DNA
<213> Artificial Sequence

<220>
<221> source
<222> 1..31
<223> /mol_type="unassigned DNA"
      /note="Oligonucleotide antisense probe designed on
      the basis of MLH1 gene sequence for c.200G>A site"
      /organism="Artificial Sequence"

<400> 36
acatacaata tccacatctt ctttcctgat c                                      31


<210> 37
<211> 23
<212> DNA
<213> Artificial Sequence

<220>
<221> source
<222> 1..23
<223> /mol_type="unassigned DNA"
      /note="Oligonucleotide sense probe designed on
      the basis of MLH1 gene sequence for c.203T>A site"
      /organism="Artificial Sequence"

<400> 37
gatccaagac aatggcaccg gga                                               23

<210> 38
<211> 32
<212> DNA
<213> Artificial Sequence

<220>
<221> source
<222> 1..32
<223> /mol_type="unassigned DNA"
      /note="Oligonucleotide antisense probe designed on
      the basis of MLH1 gene sequence for c.203T>A site"
      /organism="Artificial Sequence"

<400> 38
tttcacatac aatatccaga tcttctttcc tg                                32


<210> 39
<211> 23
<212> DNA
<213> Artificial Sequence

<220>
<221> source
<222> 1..23
<223> /mol_type="unassigned DNA"
      /note="Oligonucleotide sense probe designed on
      the basis of MLH1 gene sequence for c.206G>A site"
      /organism="Artificial Sequence"

<400> 39
ccaagacaat ggcaccggga tca                                          23


<210> 40
<211> 32
<212> DNA
<213> Artificial Sequence

<220>
<221> source
<222> 1..32
<223> /mol_type="unassigned DNA"
      /note="Oligonucleotide antisense probe designed on
      the basis of MLH1 gene sequence for c.206G>A site"
      /organism="Artificial Sequence"

<400> 40
acctttcaca tacaatatcc agatcttctt tc                                32


<210> 41
<211> 30
<212> DNA
<213> Artificial Sequence

<220>
<221> source
<222> 1..30
<223> /mol_type="unassigned DNA"
      /note="Oligonucleotide sense probe designed on
      the basis of MLH1 gene sequence for c.229T>C site"
      /organism="Artificial Sequence"

<400> 41
gggatgagga aagaagatct ggatattgta                                        30


<210> 42
<211> 29
<212> DNA
<213> Artificial Sequence

<220>
<221> source
<222> 1..29
<223> /mol_type="unassigned DNA"
      /note="Oligonucleotide antisense probe designed on
      the basis of MLH1 gene sequence for c.229T>C site"
      /organism="Artificial Sequence"

<400> 42
ctgcagttta ctagtagaga acctttcac                                         29


<210> 43
<211> 30
<212> DNA
<213> Artificial Sequence

<220>
<221> source
<222> 1..30
<223> /mol_type="unassigned DNA"
      /note="Oligonucleotide sense probe designed on
      the basis of MLH1 gene sequence for c.230G>A site"
      /organism="Artificial Sequence"

<400> 43
ggatcaggaa agaagatctg gatattgtat                                        30


<210> 44
<211> 30
<212> DNA
<213> Artificial Sequence

<220>
<221> source
<222> 1..30
<223> /mol_type="unassigned DNA"
      /note="Oligonucleotide antisense probe designed on
      the basis of MLH1 gene sequence for c.230G>A site"
      /organism="Artificial Sequence"

<400> 44
gactgcagtt tactagtagt gaacctttca                                        30


<210> 45
<211> 31
<212> DNA
<213> Artificial Sequence

<220>
<221> source
<222> 1..31
<223> /mol_type="unassigned DNA"
      /note="Oligonucleotide sense probe designed on
      the basis of MLH1 gene sequence for c.238T>G site"
      /organism="Artificial Sequence"

<400> 45
gaaagaagat ctggatattg tatgtgaaag g                                      31


<210> 46
<211> 29
<212> DNA
<213> Artificial Sequence

<220>
<221> source
<222> 1..29
<223> /mol_type="unassigned DNA"
      /note="Oligonucleotide antisense probe designed on
      the basis of MLH1 gene sequence for c.238T>G site"
      /organism="Artificial Sequence"

<400> 46
ctcaaaggac tgcagtttac tagtagtga                                         29


<210> 47
<211> 31
<212> DNA
<213> Artificial Sequence

<220>
<221> source
<222> 1..31
<223> /mol_type="unassigned DNA"
      /note="Oligonucleotide sense probe designed on
      the basis of MLH1 gene sequence for c.250A>G site"
      /organism="Artificial Sequence"

<400> 47
ggatattgta tgagaaaggt tcactactag t                                      31


<210> 48
<211> 27

<212> DNA
<213> Artificial Sequence

<220>
<221> source
<222> 1..27
<223> /mol_type="unassigned DNA"
      /note="Oligonucleotide antisense probe designed on
      the basis of MLH1 gene sequence for c.250A>G site"
      /organism="Artificial Sequence"

<400> 48
tggctaaatc ctcaaacgac tgcagtt                                          27


<210> 49
<211> 34
<212> DNA
<213> Artificial Sequence

<220>
<221> source
<222> 1..34
<223> /mol_type="unassigned DNA"
      /note="Oligonucleotide sense probe designed on
      the basis of MLH1 gene sequence for c.256C>T site"
      /organism="Artificial Sequence"

<400> 49
tattgtatga gaaaggttca ctactagtaa actg                                  34


<210> 50
<211> 30
<212> DNA
<213> Artificial Sequence

<220>
<221> source
<222> 1..30
<223> /mol_type="unassigned DNA"
      /note="Oligonucleotide antisense probe designed on
      the basis of MLH1 gene sequence for c.256C>T site"
      /organism="Artificial Sequence"

<400> 50
tagaaatact ggctaaatcc tcaaaggact                                       30


<210> 51
<211> 28
<212> DNA
<213> Artificial Sequence

<220>
<221> source
<222> 1..28
<223> /mol_type="unassigned DNA"

/note="Oligonucleotide sense probe designed on
the basis of MLH1 gene sequence for c.277A>G site"
/organism="Artificial Sequence"

<400> 51
taaactgcag tcctttgagg atttagcc                                          28


<210> 52
<211> 27
<212> DNA
<213> Artificial Sequence

<220>
<221> source
<222> 1..27
<223> /mol_type="unassigned DNA"
/note="Oligonucleotide antisense probe designed on
the basis of MLH1 gene sequence for c.277A>G site"
/organism="Artificial Sequence"

<400> 52
cacctcgaaa gccataggaa gaaatac                                           27


<210> 53
<211> 30
<212> DNA
<213> Artificial Sequence

<220>
<221> source
<222> 1..30
<223> /mol_type="unassigned DNA"
/note="Oligonucleotide sense probe designed on
the basis of MLH1 gene sequence for c.298C>T site"
/organism="Artificial Sequence"

<400> 53
gatttaccca gtatttctac ctatggcttt                                        30


<210> 54
<211> 30
<212> DNA
<213> Artificial Sequence

<220>
<221> source
<222> 1..30
<223> /mol_type="unassigned DNA"
/note="Oligonucleotide antisense probe designed on
the basis of MLH1 gene sequence for c.298C>T site"
/organism="Artificial Sequence"

<400> 54
atttcttgaa tctttagctt acctcacctc                                        30

<210> 55
<211> 30
<212> DNA
<213> Artificial Sequence

<220>
<221> source
<222> 1..30
<223> /mol_type="unassigned DNA"
      /note="Oligonucleotide sense probe designed on
      the basis of MLH1 gene sequence for c.299G>C site"
      /organism="Artificial Sequence"

<400> 55
atttacccag tatttctacc tatggctttc                                        30


<210> 56
<211> 31
<212> DNA
<213> Artificial Sequence

<220>
<221> source
<222> 1..31
<223> /mol_type="unassigned DNA"
      /note="Oligonucleotide antisense probe designed on
      the basis of MLH1 gene sequence for c.299G>C site"
      /organism="Artificial Sequence"

<400> 56
acatttcttg aatctttagc ttacctcacc t                                      31


<210> 57
<211> 28
<212> DNA
<213> Artificial Sequence

<220>
<221> source
<222> 1..28
<223> /mol_type="unassigned DNA"
      /note="Oligonucleotide sense probe designed on
      the basis of MLH1 gene sequence for c.304G>A site"
      /organism="Artificial Sequence"

<400> 57
cagtatttct acgtatggct ttcgaggt                                          28


<210> 58
<211> 36
<212> DNA
<213> Artificial Sequence

<220>

<221> source
<222> 1..36
<223> /mol_type="unassigned DNA"
      /note="Oligonucleotide antisense probe designed on
      the basis of MLH1 gene sequence for c.304G>A site"
      /organism="Artificial Sequence"

<400> 58
gatattttac acatttcttg aatcttttgc ttacct                                36


<210> 59
<211> 28
<212> DNA
<213> Artificial Sequence

<220>
<221> source
<222> 1..28
<223> /mol_type="unassigned DNA"
      /note="Oligonucleotide sense probe designed on
      the basis of MLH1 gene sequence for c.306G>T site"
      /organism="Artificial Sequence"

<400> 59
gtatttctac gtatggcttt cgaggtga                                         28


<210> 60
<211> 36
<212> DNA
<213> Artificial Sequence

<220>
<221> source
<222> 1..36
<223> /mol_type="unassigned DNA"
      /note="Oligonucleotide antisense probe designed on
      the basis of MLH1 gene sequence for c.306G>T site"
      /organism="Artificial Sequence"

<400> 60
aggatatttt acacatttct tgaatcttta gcttac                                36


<210> 61
<211> 28
<212> DNA
<213> Artificial Sequence

<220>
<221> source
<222> 1..28
<223> /mol_type="unassigned DNA"
      /note="Oligonucleotide sense probe designed on
      the basis of MLH1 gene sequence for c.306+1G>A site"
      /organism="Artificial Sequence"

<400> 61
tatttctacg tatggctttc gaggtgag                                          28


<210> 62
<211> 36
<212> DNA
<213> Artificial Sequence

<220>
<221> source
<222> 1..36
<223> /mol_type="unassigned DNA"
      /note="Oligonucleotide antisense probe designed on
      the basis of MLH1 gene sequence for c.306+1G>A site"
      /organism="Artificial Sequence"

<400> 62
gaggatattt tacacatttc ttgaatcttt agctta                                 36


<210> 63
<211> 26
<212> DNA
<213> Artificial Sequence

<220>
<221> source
<222> 1..26
<223> /mol_type="unassigned DNA"
      /note="Oligonucleotide sense probe designed on
      the basis of MLH1 gene sequence for c.320T>G site"
      /organism="Artificial Sequence"

<400> 63
ttttcttcct tagggtttgg ccagca                                            26


<210> 64
<211> 30
<212> DNA
<213> Artificial Sequence

<220>
<221> source
<222> 1..30
<223> /mol_type="unassigned DNA"
      /note="Oligonucleotide antisense probe designed on
      the basis of MLH1 gene sequence for c.320T>G site"
      /organism="Artificial Sequence"

<400> 64
gttgtaatag taacatgagg cacatggctt                                        30


<210> 65
<211> 24
<212> DNA

<213> Artificial Sequence

<220>
<221> source
<222> 1..24
<223> /mol_type="unassigned DNA"
      /note="Oligonucleotide sense probe designed on
      the basis of MLH1 gene sequence for c.332C>T site"
      /organism="Artificial Sequence"

<400> 65
ctttgcccag cataagccat gtgg                                              24


<210> 66
<211> 32
<212> DNA
<213> Artificial Sequence

<220>
<221> source
<222> 1..32
<223> /mol_type="unassigned DNA"
      /note="Oligonucleotide antisense probe designed on
      the basis of MLH1 gene sequence for c.332C>T site"
      /organism="Artificial Sequence"

<400> 66
catcagctgt tttcgttgta atagtaacat ga                                     32


<210> 67
<211> 31
<212> DNA
<213> Artificial Sequence

<220>
<221> source
<222> 1..31
<223> /mol_type="unassigned DNA"
      /note="Oligonucleotide sense probe designed on
      the basis of MLH1 gene sequence for c.350C>G/T site"
      /organism="Artificial Sequence"

<400> 67
ataagcgatg tggctcatgt tactattaca a                                      31


<210> 68
<211> 27
<212> DNA
<213> Artificial Sequence

<220>
<221> source
<222> 1..27
<223> /mol_type="unassigned DNA"
      /note="Oligonucleotide antisense probe designed on

the basis of MLH1 gene sequence for c.350C>G/T site"
/organism="Artificial Sequence"

<400> 68
tatgcacact ttccatcagc tgttttc 27

<210> 69
<211> 40
<212> DNA
<213> Artificial Sequence

<220>
<221> source
<222> 1..40
<223> /mol_type="unassigned DNA"
/note="Oligonucleotide sense probe designed on
the basis of MLH1 gene sequence for c.382G>C site"
/organism="Artificial Sequence"

<400> 69
ctctctactg gatattaatt tgttatattt tctcattaga 40

<210> 70
<211> 29
<212> DNA
<213> Artificial Sequence

<220>
<221> source
<222> 1..29
<223> /mol_type="unassigned DNA"
/note="Oligonucleotide antisense probe designed on
the basis of MLH1 gene sequence for c.382G>C site"
/organism="Artificial Sequence"

<400> 70
ggctttcagt tttccatctg agtaacttg 29

<210> 71
<211> 41
<212> DNA
<213> Artificial Sequence

<220>
<221> source
<222> 1..41
<223> /mol_type="unassigned DNA"
/note="Oligonucleotide sense probe designed on
the basis of MLH1 gene sequence for c.392C>A site"
/organism="Artificial Sequence"

<400> 71
ggatattaat ttgttatatt ttctcattag agcaagttac t 41

<210> 72
<211> 27
<212> DNA
<213> Artificial Sequence

<220>
<221> source
<222> 1..27
<223> /mol_type="unassigned DNA"
      /note="Oligonucleotide antisense probe designed on
      the basis of MLH1 gene sequence for c.392C>A site"
      /organism="Artificial Sequence"

<400> 72
ttaggagggg ctttcagttt tccatct                                          27


<210> 73
<211> 45
<212> DNA
<213> Artificial Sequence

<220>
<221> source
<222> 1..45
<223> /mol_type="unassigned DNA"
      /note="Oligonucleotide sense probe designed on
      the basis of MLH1 gene sequence for c.394G>C site"
      /organism="Artificial Sequence"

<400> 73
ctggatatta atttgttata ttttctcatt agagcaagtt actca                      45


<210> 74
<211> 27
<212> DNA
<213> Artificial Sequence

<220>
<221> source
<222> 1..27
<223> /mol_type="unassigned DNA"
      /note="Oligonucleotide antisense probe designed on
      the basis of MLH1 gene sequence for c.394G>C site"
      /organism="Artificial Sequence"

<400> 74
gtttaggagg ggctttcagt tttccat                                          27


<210> 75
<211> 49
<212> DNA
<213> Artificial Sequence

<220>
<221> source

<222> 1..49
<223> /mol_type="unassigned DNA"
      /note="Oligonucleotide sense probe designed on
      the basis of MLH1 gene sequence for c.453+79A>G site"
      /organism="Artificial Sequence"

<400> 75
ttgaagcttt gtttgtataa atattggaat aaaaaataaa attgcttct                49


<210> 76
<211> 41
<212> DNA
<213> Artificial Sequence

<220>
<221> source
<222> 1..41
<223> /mol_type="unassigned DNA"
      /note="Oligonucleotide antisense probe designed on
      the basis of MLH1 gene sequence for c.453+79A>G site"
      /organism="Artificial Sequence"

<400> 76
attaactagt gcaaattcat tttattatta ccctgaaaac t                        41


<210> 77
<211> 40
<212> DNA
<213> Artificial Sequence

<220>
<221> source
<222> 1..40
<223> /mol_type="unassigned DNA"
      /note="Oligonucleotide sense probe designed on
      the basis of MLH1 gene sequence for c.454-1G>A site"
      /organism="Artificial Sequence"

<400> 77
tacttctatg aatttacaag aaaaatcaat cttctgttca                          40


<210> 78
<211> 27
<212> DNA
<213> Artificial Sequence

<220>
<221> source
<222> 1..27
<223> /mol_type="unassigned DNA"
      /note="Oligonucleotide antisense probe designed on
      the basis of MLH1 gene sequence for c.454-1G>A site"
      /organism="Artificial Sequence"

<400> 78

ggctatgttg taaaaaaggt cctccac                                          27

<210> 79
<211> 24
<212> DNA
<213> Artificial Sequence

<220>
<221> source
<222> 1..24
<223> /mol_type="unassigned DNA"
      /note="Oligonucleotide sense probe designed on
      the basis of MLH1 gene sequence for c.464T>G site"
      /organism="Artificial Sequence"

<400> 79
aatcttctgt tcaggtggag gacc                                             24

<210> 80
<211> 29
<212> DNA
<213> Artificial Sequence

<220>
<221> source
<222> 1..29
<223> /mol_type="unassigned DNA"
      /note="Oligonucleotide antisense probe designed on
      the basis of MLH1 gene sequence for c.464T>G site"
      /organism="Artificial Sequence"

<400> 80
cttttctcct cgtggctatg ttgtaaaaa                                        29

<210> 81
<211> 30
<212> DNA
<213> Artificial Sequence

<220>
<221> source
<222> 1..30
<223> /mol_type="unassigned DNA"
      /note="Oligonucleotide sense probe designed on
      the basis of MLH1 gene sequence for c.479C>T site"
      /organism="Artificial Sequence"

<400> 81
ttcacgtgga ggaccttttt tacaacatag                                       30

<210> 82
<211> 32
<212> DNA
<213> Artificial Sequence

<220>
<221> source
<222> 1..32
<223> /mol_type="unassigned DNA"
      /note="Oligonucleotide antisense probe designed on
      the basis of MLH1 gene sequence for c.479C>T site"
      /organism="Artificial Sequence"

<400> 82
cacttggatt ttttaaagct tttctcctcg tg                                    32


<210> 83
<211> 30
<212> DNA
<213> Artificial Sequence

<220>
<221> source
<222> 1..30
<223> /mol_type="unassigned DNA"
      /note="Oligonucleotide sense probe designed on
      the basis of MLH1 gene sequence for c.544A>G site"
      /organism="Artificial Sequence"

<400> 83
gaatatggga aaattttgga agttgttggc                                       30


<210> 84
<211> 25
<212> DNA
<213> Artificial Sequence

<220>
<221> source
<222> 1..25
<223> /mol_type="unassigned DNA"
      /note="Oligonucleotide antisense probe designed on
      the basis of MLH1 gene sequence for c.544A>G site"
      /organism="Artificial Sequence"

<400> 84
ccactccgag attttggact gtacc                                            25


<210> 85
<211> 35
<212> DNA
<213> Artificial Sequence

<220>
<221> source
<222> 1..35
<223> /mol_type="unassigned DNA"
      /note="Oligonucleotide sense probe designed on
      the basis of MLH1 gene sequence for c.546-2A>G site"

/organism="Artificial Sequence"

<400> 85
gcaactcttt tcttactctt ttgtttttct tttcc                                35

<210> 86
<211> 30
<212> DNA
<213> Artificial Sequence

<220>
<221> source
<222> 1..30
<223> /mol_type="unassigned DNA"
      /note="Oligonucleotide antisense probe designed on
      the basis of MLH1 gene sequence for c.546-2A>G site"
      /organism="Artificial Sequence"

<400> 86
aactaatgcc tgcattgtgt actgaatacc                                      30

<210> 87
<211> 31
<212> DNA
<213> Artificial Sequence

<220>
<221> source
<222> 1..31
<223> /mol_type="unassigned DNA"
      /note="Oligonucleotide sense probe designed on
      the basis of MLH1 gene sequence for c.577T>C site"
      /organism="Artificial Sequence"

<400> 87
gtattcagta cacaatgcag gcattagttt c                                    31

<210> 88
<211> 32
<212> DNA
<213> Artificial Sequence

<220>
<221> source
<222> 1..32
<223> /mol_type="unassigned DNA"
      /note="Oligonucleotide antisense probe designed on
      the basis of MLH1 gene sequence for c.577T>C site"
      /organism="Artificial Sequence"

<400> 88
ccccataaac caagaactta cttttttaac tg                                  32

<210> 89

<211> 35
<212> DNA
<213> Artificial Sequence

<220>
<221> source
<222> 1..35
<223> /mol_type="unassigned DNA"
      /note="Oligonucleotide sense probe designed on
      the basis of MLH1 gene sequence for c.589-2A>G site"
      /organism="Artificial Sequence"

<400> 89
caataaatcc ttgtgtcttc tgctgtttgt ttatc                                35


<210> 90
<211> 27
<212> DNA
<213> Artificial Sequence

<220>
<221> source
<222> 1..27
<223> /mol_type="unassigned DNA"
      /note="Oligonucleotide antisense probe designed on
      the basis of MLH1 gene sequence for c.589-2A>G site"
      /organism="Artificial Sequence"

<400> 90
ctaacatcag ctactgtctc tccttgc                                         27


<210> 91
<211> 29
<212> DNA
<213> Artificial Sequence

<220>
<221> source
<222> 1..29
<223> /mol_type="unassigned DNA"
      /note="Oligonucleotide sense probe designed on
      the basis of MLH1 gene sequence for c.595G>C site"
      /organism="Artificial Sequence"

<400> 91
gtcttctggt gtttgtttat cagcaagga                                       29


<210> 92
<211> 23
<212> DNA
<213> Artificial Sequence

<220>
<221> source
<222> 1..23

<223> /mol_type="unassigned DNA"
      /note="Oligonucleotide antisense probe designed on
      the basis of MLH1 gene sequence for c.595G>C site"
      /organism="Artificial Sequence"

<400> 92
ttaactaaaa gggggctctg aca                                              23


<210> 93
<211> 23
<212> DNA
<213> Artificial Sequence

<220>
<221> source
<222> 1..23
<223> /mol_type="unassigned DNA"
      /note="Oligonucleotide sense probe designed on
      the basis of MLH1 gene sequence for c.637G>A site"
      /organism="Artificial Sequence"

<400> 93
ggacactacc caatgcctca acc                                              23


<210> 94
<211> 30
<212> DNA
<213> Artificial Sequence

<220>
<221> source
<222> 1..30
<223> /mol_type="unassigned DNA"
      /note="Oligonucleotide antisense probe designed on
      the basis of MLH1 gene sequence for c.637G>A site"
      /organism="Artificial Sequence"

<400> 94
catttccaaa gatggagcga atattgtcca                                       30


<210> 95
<211> 26
<212> DNA
<213> Artificial Sequence

<220>
<221> source
<222> 1..26
<223> /mol_type="unassigned DNA"
      /note="Oligonucleotide sense probe designed on
      the basis of MLH1 gene sequence for c.649C>T site"
      /organism="Artificial Sequence"

<400> 95
ccaatgcctc aaccgtggac aatatt                                           26

<210> 96
<211> 27
<212> DNA
<213> Artificial Sequence

<220>
<221> source
<222> 1..27
<223> /mol_type="unassigned DNA"
      /note="Oligonucleotide antisense probe designed on
      the basis of MLH1 gene sequence for c.649C>T site"
      /organism="Artificial Sequence"

<400> 96
gactaacagc atttccaaag atcgagc                                          27


<210> 97
<211> 25
<212> DNA
<213> Artificial Sequence

<220>
<221> source
<222> 1..25
<223> /mol_type="unassigned DNA"
      /note="Oligonucleotide sense probe designed on
      the basis of MLH1 gene sequence for c.655A>C/G site"
      /organism="Artificial Sequence"

<400> 97
ctcaaccgtg gacaatattc gctcc                                            25


<210> 98
<211> 29
<212> DNA
<213> Artificial Sequence

<220>
<221> source
<222> 1..29
<223> /mol_type="unassigned DNA"
      /note="Oligonucleotide antisense probe designed on
      the basis of MLH1 gene sequence for c.655A>C/G site"
      /organism="Artificial Sequence"

<400> 98
gacataccga ctaacagcat ttccaaaga                                        29


<210> 99
<211> 28
<212> DNA
<213> Artificial Sequence

<220>
<221> source
<222> 1..28
<223> /mol_type="unassigned DNA"
      /note="Oligonucleotide sense probe designed on
      the basis of MLH1 gene sequence for c.676C>T site"
      /organism="Artificial Sequence"

<400> 99
tcgctcgatc tttggaaatg ctgttagt                                          28


<210> 100
<211> 45
<212> DNA
<213> Artificial Sequence

<220>
<221> source
<222> 1..45
<223> /mol_type="unassigned DNA"
      /note="Oligonucleotide antisense probe designed on
      the basis of MLH1 gene sequence for c.676C>T site"
      /organism="Artificial Sequence"

<400> 100
gataataaat gtaatagatt tttttatata ggttatcgac atacc                       45


<210> 101
<211> 27
<212> DNA
<213> Artificial Sequence

<220>
<221> source
<222> 1..27
<223> /mol_type="unassigned DNA"
      /note="Oligonucleotide sense probe designed on
      the basis of MLH1 gene sequence for c.677G>A/T site"
      /organism="Artificial Sequence"

<400> 101
gctcgatctt tggaaatgct gttagtc                                           27


<210> 102
<211> 44
<212> DNA
<213> Artificial Sequence

<220>
<221> source
<222> 1..44
<223> /mol_type="unassigned DNA"
      /note="Oligonucleotide antisense probe designed on
      the basis of MLH1 gene sequence for c.677G>A/T site"
      /organism="Artificial Sequence"

<400> 102
gtatgtcgat aacctatata aaaaaatctt ttacatttat tatc                44

<210> 103
<211> 27
<212> DNA
<213> Artificial Sequence

<220>
<221> source
<222> 1..27
<223> /mol_type="unassigned DNA"
      /note="Oligonucleotide sense probe designed on
      the basis of MLH1 gene sequence for c.677+3A>G site"
      /organism="Artificial Sequence"

<400> 103
ccatctttgg aaatgctgtt agtcggt                                   27

<210> 104
<211> 44
<212> DNA
<213> Artificial Sequence

<220>
<221> source
<222> 1..44
<223> /mol_type="unassigned DNA"
      /note="Oligonucleotide antisense probe designed on
      the basis of MLH1 gene sequence for c.677+3A>G site"
      /organism="Artificial Sequence"

<400> 104
caagataata aatgtataag atttttttat ataggttatc gaca                44

<210> 105
<211> 30
<212> DNA
<213> Artificial Sequence

<220>
<221> source
<222> 1..30
<223> /mol_type="unassigned DNA"
      /note="Oligonucleotide sense probe designed on
      the basis of MLH1 gene sequence for c.731G>A site"
      /organism="Artificial Sequence"

<400> 105
aggataaaac cctagccttc aaaatgaatg                                30

<210> 106
<211> 32

<212> DNA
<213> Artificial Sequence

<220>
<221> source
<222> 1..32
<223> /mol_type="unassigned DNA"
      /note="Oligonucleotide antisense probe designed on
      the basis of MLH1 gene sequence for c.731G>A site"
      /organism="Artificial Sequence"

<400> 106
tcttcactga gtagtttgca ttggatatgt aa                                    32


<210> 107
<211> 33
<212> DNA
<213> Artificial Sequence

<220>
<221> source
<222> 1..33
<223> /mol_type="unassigned DNA"
      /note="Oligonucleotide sense probe designed on
      the basis of MLH1 gene sequence for c.739T>C site"
      /organism="Artificial Sequence"

<400> 107
aaaacgctag ccttcaaaat gaatggttac ata                                   33


<210> 108
<211> 28
<212> DNA
<213> Artificial Sequence

<220>
<221> source
<222> 1..28
<223> /mol_type="unassigned DNA"
      /note="Oligonucleotide antisense probe designed on
      the basis of MLH1 gene sequence for c.739T>C site"
      /organism="Artificial Sequence"

<400> 108
cacttcttca ctgagtagtt tgcattgg                                         28


<210> 109
<211> 31
<212> DNA
<213> Artificial Sequence

<220>
<221> source
<222> 1..31
<223> /mol_type="unassigned DNA"

      /note="Oligonucleotide sense probe designed on
      the basis of MLH1 gene sequence for c.778C>T site"
      /organism="Artificial Sequence"

<400> 109
aaactactca gtgaagaagt gcatcttctt a                                    31


<210> 110
<211> 33
<212> DNA
<213> Artificial Sequence

<220>
<221> source
<222> 1..33
<223> /mol_type="unassigned DNA"
      /note="Oligonucleotide antisense probe designed on
      the basis of MLH1 gene sequence for c.778C>T site"
      /organism="Artificial Sequence"

<400> 110
atgtggttct ttttaactta cggttgatga aga                                  33


<210> 111
<211> 30
<212> DNA
<213> Artificial Sequence

<220>
<221> source
<222> 1..30
<223> /mol_type="unassigned DNA"
      /note="Oligonucleotide sense probe designed on
      the basis of MLH1 gene sequence for c.790+1G>A site"
      /organism="Artificial Sequence"

<400> 111
agaagtgcat cttcttactc ttcatcaacc                                      30


<210> 112
<211> 32
<212> DNA
<213> Artificial Sequence

<220>
<221> source
<222> 1..32
<223> /mol_type="unassigned DNA"
      /note="Oligonucleotide antisense probe designed on
      the basis of MLH1 gene sequence for c.790+1G>A site"
      /organism="Artificial Sequence"

<400> 112
agtggatttc ccatgtggtt ctttttaact ta                                   32

<210> 113
<211> 36
<212> DNA
<213> Artificial Sequence

<220>
<221> source
<222> 1..36
<223> /mol_type="unassigned DNA"
      /note="Oligonucleotide sense probe designed on
      the basis of MLH1 gene sequence for c.793C>T site"
      /organism="Artificial Sequence"

<400> 113
gttttgaact ggttgctttc tttttattgt ttagat                                36


<210> 114
<211> 30
<212> DNA
<213> Artificial Sequence

<220>
<221> source
<222> 1..30
<223> /mol_type="unassigned DNA"
      /note="Oligonucleotide antisense probe designed on
      the basis of MLH1 gene sequence for c.793C>T site"
      /organism="Artificial Sequence"

<400> 114
cttttctcaa ggaagtagat tctaccagac                                       30


<210> 115
<211> 37
<212> DNA
<213> Artificial Sequence

<220>
<221> source
<222> 1..37
<223> /mol_type="unassigned DNA"
      /note="Oligonucleotide sense probe designed on
      the basis of MLH1 gene sequence for c.794G>A site"
      /organism="Artificial Sequence"

<400> 115
gttttgaact ggttgctttc tttttattgt ttagatc                               37


<210> 116
<211> 30
<212> DNA
<213> Artificial Sequence

<220>

<221> source
<222> 1..30
<223> /mol_type="unassigned DNA"
      /note="Oligonucleotide antisense probe designed on
      the basis of MLH1 gene sequence for c.794G>A site"
      /organism="Artificial Sequence"

<400> 116
gcttttctct aggaagttga ttctaccaga                                        30


<210> 117
<211> 34
<212> DNA
<213> Artificial Sequence

<220>
<221> source
<222> 1..34
<223> /mol_type="unassigned DNA"
      /note="Oligonucleotide sense probe designed on
      the basis of MLH1 gene sequence for c.803A>G site"
      /organism="Artificial Sequence"

<400> 117
ttgctttctt tttattgttt agatcgtctg gtag                                   34


<210> 118
<211> 32
<212> DNA
<213> Artificial Sequence

<220>
<221> source
<222> 1..32
<223> /mol_type="unassigned DNA"
      /note="Oligonucleotide antisense probe designed on
      the basis of MLH1 gene sequence for c.803A>G site"
      /organism="Artificial Sequence"

<400> 118
ctgtttctat ggcttttctc taggaagttg at                                     32


<210> 119
<211> 29
<212> DNA
<213> Artificial Sequence

<220>
<221> source
<222> 1..29
<223> /mol_type="unassigned DNA"
      /note="Oligonucleotide sense probe designed on
      the basis of MLH1 gene sequence for c.814T>G site"
      /organism="Artificial Sequence"

<400> 119
gtttagatcg tctggtagaa tcaacttcc                                          29


<210> 120
<211> 30
<212> DNA
<213> Artificial Sequence

<220>
<221> source
<222> 1..30
<223> /mol_type="unassigned DNA"
      /note="Oligonucleotide antisense probe designed on
      the basis of MLH1 gene sequence for c.814T>G site"
      /organism="Artificial Sequence"

<400> 120
ctgcatacac tgtttctatg gcttttctca                                         30


<210> 121
<211> 29
<212> DNA
<213> Artificial Sequence

<220>
<221> source
<222> 1..29
<223> /mol_type="unassigned DNA"
      /note="Oligonucleotide sense probe designed on
      the basis of MLH1 gene sequence for c.842C>T site"
      /organism="Artificial Sequence"

<400> 121
cttgagaaaa gccatagaaa cagtgtatg                                          29


<210> 122
<211> 26
<212> DNA
<213> Artificial Sequence

<220>
<221> source
<222> 1..26
<223> /mol_type="unassigned DNA"
      /note="Oligonucleotide antisense probe designed on
      the basis of MLH1 gene sequence for c.842C>T site"
      /organism="Artificial Sequence"

<400> 122
ggtgtgtgtt tttgggcaaa taggct                                             26


<210> 123
<211> 28
<212> DNA

<213> Artificial Sequence

<220>
<221> source
<222> 1..28
<223> /mol_type="unassigned DNA"
      /note="Oligonucleotide sense probe designed on
      the basis of MLH1 gene sequence for c.875T>C site"
      /organism="Artificial Sequence"

<400> 123
tatttgccca aaaacacaca cccattcc                                          28


<210> 124
<211> 27
<212> DNA
<213> Artificial Sequence

<220>
<221> source
<222> 1..27
<223> /mol_type="unassigned DNA"
      /note="Oligonucleotide antisense probe designed on
      the basis of MLH1 gene sequence for c.875T>C site"
      /organism="Artificial Sequence"

<400> 124
agtttggtgc tacatttcct gaggtac                                           27


<210> 125
<211> 27
<212> DNA
<213> Artificial Sequence

<220>
<221> source
<222> 1..27
<223> /mol_type="unassigned DNA"
      /note="Oligonucleotide sense probe designed on
      the basis of MLH1 gene sequence for c.882C>T site"
      /organism="Artificial Sequence"

<400> 125
caaaaacaca cacccattcc tgtacct                                           27


<210> 126
<211> 27
<212> DNA
<213> Artificial Sequence

<220>
<221> source
<222> 1..27
<223> /mol_type="unassigned DNA"
      /note="Oligonucleotide antisense probe designed on

the basis of MLH1 gene sequence for c.882C>T site"
      /organism="Artificial Sequence"

<400> 126
gttgaggagt ttggtgctac attacct                                          27


<210> 127
<211> 27
<212> DNA
<213> Artificial Sequence

<220>
<221> source
<222> 1..27
<223> /mol_type="unassigned DNA"
      /note="Oligonucleotide sense probe designed on
      the basis of MLH1 gene sequence for c.883delAGGT site"
      /organism="Artificial Sequence"

<400> 127
aaaacacaca cccattcctg tacctca                                          27


<210> 128
<211> 28
<212> DNA
<213> Artificial Sequence

<220>
<221> source
<222> 1..28
<223> /mol_type="unassigned DNA"
      /note="Oligonucleotide antisense probe designed on
      the basis of MLH1 gene sequence for c.883delAGGT site"
      /organism="Artificial Sequence"

<400> 128
tcttggttga ggagtttggt gctacatt                                         28


<210> 129
<211> 27
<212> DNA
<213> Artificial Sequence

<220>
<221> source
<222> 1..27
<223> /mol_type="unassigned DNA"
      /note="Oligonucleotide sense probe designed on
      the basis of MLH1 gene sequence for c.883A>C site"
      /organism="Artificial Sequence"

<400> 129
aaaacacaca cccattcctg tacctca                                          27

<210> 130
<211> 28
<212> DNA
<213> Artificial Sequence

<220>
<221> source
<222> 1..28
<223> /mol_type="unassigned DNA"
      /note="Oligonucleotide antisense probe designed on
      the basis of MLH1 gene sequence for c.883A>C site"
      /organism="Artificial Sequence"

<400> 130
tcttggttga ggagtttggt gctacatt                                          28


<210> 131
<211> 33
<212> DNA
<213> Artificial Sequence

<220>
<221> source
<222> 1..33
<223> /mol_type="unassigned DNA"
      /note="Oligonucleotide sense probe designed on
      the basis of MLH1 gene sequence for c.884-2A>C site"
      /organism="Artificial Sequence"

<400> 131
tctaaggtaa ttgttctctc ttattttcct gac                                    33


<210> 132
<211> 28
<212> DNA
<213> Artificial Sequence

<220>
<221> source
<222> 1..28
<223> /mol_type="unassigned DNA"
      /note="Oligonucleotide antisense probe designed on
      the basis of MLH1 gene sequence for c.884-2A>C site"
      /organism="Artificial Sequence"

<400> 132
tcgacattct ggggactgat ttctaaac                                          28


<210> 133
<211> 22
<212> DNA
<213> Artificial Sequence

<220>
<221> source

<222> 1..22
<223> /mol_type="unassigned DNA"
      /note="Oligonucleotide sense probe designed on
      the basis of MLH1 gene sequence for c.1013A>G site"
      /organism="Artificial Sequence"

<400> 133
gagagcaagc tcctggcctc ca                                                22


<210> 134
<211> 25
<212> DNA
<213> Artificial Sequence

<220>
<221> source
<222> 1..25
<223> /mol_type="unassigned DNA"
      /note="Oligonucleotide antisense probe designed on
      the basis of MLH1 gene sequence for c.1013A>G site"
      /organism="Artificial Sequence"

<400> 134
ctgggtgaag tacatgctgg aggaa                                             25


<210> 135
<211> 26
<212> DNA
<213> Artificial Sequence

<220>
<221> source
<222> 1..26
<223> /mol_type="unassigned DNA"
      /note="Oligonucleotide sense probe designed on
      the basis of MLH1 gene sequence for c.1038G>C site"
      /organism="Artificial Sequence"

<400> 135
aattgctcca ggatgtactt caccca                                            26


<210> 136
<211> 22
<212> DNA
<213> Artificial Sequence

<220>
<221> source
<222> 1..22
<223> /mol_type="unassigned DNA"
      /note="Oligonucleotide antisense probe designed on
      the basis of MLH1 gene sequence for c.1038G>C site"
      /organism="Artificial Sequence"

<400> 136

agggcgcttc tcatccagct ac                                                22

<210> 137
<211> 25
<212> DNA
<213> Artificial Sequence

<220>
<221> source
<222> 1..25
<223> /mol_type="unassigned DNA"
      /note="Oligonucleotide sense probe designed on
      the basis of MLH1 gene sequence for c.1151T>A site"
      /organism="Artificial Sequence"

<400> 137
gataaggtct atgcccacca gatgg                                             25

<210> 138
<211> 25
<212> DNA
<213> Artificial Sequence

<220>
<221> source
<222> 1..25
<223> /mol_type="unassigned DNA"
      /note="Oligonucleotide antisense probe designed on
      the basis of MLH1 gene sequence for c.1151T>A site"
      /organism="Artificial Sequence"

<400> 138
agattcccgg gaacagaagc ttgat                                             25

<210> 139
<211> 25
<212> DNA
<213> Artificial Sequence

<220>
<221> source
<222> 1..25
<223> /mol_type="unassigned DNA"
      /note="Oligonucleotide sense probe designed on
      the basis of MLH1 gene sequence for c.1166G>A site"
      /organism="Artificial Sequence"

<400> 139
caccagatgg ttcgtacaga ttccc                                             25

<210> 140
<211> 27
<212> DNA
<213> Artificial Sequence

<220>
<221> source
<222> 1..27
<223> /mol_type="unassigned DNA"
      /note="Oligonucleotide antisense probe designed on
      the basis of MLH1 gene sequence for c.1166G>A site"
      /organism="Artificial Sequence"

<400> 140
tgcacaaatg catcaagctt ctgttcc                                          27


<210> 141
<211> 22
<212> DNA
<213> Artificial Sequence

<220>
<221> source
<222> 1..22
<223> /mol_type="unassigned DNA"
      /note="Oligonucleotide sense probe designed on
      the basis of MLH1 gene sequence for c.1217G>A site"
      /organism="Artificial Sequence"

<400> 141
cctctgagca aacccctgtc ca                                               22


<210> 142
<211> 22
<212> DNA
<213> Artificial Sequence

<220>
<221> source
<222> 1..22
<223> /mol_type="unassigned DNA"
      /note="Oligonucleotide antisense probe designed on
      the basis of MLH1 gene sequence for c.1217G>A site"
      /organism="Artificial Sequence"

<400> 142
tgtgacaatg gcctggggct ga                                               22


<210> 143
<211> 26
<212> DNA
<213> Artificial Sequence

<220>
<221> source
<222> 1..26
<223> /mol_type="unassigned DNA"
      /note="Oligonucleotide sense probe designed on
      the basis of MLH1 gene sequence for c.1252delGA site"

/organism="Artificial Sequence"

<400> 143
aggccattgt cacagaggat aagaca                                          26


<210> 144
<211> 26
<212> DNA
<213> Artificial Sequence

<220>
<221> source
<222> 1..26
<223> /mol_type="unassigned DNA"
      /note="Oligonucleotide antisense probe designed on
      the basis of MLH1 gene sequence for c.1252delGA site"
      /organism="Artificial Sequence"

<400> 144
ctgccaagcc ctgccactag aaatat                                          26


<210> 145
<211> 20
<212> DNA
<213> Artificial Sequence

<220>
<221> source
<222> 1..20
<223> /mol_type="unassigned DNA"
      /note="Oligonucleotide sense probe designed on
      the basis of MLH1 gene sequence for c.1321G>A site"
      /organism="Artificial Sequence"

<400> 145
tcccaccccc tgctgaagtg                                                 20


<210> 146
<211> 25
<212> DNA
<213> Artificial Sequence

<220>
<221> source
<222> 1..25
<223> /mol_type="unassigned DNA"
      /note="Oligonucleotide antisense probe designed on
      the basis of MLH1 gene sequence for c.1321G>A site"
      /organism="Artificial Sequence"

<400> 146
ccctcgaagc tctgattttt ggcag                                           25


<210> 147

<211> 22
<212> DNA
<213> Artificial Sequence

<220>
<221> source
<222> 1..22
<223> /mol_type="unassigned DNA"
      /note="Oligonucleotide sense probe designed on
      the basis of MLH1 gene sequence for c.1409+1G>C site"
      /organism="Artificial Sequence"

<400> 147
gacctacttc cagcaacccc ag                                              22


<210> 148
<211> 26
<212> DNA
<213> Artificial Sequence

<220>
<221> source
<222> 1..26
<223> /mol_type="unassigned DNA"
      /note="Oligonucleotide antisense probe designed on
      the basis of MLH1 gene sequence for c.1409+1G>C site"
      /organism="Artificial Sequence"

<400> 148
gcctgtactt ttcccaaaag gccata                                          26


<210> 149
<211> 36
<212> DNA
<213> Artificial Sequence

<220>
<221> source
<222> 1..36
<223> /mol_type="unassigned DNA"
      /note="Oligonucleotide sense probe designed on
      the basis of MLH1 gene sequence for c.1421G>A site"
      /organism="Artificial Sequence"

<400> 149
ctccacttcc ttttcttcat tgcagaaaga gacatc                               36


<210> 150
<211> 29
<212> DNA
<213> Artificial Sequence

<220>
<221> source
<222> 1..29

<223> /mol_type="unassigned DNA"
      /note="Oligonucleotide antisense probe designed on
      the basis of MLH1 gene sequence for c.1421G>A site"
      /organism="Artificial Sequence"

<400> 150
attctgatgt ggaaatggtg gaagatgat                                    29


<210> 151
<211> 28
<212> DNA
<213> Artificial Sequence

<220>
<221> source
<222> 1..28
<223> /mol_type="unassigned DNA"
      /note="Oligonucleotide sense probe designed on
      the basis of MLH1 gene sequence for c.1474G>A site"
      /organism="Artificial Sequence"

<400> 151
ggaagatgat tcgcgaaagg aaatgact                                     28


<210> 152
<211> 21
<212> DNA
<213> Artificial Sequence

<220>
<221> source
<222> 1..21
<223> /mol_type="unassigned DNA"
      /note="Oligonucleotide antisense probe designed on
      the basis of MLH1 gene sequence for c.1474G>A site"
      /organism="Artificial Sequence"

<400> 152
ttctccgggg ggtacaagct g                                            21


<210> 153
<211> 21
<212> DNA
<213> Artificial Sequence

<220>
<221> source
<222> 1..21
<223> /mol_type="unassigned DNA"
      /note="Oligonucleotide sense probe designed on
      the basis of MLH1 gene sequence for site c.1489dupCTGA CTG CAG
      CTT GTA CCC CCC "
      /organism="Artificial Sequence"

<400> 153

tgactgcagc ttgtaccccc c                                                21

<210> 154
<211> 23
<212> DNA
<213> Artificial Sequence

<220>
<221> source
<222> 1..23
<223> /mol_type="unassigned DNA"
      /note="Oligonucleotide antisense probe designed on
      the basis of MLH1 gene sequence for c.1489dupC site"
      /organism="Artificial Sequence"

<400> 154
gttaatgatc cttctccggg ggg                                              23

<210> 155
<211> 21
<212> DNA
<213> Artificial Sequence

<220>
<221> source
<222> 1..21
<223> /mol_type="unassigned DNA"
      /note="Oligonucleotide sense probe designed on
      the basis of MLH1 gene sequence for c.1490insC site"
      /organism="Artificial Sequence"

<400> 155
gactgcagct tgtacccccc g                                                21

<210> 156
<211> 30
<212> DNA
<213> Artificial Sequence

<220>
<221> source
<222> 1..30
<223> /mol_type="unassigned DNA"
      /note="Oligonucleotide antisense probe designed on
      the basis of MLH1 gene sequence for c.1490insC site"
      /organism="Artificial Sequence"

<400> 156
aaaacactag tgaggttaat gatccttctc                                       30

<210> 157
<211> 27
<212> DNA
<213> Artificial Sequence

<220>
<221> source
<222> 1..27
<223> /mol_type="unassigned DNA"
      /note="Oligonucleotide sense probe designed on
      the basis of MLH1 gene sequence for c.1517T>C site"
      /organism="Artificial Sequence"

<400> 157
ggagaaggat cattaacctc actagtg                                          27


<210> 158
<211> 30
<212> DNA
<213> Artificial Sequence

<220>
<221> source
<222> 1..30
<223> /mol_type="unassigned DNA"
      /note="Oligonucleotide antisense probe designed on
      the basis of MLH1 gene sequence for c.1517T>C site"
      /organism="Artificial Sequence"

<400> 158
tggtcattaa tttcttcctg gagactcaaa                                       30


<210> 159
<211> 30
<212> DNA
<213> Artificial Sequence

<220>
<221> source
<222> 1..30
<223> /mol_type="unassigned DNA"
      /note="Oligonucleotide sense probe designed on
      the basis of MLH1 gene sequence for c.1528C>T site"
      /organism="Artificial Sequence"

<400> 159
atcattaacg tcactagtgt tttgagtctc                                       30


<210> 160
<211> 28
<212> DNA
<213> Artificial Sequence

<220>
<221> source
<222> 1..28
<223> /mol_type="unassigned DNA"
      /note="Oligonucleotide antisense probe designed on
      the basis of MLH1 gene sequence for c.1528C>T site"

/organism="Artificial Sequence"

<400> 160
tcatgtccct gctcattaat ttcttcct                                          28


<210> 161
<211> 24
<212> DNA
<213> Artificial Sequence

<220>
<221> source
<222> 1..24
<223> /mol_type="unassigned DNA"
      /note="Oligonucleotide sense probe designed on
      the basis of MLH1 gene sequence for c.1558+14G>A site"
      /organism="Artificial Sequence"

<400> 161
gggacatgag ggtacgtaaa cgct                                              24


<210> 162
<211> 21
<212> DNA
<213> Artificial Sequence

<220>
<221> source
<222> 1..21
<223> /mol_type="unassigned DNA"
      /note="Oligonucleotide antisense probe designed on
      the basis of MLH1 gene sequence for c.1558+14G>A site"
      /organism="Artificial Sequence"

<400> 162
ctatgcatcc caggcaggcc a                                                 21


<210> 163
<211> 31
<212> DNA
<213> Artificial Sequence

<220>
<221> source
<222> 1..31
<223> /mol_type="unassigned DNA"
      /note="Oligonucleotide sense probe designed on
      the basis of MLH1 gene sequence for c.1569G>T site"
      /organism="Artificial Sequence"

<400> 163
gttttatttt ttgttttgca gttctccggg a                                      31


<210> 164

<211> 25
<212> DNA
<213> Artificial Sequence

<220>
<221> source
<222> 1..25
<223> /mol_type="unassigned DNA"
      /note="Oligonucleotide antisense probe designed on
      the basis of MLH1 gene sequence for c.1569G>T site"
      /organism="Artificial Sequence"

<400> 164
ccacgaagga gtggttatgc aacat                                          25


<210> 165
<211> 30
<212> DNA
<213> Artificial Sequence

<220>
<221> source
<222> 1..30
<223> /mol_type="unassigned DNA"
      /note="Oligonucleotide sense probe designed on
      the basis of MLH1 gene sequence for c.1625A>T site"
      /organism="Artificial Sequence"

<400> 165
gctgtgtgaa tcctcagtgg ggcttggcac                                     30


<210> 166
<211> 29
<212> DNA
<213> Artificial Sequence

<220>
<221> source
<222> 1..29
<223> /mol_type="unassigned DNA"
      /note="Oligonucleotide antisense probe designed on
      the basis of MLH1 gene sequence for c.1625A>T site"
      /organism="Artificial Sequence"

<400> 166
tgttgagaag gtataacttg gtttgatgc                                      29


<210> 167
<211> 28
<212> DNA
<213> Artificial Sequence

<220>
<221> source
<222> 1..28

<223> /mol_type="unassigned DNA"
      /note="Oligonucleotide sense probe designed on
      the basis of MLH1 gene sequence for c.1646T>C site"
      /organism="Artificial Sequence"

<400> 167
ttggcacagc atcaaaccaa gttatacc                                          28


<210> 168
<211> 29
<212> DNA
<213> Artificial Sequence

<220>
<221> source
<222> 1..29
<223> /mol_type="unassigned DNA"
      /note="Oligonucleotide antisense probe designed on
      the basis of MLH1 gene sequence for c.1646T>C site"
      /organism="Artificial Sequence"

<400> 168
ggatttacct aagcttggtg gtgttgaga                                         29


<210> 169
<211> 28
<212> DNA
<213> Artificial Sequence

<220>
<221> source
<222> 1..28
<223> /mol_type="unassigned DNA"
      /note="Oligonucleotide sense probe designed on
      the basis of MLH1 gene sequence for c.1649T>C site"
      /organism="Artificial Sequence"

<400> 169
gcacagcatc aaaccaagtt ataccttc                                          28


<210> 170
<211> 27
<212> DNA
<213> Artificial Sequence

<220>
<221> source
<222> 1..27
<223> /mol_type="unassigned DNA"
      /note="Oligonucleotide antisense probe designed on
      the basis of MLH1 gene sequence for c.1649T>C site"
      /organism="Artificial Sequence"

<400> 170
ctgatttacc taagcttggt ggtgttg                                           27

<210> 171
<211> 30
<212> DNA
<213> Artificial Sequence

<220>
<221> source
<222> 1..30
<223> /mol_type="unassigned DNA"
      /note="Oligonucleotide sense probe designed on
      the basis of MLH1 gene sequence for c.1652A>C site"
      /organism="Artificial Sequence"

<400> 171
cacagcatca aaccaagtta taccttctca                                        30


<210> 172
<211> 26
<212> DNA
<213> Artificial Sequence

<220>
<221> source
<222> 1..26
<223> /mol_type="unassigned DNA"
      /note="Oligonucleotide antisense probe designed on
      the basis of MLH1 gene sequence for c.1652A>C site"
      /organism="Artificial Sequence"

<400> 172
agctgattta cctaagcttg gtggtg                                            26


<210> 173
<211> 30
<212> DNA
<213> Artificial Sequence

<220>
<221> source
<222> 1..30
<223> /mol_type="unassigned DNA"
      /note="Oligonucleotide sense probe designed on
      the basis of MLH1 gene sequence for c.1658delCCA site"
      /organism="Artificial Sequence"

<400> 173
atcaaaccaa gttatacctt ctcaacacca                                        30


<210> 174
<211> 27
<212> DNA
<213> Artificial Sequence

<220>
<221> source
<222> 1..27
<223> /mol_type="unassigned DNA"
      /note="Oligonucleotide antisense probe designed on
      the basis of MLH1 gene sequence for c.1658delCCA site"
      /organism="Artificial Sequence"

<400> 174
tcagctgatt tacctaaggt tggtggt                                          27


<210> 175
<211> 31
<212> DNA
<213> Artificial Sequence

<220>
<221> source
<222> 1..31
<223> /mol_type="unassigned DNA"
      /note="Oligonucleotide sense probe designed on
      the basis of MLH1 gene sequence for c.1668-19A>G site"
      /organism="Artificial Sequence"

<400> 175
tcaagcatga attcagcttt tccttaaagt c                                     31


<210> 176
<211> 35
<212> DNA
<213> Artificial Sequence

<220>
<221> source
<222> 1..35
<223> /mol_type="unassigned DNA"
      /note="Oligonucleotide antisense probe designed on
      the basis of MLH1 gene sequence for c.1668-19A>G site"
      /organism="Artificial Sequence"

<400> 176
ggtagaacac ttcttcactg aaaataaaaa tgaag                                 35


<210> 177
<211> 38
<212> DNA
<213> Artificial Sequence

<220>
<221> source
<222> 1..38
<223> /mol_type="unassigned DNA"
      /note="Oligonucleotide sense probe designed on
      the basis of MLH1 gene sequence for c.1672G>T site"
      /organism="Artificial Sequence"

<400> 177
cttttcctta aagtcacttc atttttattt tcagtgaa                                38

<210> 178
<211> 34
<212> DNA
<213> Artificial Sequence

<220>
<221> source
<222> 1..34
<223> /mol_type="unassigned DNA"
      /note="Oligonucleotide antisense probe designed on
      the basis of MLH1 gene sequence for c.1672G>T site"
      /organism="Artificial Sequence"

<400> 178
gcaaaatcat aaatgagtat ctggtagaac agtt                                    34

<210> 179
<211> 28
<212> DNA
<213> Artificial Sequence

<220>
<221> source
<222> 1..28
<223> /mol_type="unassigned DNA"
      /note="Oligonucleotide sense probe designed on
      the basis of MLH1 gene sequence for c.1693A>T site"
      /organism="Artificial Sequence"

<400> 179
gagtgaagaa ctgttctacc agatactc                                           28

<210> 180
<211> 35
<212> DNA
<213> Artificial Sequence

<220>
<221> source
<222> 1..35
<223> /mol_type="unassigned DNA"
      /note="Oligonucleotide antisense probe designed on
      the basis of MLH1 gene sequence for c.1693A>T site"
      /organism="Artificial Sequence"

<400> 180
taacctgaga acaccaaaat tggcaaaatc ataaa                                   35

<210> 181
<211> 34

<212> DNA
<213> Artificial Sequence

<220>
<221> source
<222> 1..34
<223> /mol_type="unassigned DNA"
      /note="Oligonucleotide sense probe designed on
      the basis of MLH1 gene sequence for c.1721T>C site"
      /organism="Artificial Sequence"

<400> 181
atactcattt atgattttgc caattttggt gttc                                34


<210> 182
<211> 30
<212> DNA
<213> Artificial Sequence

<220>
<221> source
<222> 1..30
<223> /mol_type="unassigned DNA"
      /note="Oligonucleotide antisense probe designed on
      the basis of MLH1 gene sequence for c.1721T>C site"
      /organism="Artificial Sequence"

<400> 182
tgaaaaggat ctaaacttac cgataacctg                                     30


<210> 183
<211> 31
<212> DNA
<213> Artificial Sequence

<220>
<221> source
<222> 1..31
<223> /mol_type="unassigned DNA"
      /note="Oligonucleotide sense probe designed on
      the basis of MLH1 gene sequence for c.1731G>A site"
      /organism="Artificial Sequence"

<400> 183
attttgccaa ttttggtgtt ctcaggttat c                                   31


<210> 184
<211> 36
<212> DNA
<213> Artificial Sequence

<220>
<221> source
<222> 1..36
<223> /mol_type="unassigned DNA"

/note="Oligonucleotide antisense probe designed on
the basis of MLH1 gene sequence for c.1731G>A site"
/organism="Artificial Sequence"

<400> 184
gttgaaatta cagaagtgaa aaggatctaa acttac                                36

<210> 185
<211> 26
<212> DNA
<213> Artificial Sequence

<220>
<221> source
<222> 1..26
<223> /mol_type="unassigned DNA"
/note="Oligonucleotide sense probe designed on
the basis of MLH1 gene sequence for c.1733A>G site"
/organism="Artificial Sequence"

<400> 185
ccttcatgtt cttgcttctt cctagg                                26

<210> 186
<211> 31
<212> DNA
<213> Artificial Sequence

<220>
<221> source
<222> 1..31
<223> /mol_type="unassigned DNA"
/note="Oligonucleotide antisense probe designed on
the basis of MLH1 gene sequence for c.1733A>G site"
/organism="Artificial Sequence"

<400> 186
aagcttggca aggtcaaaga gcggtgctgg c                                31

<210> 187
<211> 21
<212> DNA
<213> Artificial Sequence

<220>
<221> source
<222> 1..21
<223> /mol_type="unassigned DNA"
/note="Oligonucleotide sense probe designed on
the basis of MLH1 gene sequence for c.1744C>G site"
/organism="Artificial Sequence"

<400> 187
tcttcctagg agccagcacc g                                21

<210> 188
<211> 25
<212> DNA
<213> Artificial Sequence

<220>
<221> source
<222> 1..25
<223> /mol_type="unassigned DNA"
      /note="Oligonucleotide antisense probe designed on
      the basis of MLH1 gene sequence for c.1744C>G site"
      /organism="Artificial Sequence"

<400> 188
cttgccatgc ttgccttaga tagtc                                          25


<210> 189
<211> 22
<212> DNA
<213> Artificial Sequence

<220>
<221> source
<222> 1..22
<223> /mol_type="unassigned DNA"
      /note="Oligonucleotide sense probe designed on
      the basis of MLH1 gene sequence for c.1756G>C site"
      /organism="Artificial Sequence"

<400> 189
gccagcaccg ctctttgacc tt                                             22


<210> 190
<211> 25
<212> DNA
<213> Artificial Sequence

<220>
<221> source
<222> 1..25
<223> /mol_type="unassigned DNA"
      /note="Oligonucleotide antisense probe designed on
      the basis of MLH1 gene sequence for c.1756G>C site"
      /organism="Artificial Sequence"

<400> 190
ttgccttaga tagtgcagag agtgg                                          25


<210> 191
<211> 24
<212> DNA
<213> Artificial Sequence

<220>

<221> source
<222> 1..24
<223> /mol_type="unassigned DNA"
      /note="Oligonucleotide sense probe designed on
      the basis of MLH1 gene sequence for c.1766C>A site"
      /organism="Artificial Sequence"

<400> 191
cgctctttga ccttgccatg cttg                                              24


<210> 192
<211> 24
<212> DNA
<213> Artificial Sequence

<220>
<221> source
<222> 1..24
<223> /mol_type="unassigned DNA"
      /note="Oligonucleotide antisense probe designed on
      the basis of MLH1 gene sequence for c.1766C>A site"
      /organism="Artificial Sequence"

<400> 192
cagccactct ctggactatc taag                                              24


<210> 193
<211> 24
<212> DNA
<213> Artificial Sequence

<220>
<221> source
<222> 1..24
<223> /mol_type="unassigned DNA"
      /note="Oligonucleotide sense probe designed on
      the basis of MLH1 gene sequence for c.1783delAG site"
      /organism="Artificial Sequence"

<400> 193
caggcactct ctggactatc taag                                              24


<210> 194
<211> 23
<212> DNA
<213> Artificial Sequence

<220>
<221> source
<222> 1..23
<223> /mol_type="unassigned DNA"
      /note="Oligonucleotide antisense probe designed on
      the basis of MLH1 gene sequence for c.1783delAG site"
      /organism="Artificial Sequence"

<400> 194
cttcctctgt ccagccactc tct                                              23


<210> 195
<211> 22
<212> DNA
<213> Artificial Sequence

<220>
<221> source
<222> 1..22
<223> /mol_type="unassigned DNA"
      /note="Oligonucleotide sense probe designed on
      the basis of MLH1 gene sequence for c.1808C>G site"
      /organism="Artificial Sequence"

<400> 195
ggctggacag aggaagatgg tc                                               22


<210> 196
<211> 29
<212> DNA
<213> Artificial Sequence

<220>
<221> source
<222> 1..29
<223> /mol_type="unassigned DNA"
      /note="Oligonucleotide antisense probe designed on
      the basis of MLH1 gene sequence for c.1808C>G site"
      /organism="Artificial Sequence"

<400> 196
caacaatgta ttcagcaagt ccttctttg                                        29


<210> 197
<211> 24
<212> DNA
<213> Artificial Sequence

<220>
<221> source
<222> 1..24
<223> /mol_type="unassigned DNA"
      /note="Oligonucleotide sense probe designed on
      the basis of MLH1 gene sequence for c.1820T>A site"
      /organism="Artificial Sequence"

<400> 197
aggaagatgg tcgcaaagaa ggac                                             24


<210> 198
<211> 32
<212> DNA

<213> Artificial Sequence

<220>
<221> source
<222> 1..32
<223> /mol_type="unassigned DNA"
      /note="Oligonucleotide antisense probe designed on
      the basis of MLH1 gene sequence for c.1820T>A site"
      /organism="Artificial Sequence"

<400> 198
tcttcttcag taactcaaca atgtattcag ca                                    32


<210> 199
<211> 32
<212> DNA
<213> Artificial Sequence

<220>
<221> source
<222> 1..32
<223> /mol_type="unassigned DNA"
      /note="Oligonucleotide sense probe designed on
      the basis of MLH1 gene sequence for c.1846delAAG site"
      /organism="Artificial Sequence"

<400> 199
ttgctgaata cattgttgag tttctgaaga ag                                    32


<210> 200
<211> 25
<212> DNA
<213> Artificial Sequence

<220>
<221> source
<222> 1..25
<223> /mol_type="unassigned DNA"
      /note="Oligonucleotide antisense probe designed on
      the basis of MLH1 gene sequence for c.1846delAAG site"
      /organism="Artificial Sequence"

<400> 200
gtctgcaagc atctcagcct tcttc                                            25


<210> 201
<211> 32
<212> DNA
<213> Artificial Sequence

<220>
<221> source
<222> 1..32
<223> /mol_type="unassigned DNA"
      /note="Oligonucleotide sense probe designed on

the basis of MLH1 gene sequence for c.1852_1853delAAinsGC site"
/organism="Artificial Sequence"

<400> 201
ttgctgaata cattgttgag tttctgaaga ag 32


<210> 202
<211> 26
<212> DNA
<213> Artificial Sequence

<220>
<221> source
<222> 1..26
<223> /mol_type="unassigned DNA"
/note="Oligonucleotide antisense probe designed on
the basis of MLH1 gene sequence for c.1852_1853delAAinsGC site"
/organism="Artificial Sequence"

<400> 202
agaaatagtc tgcaagcatc tcagcc 26


<210> 203
<211> 32
<212> DNA
<213> Artificial Sequence

<220>
<221> source
<222> 1..32
<223> /mol_type="unassigned DNA"
/note="Oligonucleotide sense probe designed on
the basis of MLH1 gene sequence for c.1852delAAG site"
/organism="Artificial Sequence"

<400> 203
ttgctgaata cattgttgag tttctgaaga ag 32


<210> 204
<211> 25
<212> DNA
<213> Artificial Sequence

<220>
<221> source
<222> 1..25
<223> /mol_type="unassigned DNA"
/note="Oligonucleotide antisense probe designed on
the basis of MLH1 gene sequence for c.1852delAAG site"
/organism="Artificial Sequence"

<400> 204
gtctgcaagc atctcagcct tcttc 25

<210> 205
<211> 32
<212> DNA
<213> Artificial Sequence

<220>
<221> source
<222> 1..32
<223> /mol_type="unassigned DNA"
      /note="Oligonucleotide sense probe designed on
      the basis of MLH1 gene sequence for c.1853A>C/G site"
      /organism="Artificial Sequence"

<400> 205
tgctgaatac attgttgagt ttctgaagaa ga                                    32


<210> 206
<211> 26
<212> DNA
<213> Artificial Sequence

<220>
<221> source
<222> 1..26
<223> /mol_type="unassigned DNA"
      /note="Oligonucleotide antisense probe designed on
      the basis of MLH1 gene sequence for c.1853A>C/G site"
      /organism="Artificial Sequence"

<400> 206
agaaatagtc tgcaagcatc tcagcc                                           26


<210> 207
<211> 27
<212> DNA
<213> Artificial Sequence

<220>
<221> source
<222> 1..27
<223> /mol_type="unassigned DNA"
      /note="Oligonucleotide sense probe designed on
      the basis of MLH1 gene sequence for c.1865T>A site"
      /organism="Artificial Sequence"

<400> 207
agtttctgaa gaagaaggct gagatgc                                          27


<210> 208
<211> 31
<212> DNA
<213> Artificial Sequence

<220>
<221> source

<222> 1..31
<223> /mol_type="unassigned DNA"
      /note="Oligonucleotide antisense probe designed on
      the basis of MLH1 gene sequence for c.1865T>A site"
      /organism="Artificial Sequence"

<400> 208
ctcatcaatt tccaaagaga aatagtctgc a                                    31


<210> 209
<211> 24
<212> DNA
<213> Artificial Sequence

<220>
<221> source
<222> 1..24
<223> /mol_type="unassigned DNA"
      /note="Oligonucleotide sense probe designed on
      the basis of MLH1 gene sequence for c.1918C>T site"
      /organism="Artificial Sequence"

<400> 209
ggttcccttc gtgcttgcag gaaa                                            24


<210> 210
<211> 25
<212> DNA
<213> Artificial Sequence

<220>
<221> source
<222> 1..25
<223> /mol_type="unassigned DNA"
      /note="Oligonucleotide antisense probe designed on
      the basis of MLH1 gene sequence for c.1918C>T site"
      /organism="Artificial Sequence"

<400> 210
ggcacatagt tgtcaatcag aaggg                                           25


<210> 211
<211> 28
<212> DNA
<213> Artificial Sequence

<220>
<221> source
<222> 1..28
<223> /mol_type="unassigned DNA"
      /note="Oligonucleotide sense probe designed on
      the basis of MLH1 gene sequence for c.1937A>G site"
      /organism="Artificial Sequence"

<400> 211

attggattac cccttctgat tgacaact                                          28


<210> 212
<211> 21
<212> DNA
<213> Artificial Sequence

<220>
<221> source
<222> 1..21
<223> /mol_type="unassigned DNA"
      /note="Oligonucleotide antisense probe designed on
      the basis of MLH1 gene sequence for c.1937A>G site"
      /organism="Artificial Sequence"

<400> 212
agtccgtcca aagggggcac a                                                 21


<210> 213
<211> 29
<212> DNA
<213> Artificial Sequence

<220>
<221> source
<222> 1..29
<223> /mol_type="unassigned DNA"
      /note="Oligonucleotide sense probe designed on
      the basis of MLH1 gene sequence for c.1942C>T site"
      /organism="Artificial Sequence"

<400> 213
gattacccct tctgattgtc aactatgtg                                         29


<210> 214
<211> 21
<212> DNA
<213> Artificial Sequence

<220>
<221> source
<222> 1..21
<223> /mol_type="unassigned DNA"
      /note="Oligonucleotide antisense probe designed on
      the basis of MLH1 gene sequence for c.1942C>T site"
      /organism="Artificial Sequence"

<400> 214
taggcagtcc ctccaaaggg g                                                 21


<210> 215
<211> 26
<212> DNA
<213> Artificial Sequence

<220>
<221> source
<222> 1..26
<223> /mol_type="unassigned DNA"
      /note="Oligonucleotide sense probe designed on
      the basis of MLH1 gene sequence for c.1943C>T site"
      /organism="Artificial Sequence"

<400> 215
accccttctg attgtcaact atgtgc                                          26


<210> 216
<211> 22
<212> DNA
<213> Artificial Sequence

<220>
<221> source
<222> 1..22
<223> /mol_type="unassigned DNA"
      /note="Oligonucleotide antisense probe designed on
      the basis of MLH1 gene sequence for c.1943C>T site"
      /organism="Artificial Sequence"

<400> 216
gataggcagt cgctccaaag gg                                              22


<210> 217
<211> 20
<212> DNA
<213> Artificial Sequence

<220>
<221> source
<222> 1..20
<223> /mol_type="unassigned DNA"
      /note="Oligonucleotide sense probe designed on
      the basis of MLH1 gene sequence for c.1958T>G site"
      /organism="Artificial Sequence"

<400> 217
   tgtgccccct ttggacggac                                                  20


<210> 218
<211> 26
<212> DNA
<213> Artificial Sequence

<220>
<221> source
<222> 1..26
<223> /mol_type="unassigned DNA"
      /note="Oligonucleotide antisense probe designed on
      the basis of MLH1 gene sequence for c.1958T>G site"

/organism="Artificial Sequence"

<400> 218
tggctagtcg aagaatgaag ataggc                                          26


<210> 219
<211> 21
<212> DNA
<213> Artificial Sequence

<220>
<221> source
<222> 1..21
<223> /mol_type="unassigned DNA"
      /note="Oligonucleotide sense probe designed on
      the basis of MLH1 gene sequence for c.1959G>T site"
      /organism="Artificial Sequence"

<400> 219
tgtgccccct ttggagggac t                                               21


<210> 220
<211> 26
<212> DNA
<213> Artificial Sequence

<220>
<221> source
<222> 1..26
<223> /mol_type="unassigned DNA"
      /note="Oligonucleotide antisense probe designed on
      the basis of MLH1 gene sequence for c.1959G>T site"
      /organism="Artificial Sequence"

<400> 220
gtggctagtc gaagaatgaa gatagg                                          26


<210> 221
<211> 19
<212> DNA
<213> Artificial Sequence

<220>
<221> source
<222> 1..19
<223> /mol_type="unassigned DNA"
      /note="Oligonucleotide sense probe designed on
      the basis of MLH1 gene sequence for c.1961C>T site"
      /organism="Artificial Sequence"

<400> 221
ccgcctttgg agggactgc                                                  19


<210> 222

<211> 28
<212> DNA
<213> Artificial Sequence

<220>
<221> source
<222> 1..28
<223> /mol_type="unassigned DNA"
      /note="Oligonucleotide antisense probe designed on
      the basis of MLH1 gene sequence for c.1961C>T site"
      /organism="Artificial Sequence"

<400> 222
ctcagtggct agtcgaagaa tgaagata                                        28


<210> 223
<211> 20
<212> DNA
<213> Artificial Sequence

<220>
<221> source
<222> 1..20
<223> /mol_type="unassigned DNA"
      /note="Oligonucleotide sense probe designed on
      the basis of MLH1 gene sequence for c.1963A>G site"
      /organism="Artificial Sequence"

<400> 223
cgcctttgga gggactgcct                                                  20


<210> 224
<211> 26
<212> DNA
<213> Artificial Sequence

<220>
<221> source
<222> 1..26
<223> /mol_type="unassigned DNA"
      /note="Oligonucleotide antisense probe designed on
      the basis of MLH1 gene sequence for c.1963A>G site"
      /organism="Artificial Sequence"

<400> 224
ctcagtggct agtcgaagaa tgaaga                                           26


<210> 225
<211> 21
<212> DNA
<213> Artificial Sequence

<220>
<221> source
<222> 1..21

<223> /mol_type="unassigned DNA"
      /note="Oligonucleotide sense probe designed on
      the basis of MLH1 gene sequence for c.1964T>C site"
      /organism="Artificial Sequence"

<400> 225
ccccattgga gggactgcct a                                                21


<210> 226
<211> 25
<212> DNA
<213> Artificial Sequence

<220>
<221> source
<222> 1..25
<223> /mol_type="unassigned DNA"
      /note="Oligonucleotide antisense probe designed on
      the basis of MLH1 gene sequence for c.1964T>C site"
      /organism="Artificial Sequence"

<400> 226
ctcagtggct agtcgaagaa tgaag                                            25


<210> 227
<211> 25
<212> DNA
<213> Artificial Sequence

<220>
<221> source
<222> 1..25
<223> /mol_type="unassigned DNA"
      /note="Oligonucleotide sense probe designed on
      the basis of MLH1 gene sequence for c.1976G>A/C site"
      /organism="Artificial Sequence"

<400> 227
gtgggactgc ctatcttcat tcttc                                            25


<210> 228
<211> 26
<212> DNA
<213> Artificial Sequence

<220>
<221> source
<222> 1..26
<223> /mol_type="unassigned DNA"
      /note="Oligonucleotide antisense probe designed on
      the basis of MLH1 gene sequence for c.1976G>A/C site"
      /organism="Artificial Sequence"

<400> 228
cttgatcact gacctcagtg gctagt                                           26

<210> 229
<211> 26
<212> DNA
<213> Artificial Sequence

<220>
<221> source
<222> 1..26
<223> /mol_type="unassigned DNA"
      /note="Oligonucleotide sense probe designed on
      the basis of MLH1 gene sequence for c.1984A>C site"
      /organism="Artificial Sequence"

<400> 229
tgcctatctt cattcttcga ctagcc                                          26


<210> 230
<211> 28
<212> DNA
<213> Artificial Sequence

<220>
<221> source
<222> 1..28
<223> /mol_type="unassigned DNA"
      /note="Oligonucleotide antisense probe designed on
      the basis of MLH1 gene sequence for c.1984A>C site"
      /organism="Artificial Sequence"

<400> 230
ttagtatctg cttgatcact gacctcag                                        28


<210> 231
<211> 28
<212> DNA
<213> Artificial Sequence

<220>
<221> source
<222> 1..28
<223> /mol_type="unassigned DNA"
      /note="Oligonucleotide sense probe designed on
      the basis of MLH1 gene sequence for c.1989G>T site"
      /organism="Artificial Sequence"

<400> 231
ctatcttcat tcttcgacta gccactga                                        28


<210> 232
<211> 29
<212> DNA
<213> Artificial Sequence

<220>
<221> source
<222> 1..29
<223> /mol_type="unassigned DNA"
      /note="Oligonucleotide antisense probe designed on
      the basis of MLH1 gene sequence for c.1989G>T site"
      /organism="Artificial Sequence"

<400> 232
aaatgcttag tatctgcttg atcactgac                                         29


<210> 233
<211> 29
<212> DNA
<213> Artificial Sequence

<220>
<221> source
<222> 1..29
<223> /mol_type="unassigned DNA"
      /note="Oligonucleotide sense probe designed on
      the basis of MLH1 gene sequence for c.2027T>G site"
      /organism="Artificial Sequence"

<400> 233
ggacgaagaa aaggaatgtt ttgaaagcc                                         29


<210> 234
<211> 30
<212> DNA
<213> Artificial Sequence

<220>
<221> source
<222> 1..30
<223> /mol_type="unassigned DNA"
      /note="Oligonucleotide antisense probe designed on
      the basis of MLH1 gene sequence for c.2027T>G site"
      /organism="Artificial Sequence"

<400> 234
atggaataga acatagcgca ttctttactg                                        30


<210> 235
<211> 30
<212> DNA
<213> Artificial Sequence

<220>
<221> source
<222> 1..30
<223> /mol_type="unassigned DNA"
      /note="Oligonucleotide sense probe designed on
      the basis of MLH1 gene sequence for c.2040C>A_SE site"
      /organism="Artificial Sequence"

<400> 235
ggaatgtttt gaaagcctca gtaaagaatg                                        30


<210> 236
<211> 26
<212> DNA
<213> Artificial Sequence

<220>
<221> source
<222> 1..26
<223> /mol_type="unassigned DNA"
      /note="Oligonucleotide antisense probe designed on
      the basis of MLH1 gene sequence for c.2040C>A site"
      /organism="Artificial Sequence"

<400> 236
tgctaccgga tggaatagaa catagc                                            26


<210> 237
<211> 30
<212> DNA
<213> Artificial Sequence

<220>
<221> source
<222> 1..30
<223> /mol_type="unassigned DNA"
      /note="Oligonucleotide sense probe designed on
      the basis of MLH1 gene sequence for c.2041G>A site"
      /organism="Artificial Sequence"

<400> 237
gaatgttttg aaagcctcag taaagaatgc                                        30


<210> 238
<211> 28
<212> DNA
<213> Artificial Sequence

<220>
<221> source
<222> 1..28
<223> /mol_type="unassigned DNA"
      /note="Oligonucleotide antisense probe designed on
      the basis of MLH1 gene sequence for c.2041G>A site"
      /organism="Artificial Sequence"

<400> 238
tactgcttgc ggatggaata gaacatag                                          28


<210> 239
<211> 30

<212> DNA
<213> Artificial Sequence

<220>
<221> source
<222> 1..30
<223> /mol_type="unassigned DNA"
      /note="Oligonucleotide sense probe designed on
      the basis of MLH1 gene sequence for c.2059C>T site"
      /organism="Artificial Sequence"

<400> 239
agtaaagaat gcgctatgtt ctattccatc                                        30


<210> 240
<211> 25
<212> DNA
<213> Artificial Sequence

<220>
<221> source
<222> 1..25
<223> /mol_type="unassigned DNA"
      /note="Oligonucleotide antisense probe designed on
      the basis of MLH1 gene sequence for c.2059C>T site"
      /organism="Artificial Sequence"

<400> 240
gactcctcag atatgtactg cttcc                                             25


<210> 241
<211> 25
<212> DNA
<213> Artificial Sequence

<220>
<221> source
<222> 1..25
<223> /mol_type="unassigned DNA"
      /note="Oligonucleotide sense probe designed on
      the basis of MLH1 gene sequence for c.2066A>G site"
      /organism="Artificial Sequence"

<400> 241
gctatgttct attccatccg gaagc                                             25


<210> 242
<211> 24
<212> DNA
<213> Artificial Sequence

<220>
<221> source
<222> 1..24
<223> /mol_type="unassigned DNA"

/note="Oligonucleotide antisense probe designed on
the basis of MLH1 gene sequence for c.2066A>G site"
/organism="Artificial Sequence"

<400> 242
agggtcgact cctcagatat gtac                                              24


<210> 243
<211> 20
<212> DNA
<213> Artificial Sequence

<220>
<221> source
<222> 1..20
<223> /mol_type="unassigned DNA"
/note="Oligonucleotide sense probe designed on
the basis of MLH1 gene sequence for c.2103G>C site"
/organism="Artificial Sequence"

<400> 243
tcgaccctct caggccagca                                                   20


<210> 244
<211> 23
<212> DNA
<213> Artificial Sequence

<220>
<221> source
<222> 1..23
<223> /mol_type="unassigned DNA"
/note="Oligonucleotide antisense probe designed on
the basis of MLH1 gene sequence for c.2103G>C"
/organism="Artificial Sequence"

<400> 244
gccagtctgc atcaccactg tac                                               23


<210> 245
<211> 20
<212> DNA
<213> Artificial Sequence

<220>
<221> source
<222> 1..20
<223> /mol_type="unassigned DNA"
/note="Oligonucleotide sense probe designed on
the basis of MLH1 gene sequence for c.2103+1G>A site"
/organism="Artificial Sequence"

<400> 245
cgaccctctc aggccagcag                                                   20

<210> 246
<211> 24
<212> DNA
<213> Artificial Sequence

<220>
<221> source
<222> 1..24
<223> /mol_type="unassigned DNA"
      /note="Oligonucleotide antisense probe designed on
      the basis of MLH1 gene sequence for c.2103+1G>A site"
      /organism="Artificial Sequence"

<400> 246
gtgccagtct gcatcaccac tgta                                              24


<210> 247
<211> 25
<212> DNA
<213> Artificial Sequence

<220>
<221> source
<222> 1..25
<223> /mol_type="unassigned DNA"
      /note="Oligonucleotide sense probe designed on
      the basis of MLH1 gene sequence for c.2146G>A site"
      /organism="Artificial Sequence"

<400> 247
cattgcaaac tcctggaagt ggact                                             25


<210> 248
<211> 28
<212> DNA
<213> Artificial Sequence

<220>
<221> source
<222> 1..28
<223> /mol_type="unassigned DNA"
      /note="Oligonucleotide antisense probe designed on
      the basis of MLH1 gene sequence for c.2146G>A site"
      /organism="Artificial Sequence"

<400> 248
cgcaaggctt tatagacaat gtgttcca                                          28


<210> 249
<211> 25
<212> DNA
<213> Artificial Sequence

<220>

<221> source
<222> 1..25
<223> /mol_type="unassigned DNA"
      /note="Oligonucleotide sense probe designed on
      the basis of MLH1 gene sequence for c.2152C>T site"
      /organism="Artificial Sequence"

<400> 249
aaactcctgg aagtggactg tggaa                                          25


<210> 250
<211> 27
<212> DNA
<213> Artificial Sequence

<220>
<221> source
<222> 1..27
<223> /mol_type="unassigned DNA"
      /note="Oligonucleotide antisense probe designed on
      the basis of MLH1 gene sequence for c.2152C>T site"
      /organism="Artificial Sequence"

<400> 250
gtgagcgcaa ggctttatag acaatgt                                        27


<210> 251
<211> 33
<212> DNA
<213> Artificial Sequence

<220>
<221> source
<222> 1..33
<223> /mol_type="unassigned DNA"
      /note="Oligonucleotide sense probe designed on
      the basis of MLH1 gene sequence for c.2223del11 site"
      /organism="Artificial Sequence"

<400> 251
tcctaaagat ttcacagaag atcgaaatat cct                                 33



<210> 252
<211> 29
<212> DNA
<213> Artificial Sequence

<220>
<221> source
<222> 1..29
<223> /mol_type="unassigned DNA"
      /note="Oligonucleotide antisense probe designed on
      the basis of MLH1 gene sequence for c.2223del11 site"
      /organism="Artificial Sequence"

<400> 252
ctcaaagact ttgtatagat caggcaggt                                          29

<210> 253
<211> 27
<212> DNA
<213> Artificial Sequence

<220>
<221> source
<222> 1..27
<223> /mol_type="unassigned DNA"
      /note="Oligonucleotide sense probe designed on
      the basis of MLH1 gene sequence for c.2252A>G site"
      /organism="Artificial Sequence"

<400> 253
agcttgctaa cctgcctgat ctataca                                            27


<210> 254
<211> 35
<212> DNA
<213> Artificial Sequence

<220>
<221> source
<222> 1..35
<223> /mol_type="unassigned DNA"
      /note="Oligonucleotide antisense probe designed on
      the basis of MLH1 gene sequence for c.2252A>G site"
      /organism="Artificial Sequence"

<400> 254
gcataaataa ccatatttaa cacctctcaa agact                                    35


<210> 255
<211> 22
<212> DNA
<213> Artificial Sequence

<220>
<221> source
<222> 1..22
<223> /mol_type="unassigned DNA"
      /note="Oligonucleotide sense probe designed on
      the basis of MSH2 gene sequence for c.-118T>C site"
      /organism="Artificial Sequence"

<400> 255
gaaacgcacc cctggaagct ga                                                 22


<210> 256
<211> 19
<212> DNA

<213> Artificial Sequence

<220>
<221> source
<222> 1..19
<223> /mol_type="unassigned DNA"
      /note="Oligonucleotide antisense probe designed on
      the basis of MSH2 gene sequence for c.-118T>C site"
      /organism="Artificial Sequence"

<400> 256
gccacggcga ccacaccca                                                    19


<210> 257
<211> 23
<212> DNA
<213> Artificial Sequence

<220>
<221> source
<222> 1..23
<223> /mol_type="unassigned DNA"
      /note="Oligonucleotide sense probe designed on
      the basis of MSH2 gene sequence for c.4G>A site"
      /organism="Artificial Sequence"

<400> 257
ggaggtgagg aggtttcgac atg                                               23


<210> 258
<211> 19
<212> DNA
<213> Artificial Sequence

<220>
<221> source
<222> 1..19
<223> /mol_type="unassigned DNA"
      /note="Oligonucleotide antisense probe designed on
      the basis of MSH2 gene sequence for c.4G>A site"
      /organism="Artificial Sequence"

<400> 258
gtctccttcc gctgcaccg                                                    19


<210> 259
<211> 39
<212> DNA
<213> Artificial Sequence

<220>
<221> source
<222> 1..39
<223> /mol_type="unassigned DNA"
      /note="Oligonucleotide sense probe designed on

the basis of MSH2 gene sequence for c.226C>T site"
/organism="Artificial Sequence"

<400> 259
gtaatgtact tttttttttt ttaaggagca aagaatctg 39

<210> 260
<211> 38
<212> DNA
<213> Artificial Sequence

<220>
<221> source
<222> 1..38
<223> /mol_type="unassigned DNA"
/note="Oligonucleotide antisense probe designed on
the basis of MSH2 gene sequence for c.226C>T site"
/organism="Artificial Sequence"

<400> 260
aaaagattca aaattcattt tactaagcac aacactct 38

<210> 261
<211> 33
<212> DNA
<213> Artificial Sequence

<220>
<221> source
<222> 1..33
<223> /mol_type="unassigned DNA"
/note="Oligonucleotide sense probe designed on
the basis of MSH2 gene sequence for c.339G>A site"
/organism="Artificial Sequence"

<400> 261
ttataagaat agagctggta ataaggcatc caa 33

<210> 262
<211> 35
<212> DNA
<213> Artificial Sequence

<220>
<221> source
<222> 1..35
<223> /mol_type="unassigned DNA"
/note="Oligonucleotide antisense probe designed on
the basis of MSH2 gene sequence for c.339G>A site"
/organism="Artificial Sequence"

<400> 262
ataattacct tatatgccaa ataccaatca ttctc 35

<210> 263
<211> 34
<212> DNA
<213> Artificial Sequence

<220>
<221> source
<222> 1..34
<223> /mol_type="unassigned DNA"
      /note="Oligonucleotide sense probe designed on
      the basis of MSH2 gene sequence for c.380A>G site"
      /organism="Artificial Sequence"

<400> 263
taaaatttta tttttactta cgcttctcct ggca                                34


<210> 264
<211> 30
<212> DNA
<213> Artificial Sequence

<220>
<221> source
<222> 1..30
<223> /mol_type="unassigned DNA"
      /note="Oligonucleotide antisense probe designed on
      the basis of MSH2 gene sequence for c.380A>G site"
      /organism="Artificial Sequence"

<400> 264
ccaaagagaa tgtcttcaaa ctgagagaga                                     30


<210> 265
<211> 31
<212> DNA
<213> Artificial Sequence

<220>
<221> source
<222> 1..31
<223> /mol_type="unassigned DNA"
      /note="Oligonucleotide sense probe designed on
      the basis of MSH2 gene sequence for c.435T>G site"
      /organism="Artificial Sequence"

<400> 265
tgtttggtaa caatgatatg tcagcttcca t                                   31


<210> 266
<211> 25
<212> DNA
<213> Artificial Sequence

<220>
<221> source

<222> 1..25
<223> /mol_type="unassigned DNA"
      /note="Oligonucleotide antisense probe designed on
      the basis of MSH2 gene sequence for c.435T>G site"
      /organism="Artificial Sequence"

<400> 266
cggacatttt aacacccaca acacc                                          25


<210> 267
<211> 24
<212> DNA
<213> Artificial Sequence

<220>
<221> source
<222> 1..24
<223> /mol_type="unassigned DNA"
      /note="Oligonucleotide sense probe designed on
      the basis of MSH2 gene sequence for c.499G>C site"
      /organism="Artificial Sequence"

<400> 267
agacaggttg gagttgggta tgtg                                           24


<210> 268
<211> 28
<212> DNA
<213> Artificial Sequence

<220>
<221> source
<222> 1..28
<223> /mol_type="unassigned DNA"
      /note="Oligonucleotide antisense probe designed on
      the basis of MSH2 gene sequence for c.499G>C site"
      /organism="Artificial Sequence"

<400> 268
cacagtccta gttacctctg tatggaat                                       28


<210> 269
<211> 25
<212> DNA
<213> Artificial Sequence

<220>
<221> source
<222> 1..25
<223> /mol_type="unassigned DNA"
      /note="Oligonucleotide sense probe designed on
      the basis of MSH2 gene sequence for c.505A>G site"
      /organism="Artificial Sequence"

<400> 269

ggttggagtt gggtatgtgg attcc                                          25


<210> 270
<211> 29
<212> DNA
<213> Artificial Sequence

<220>
<221> source
<222> 1..29
<223> /mol_type="unassigned DNA"
      /note="Oligonucleotide antisense probe designed on
      the basis of MSH2 gene sequence for c.505A>G site"
      /organism="Artificial Sequence"

<400> 270
gaattcacac tgtcctagtt tcctctgta                                      29


<210> 271
<211> 27
<212> DNA
<213> Artificial Sequence

<220>
<221> source
<222> 1..27
<223> /mol_type="unassigned DNA"
      /note="Oligonucleotide sense probe designed on
      the basis of MSH2 gene sequence for c.518T>C site"
      /organism="Artificial Sequence"

<400> 271
ggtatgtgga ttccatacag aggaaac                                        27


<210> 272
<211> 27
<212> DNA
<213> Artificial Sequence

<220>
<221> source
<222> 1..27
<223> /mol_type="unassigned DNA"
      /note="Oligonucleotide antisense probe designed on
      the basis of MSH2 gene sequence for c.518T>C site"
      /organism="Artificial Sequence"

<400> 272
ggtatgtgga ttcgatacag aggaaac                                        27


<210> 273
<211> 30
<212> DNA
<213> Artificial Sequence

<220>
<221> source
<222> 1..30
<223> /mol_type="unassigned DNA"
      /note="Oligonucleotide sense probe designed on
      the basis of MSH2 gene sequence for c.560T>C site"
      /organism="Artificial Sequence"

<400> 273
aattccctca taatgatcag ttctccaatc                                        30


<210> 274
<211> 24
<212> DNA
<213> Artificial Sequence

<220>
<221> source
<222> 1..24
<223> /mol_type="unassigned DNA"
      /note="Oligonucleotide antisense probe designed on
      the basis of MSH2 gene sequence for c.560T>C site"
      /organism="Artificial Sequence"

<400> 274
ccaatctgga tgacgagagc ctca                                              24


<210> 275
<211> 24
<212> DNA
<213> Artificial Sequence

<220>
<221> source
<222> 1..24
<223> /mol_type="unassigned DNA"
      /note="Oligonucleotide sense probe designed on
      the basis of MSH2 gene sequence for c.593A>G site"
      /organism="Artificial Sequence"

<400> 275
tcctcatgca gattggacca aagg                                              24


<210> 276
<211> 25
<212> DNA
<213> Artificial Sequence

<220>
<221> source
<222> 1..25
<223> /mol_type="unassigned DNA"
      /note="Oligonucleotide antisense probe designed on
      the basis of MSH2 gene sequence for c.593A>G site"

/organism="Artificial Sequence"

<400> 276
agtctctcct ccgggtaaaa cacat                                          25


<210> 277
<211> 26
<212> DNA
<213> Artificial Sequence

<220>
<221> source
<222> 1..26
<223> /mol_type="unassigned DNA"
      /note="Oligonucleotide sense probe designed on
      the basis of MSH2 gene sequence for c.595T>C site"
      /organism="Artificial Sequence"

<400> 277
tcgtcatcca gattggacca aaggaa                                         26


<210> 278
<211> 24
<212> DNA
<213> Artificial Sequence

<220>
<221> source
<222> 1..24
<223> /mol_type="unassigned DNA"
      /note="Oligonucleotide antisense probe designed on
      the basis of MSH2 gene sequence for c.595T>C site"
      /organism="Artificial Sequence"

<400> 278
cagtctctcc tccgggtaaa acac                                           24


<210> 279
<211> 31
<212> DNA
<213> Artificial Sequence

<220>
<221> source
<222> 1..31
<223> /mol_type="unassigned DNA"
      /note="Oligonucleotide sense probe designed on
      the basis of MSH2 gene sequence for c.687delA site"
      /organism="Artificial Sequence"

<400> 279
gaggaggaat tgtgatcaca gaaagaaaaa a                                   31


<210> 280

<211> 36
<212> DNA
<213> Artificial Sequence

<220>
<221> source
<222> 1..36
<223> /mol_type="unassigned DNA"
      /note="Oligonucleotide antisense probe designed on
      the basis of MSH2 gene sequence for c.687delA site"
      /organism="Artificial Sequence"

<400> 280
ctgataaatg tcttttgtgg ataagtcagc tttttt                                36


<210> 281
<211> 40
<212> DNA
<213> Artificial Sequence

<220>
<221> source
<222> 1..40
<223> /mol_type="unassigned DNA"
      /note="Oligonucleotide sense probe designed on
      the basis of MSH2 gene sequence for c.806C>T site"
      /organism="Artificial Sequence"

<400> 281
cttcgatttt taaattctta attttaggtt gcagtttcat                            40


<210> 282
<211> 30
<212> DNA
<213> Artificial Sequence

<220>
<221> source
<222> 1..30
<223> /mol_type="unassigned DNA"
      /note="Oligonucleotide antisense probe designed on
      the basis of MSH2 gene sequence for c.806C>T site"
      /organism="Artificial Sequence"

<400> 282
agttctaaaa acttgattac cgcagacagt                                       30


<210> 283
<211> 34
<212> DNA
<213> Artificial Sequence

<220>
<221> source
<222> 1..34

<223> /mol_type="unassigned DNA"
      /note="Oligonucleotide sense probe designed on
      the basis of MSH2 gene sequence for c.862C>T site"
      /organism="Artificial Sequence"

<400> 283
tttagaactc ttatcagatg attcgaactt tgga                                   34


<210> 284
<211> 30
<212> DNA
<213> Artificial Sequence

<220>
<221> source
<222> 1..30
<223> /mol_type="unassigned DNA"
      /note="Oligonucleotide antisense probe designed on
      the basis of MSH2 gene sequence for c.862C>T site"
      /organism="Artificial Sequence"

<400> 284
ggctgaagtc aaaagtagtc tgttcaaact                                        30


<210> 285
<211> 30
<212> DNA
<213> Artificial Sequence

<220>
<221> source
<222> 1..30
<223> /mol_type="unassigned DNA"
      /note="Oligonucleotide sense probe designed on
      the basis of MSH2 gene sequence for c.892C>T site"
      /organism="Artificial Sequence"

<400> 285
cagtttgaac tcactacttt tgacttcagc                                        30


<210> 286
<211> 32
<212> DNA
<213> Artificial Sequence

<220>
<221> source
<222> 1..32
<223> /mol_type="unassigned DNA"
      /note="Oligonucleotide antisense probe designed on
      the basis of MSH2 gene sequence for c.892C>T site"
      /organism="Artificial Sequence"

<400> 286
tctgactgct gcaatatcca atttcatata ct                                     32

<210> 287
<211> 26
<212> DNA
<213> Artificial Sequence

<220>
<221> source
<222> 1..26
<223> /mol_type="unassigned DNA"
      /note="Oligonucleotide sense probe designed on
      the basis of MSH2 gene sequence for c.942+3A>T site"
      /organism="Artificial Sequence"

<400> 287
gtcagagccc ttaacgtttt tcaggt                                          26


<210> 288
<211> 10
<212> DNA
<213> Artificial Sequence

<220>
<221> source
<222> 1..10
<223> /mol_type="unassigned DNA"
      /note="Lack of functional AS probe for site c.942+3A>T"
      /organism="Artificial Sequence"

<400> 288
nnnnnnnnnn                                                            10


<210> 289
<211> 29
<212> DNA
<213> Artificial Sequence

<220>
<221> source
<222> 1..29
<223> /mol_type="unassigned DNA"
      /note="Oligonucleotide sense probe designed on
      the basis of MSH2 gene sequence for c.965G>A site"
      /organism="Artificial Sequence"

<400> 289
ttactagggt tctgttgaag ataccactg                                       29


<210> 290
<211> 22
<212> DNA
<213> Artificial Sequence

<220>

<221> source
<222> 1..22
<223> /mol_type="unassigned DNA"
      /note="Oligonucleotide antisense probe designed on
      the basis of MSH2 gene sequence for c.965G>A site"
      /organism="Artificial Sequence"

<400> 290
caaggcagcc agagactgag ag                                              22


<210> 291
<211> 21
<212> DNA
<213> Artificial Sequence

<220>
<221> source
<222> 1..21
<223> /mol_type="unassigned DNA"
      /note="Oligonucleotide sense probe designed on
      the basis of MSH2 gene sequence for c.984C>T site"
      /organism="Artificial Sequence"

<400> 291
tggctctcag tctctggctg c                                               21


<210> 292
<211> 29
<212> DNA
<213> Artificial Sequence

<220>
<221> source
<222> 1..29
<223> /mol_type="unassigned DNA"
      /note="Oligonucleotide antisense probe designed on
      the basis of MSH2 gene sequence for c.984C>T site"
      /organism="Artificial Sequence"

<400> 292
ccttgagggg ttttacactt attcagcaa                                       29


<210> 293
<211> 25
<212> DNA
<213> Artificial Sequence

<220>
<221> source
<222> 1..25
<223> /mol_type="unassigned DNA"
      /note="Oligonucleotide sense probe designed on
      the basis of MSH2 gene sequence for c.998G>A site"
      /organism="Artificial Sequence"

<400> 293
tctctggctg ccttgctgaa taagt                                          25


<210> 294
<211> 30
<212> DNA
<213> Artificial Sequence

<220>
<221> source
<222> 1..30
<223> /mol_type="unassigned DNA"
      /note="Oligonucleotide antisense probe designed on
      the basis of MSH2 gene sequence for c.998G>A site"
      /organism="Artificial Sequence"

<400> 294
ttaagaagtc tttgtccttg aggggtttta                                     30


<210> 295
<211> 51
<212> DNA
<213> Artificial Sequence

<220>
<221> source
<222> 1..51
<223> /mol_type="unassigned DNA"
      /note="Oligonucleotide sense probe designed on
      the basis of MSH2 gene sequence for c.1077-10T>C site"
      /organism="Artificial Sequence"

<400> 295
gttgataaat tttaatttta tactaaaata ttttacatta attcaagtta a             51


<210> 296
<211> 37
<212> DNA
<213> Artificial Sequence

<220>
<221> source
<222> 1..37
<223> /mol_type="unassigned DNA"
      /note="Oligonucleotide antisense probe designed on
      the basis of MSH2 gene sequence for c.1077-10T>C site"
      /organism="Artificial Sequence"

<400> 296
ttctacaaaa gcttccacta aattcaatct gaaataa                             37


<210> 297
<211> 52
<212> DNA

<213> Artificial Sequence

<220>
<221> source
<222> 1..52
<223> /mol_type="unassigned DNA"
      /note="Oligonucleotide sense probe designed on
      the basis of MSH2 gene sequence for c.1077A>T site"
      /organism="Artificial Sequence"

<400> 297
ttttaatttt atactaaaat attttacatt aattcaagtt aatttatttc ag          52


<210> 298
<211> 33
<212> DNA
<213> Artificial Sequence

<220>
<221> source
<222> 1..33
<223> /mol_type="unassigned DNA"
      /note="Oligonucleotide antisense probe designed on
      the basis of MSH2 gene sequence for c.1077A>T site"
      /organism="Artificial Sequence"

<400> 298
tgcatcttct acaaaagctt ccactaaatt caa                               33


<210> 299
<211> 30
<212> DNA
<213> Artificial Sequence

<220>
<221> source
<222> 1..30
<223> /mol_type="unassigned DNA"
      /note="Oligonucleotide sense probe designed on
      the basis of MSH2 gene sequence for c.1147C>T site"
      /organism="Artificial Sequence"

<400> 299
aggcagactt tacaagatga tttacttcgt                                   30


<210> 300
<211> 25
<212> DNA
<213> Artificial Sequence

<220>
<221> source
<222> 1..25
<223> /mol_type="unassigned DNA"
      /note="Oligonucleotide antisense probe designed on

the basis of MSH2 gene sequence for c.1147C>T site"
      /organism="Artificial Sequence"

<400> 300
gcaagtcggt taagatctgg gaatc                                          25


<210> 301
<211> 30
<212> DNA
<213> Artificial Sequence

<220>
<221> source
<222> 1..30
<223> /mol_type="unassigned DNA"
      /note="Oligonucleotide sense probe designed on
      the basis of MSH2 gene sequence for c.1165C>T site"
      /organism="Artificial Sequence"

<400> 301
gatttacttc gtcgattccc agatcttaac                                     30


<210> 302
<211> 28
<212> DNA
<213> Artificial Sequence

<220>
<221> source
<222> 1..28
<223> /mol_type="unassigned DNA"
      /note="Oligonucleotide antisense probe designed on
      the basis of MSH2 gene sequence for c.1165C>T site"
      /organism="Artificial Sequence"

<400> 302
gcttctcttt gaaacttctt ggcaagtc                                       28


<210> 303
<211> 26
<212> DNA
<213> Artificial Sequence

<220>
<221> source
<222> 1..26
<223> /mol_type="unassigned DNA"
      /note="Oligonucleotide sense probe designed on
      the basis of MSH2 gene sequence for c.1168C>T site"
      /organism="Artificial Sequence"

<400> 303
ttcgtcgatt cccagatctt aaccga                                         26

<210> 304
<211> 28
<212> DNA
<213> Artificial Sequence

<220>
<221> source
<222> 1..28
<223> /mol_type="unassigned DNA"
      /note="Oligonucleotide antisense probe designed on
      the basis of MSH2 gene sequence for c.1168C>T site"
      /organism="Artificial Sequence"

<400> 304
gctgcttgtc tttgaaactt cttggcaa                                          28


<210> 305
<211> 32
<212> DNA
<213> Artificial Sequence

<220>
<221> source
<222> 1..32
<223> /mol_type="unassigned DNA"
      /note="Oligonucleotide sense probe designed on
      the basis of MSH2 gene sequence for c.1216C>T site"
      /organism="Artificial Sequence"

<400> 305
aaagacaagc agcaaactta caagatagtt ac                                     32


<210> 306
<211> 32
<212> DNA
<213> Artificial Sequence

<220>
<221> source
<222> 1..32
<223> /mol_type="unassigned DNA"
      /note="Oligonucleotide antisense probe designed on
      the basis of MSH2 gene sequence for c.1216C>T site"
      /organism="Artificial Sequence"

<400> 306
attaggtagt tgatttatac cctgatagag tc                                     32


<210> 307
<211> 30
<212> DNA
<213> Artificial Sequence

<220>
<221> source

<222> 1..30
<223> /mol_type="unassigned DNA"
      /note="Oligonucleotide sense probe designed on
      the basis of MSH2 gene sequence for c.1226delAG site"
      /organism="Artificial Sequence"

<400> 307
caaacttaca agattgttac cgactctatc                                        30


<210> 308
<211> 33
<212> DNA
<213> Artificial Sequence

<220>
<221> source
<222> 1..33
<223> /mol_type="unassigned DNA"
      /note="Oligonucleotide antisense probe designed on
      the basis of MSH2 gene sequence for c.1226delAG site"
      /organism="Artificial Sequence"

<400> 308
agcctgtata acattagcta gttgatttat acc                                    33


<210> 309
<211> 36
<212> DNA
<213> Artificial Sequence

<220>
<221> source
<222> 1..36
<223> /mol_type="unassigned DNA"
      /note="Oligonucleotide sense probe designed on
      the basis of MSH2 gene sequence for c.1255C>A site"
      /organism="Artificial Sequence"

<400> 309
ctctatcagg gtataaatca actacctaat gttata                                 36


<210> 310
<211> 29
<212> DNA
<213> Artificial Sequence

<220>
<221> source
<222> 1..29
<223> /mol_type="unassigned DNA"
      /note="Oligonucleotide antisense probe designed on
      the basis of MSH2 gene sequence for c.1255C>A site"
      /organism="Artificial Sequence"

<400> 310

cttgttacct tcatgttttt ccagagcct                                          29

<210> 311
<211> 33
<212> DNA
<213> Artificial Sequence

<220>
<221> source
<222> 1..33
<223> /mol_type="unassigned DNA"
      /note="Oligonucleotide sense probe designed on
      the basis of MSH2 gene sequence for c.1373T>G site"
      /organism="Artificial Sequence"

<400> 311
tctccaagat tcaggaaatg atagaaacaa ctt                                    33

<210> 312
<211> 39
<212> DNA
<213> Artificial Sequence

<220>
<221> source
<222> 1..39
<223> /mol_type="unassigned DNA"
      /note="Oligonucleotide antisense probe designed on
      the basis of MSH2 gene sequence for c.1373T>G site"
      /organism="Artificial Sequence"

<400> 312
cttaaattaa aaagtatatt gcatacctga tccatatct                              39

<210> 313
<211> 30
<212> DNA
<213> Artificial Sequence

<220>
<221> source
<222> 1..30
<223> /mol_type="unassigned DNA"
      /note="Oligonucleotide sense probe designed on
      the basis of MSH2 gene sequence for c.1571G>C site"
      /organism="Artificial Sequence"

<400> 313
attccagtgc acagtttgga tattactttc                                        30

<210> 314
<211> 30
<212> DNA
<213> Artificial Sequence

<220>
<221> source
<222> 1..30
<223> /mol_type="unassigned DNA"
      /note="Oligonucleotide antisense probe designed on
      the basis of MSH2 gene sequence for c.1571G>C site"
      /organism="Artificial Sequence"

<400> 314
cgaagcactt tttcttcctt acaggttaca                                        30


<210> 315
<211> 32
<212> DNA
<213> Artificial Sequence

<220>
<221> source
<222> 1..32
<223> /mol_type="unassigned DNA"
      /note="Oligonucleotide sense probe designed on
      the basis of MSH2 gene sequence for c.1654A>C site"
      /organism="Artificial Sequence"

<400> 315
tttaacacca ttcttctgga tatctacagt ac                                     32


<210> 316
<211> 35
<212> DNA
<213> Artificial Sequence

<220>
<221> source
<222> 1..35
<223> /mol_type="unassigned DNA"
      /note="Oligonucleotide antisense probe designed on
      the basis of MSH2 gene sequence for c.1654A>C site"
      /organism="Artificial Sequence"

<400> 316
gttaaaaata taataacgac ttgcaaagct gttgg                                  35


<210> 317
<211> 31
<212> DNA
<213> Artificial Sequence

<220>
<221> source
<222> 1..31
<223> /mol_type="unassigned DNA"
      /note="Oligonucleotide sense probe designed on
      the basis of MSH2 gene sequence for c.1661+12G>A site"

/organism="Artificial Sequence"

<400> 317
ggtgttaaat ttaccaacag gtttgcaagt c                                    31


<210> 318
<211> 43
<212> DNA
<213> Artificial Sequence

<220>
<221> source
<222> 1..43
<223> /mol_type="unassigned DNA"
      /note="Oligonucleotide antisense probe designed on
      the basis of MSH2 gene sequence for c.1661+12G>A site"
      /organism="Artificial Sequence"

<400> 318
ttagagcatt tagggaatta ataaagggtt aaaaatataa taa                       43


<210> 319
<211> 41
<212> DNA
<213> Artificial Sequence

<220>
<221> source
<222> 1..41
<223> /mol_type="unassigned DNA"
      /note="Oligonucleotide sense probe designed on
      the basis of MSH2 gene sequence for c.1666T>C site"
      /organism="Artificial Sequence"

<400> 319
ttaataaaac tgataaaact gttatttcga tttgcagcaa a                         41


<210> 320
<211> 39
<212> DNA
<213> Artificial Sequence

<220>
<221> source
<222> 1..39
<223> /mol_type="unassigned DNA"
      /note="Oligonucleotide antisense probe designed on
      the basis of MSH2 gene sequence for c.1666T>C site"
      /organism="Artificial Sequence"

<400> 320
ctgttttatt tttggtatac tcttcattta aagaagtca                            39


<210> 321

<211> 27
<212> DNA
<213> Artificial Sequence

<220>
<221> source
<222> 1..27
<223> /mol_type="unassigned DNA"
      /note="Oligonucleotide sense probe designed on
      the basis of MSH2 gene sequence for c.1737A>G site"
      /organism="Artificial Sequence"

<400> 321
tgaagaagcc caggatgcca ttgttaa                                          27


<210> 322
<211> 41
<212> DNA
<213> Artificial Sequence

<220>
<221> source
<222> 1..41
<223> /mol_type="unassigned DNA"
      /note="Oligonucleotide antisense probe designed on
      the basis of MSH2 gene sequence for c.1737A>G site"
      /organism="Artificial Sequence"

<400> 322
ttagttctat taagtttacc tgaagaaata ttgacaattt c                          41


<210> 323
<211> 27
<212> DNA
<213> Artificial Sequence

<220>
<221> source
<222> 1..27
<223> /mol_type="unassigned DNA"
      /note="Oligonucleotide sense probe designed on
      the basis of MSH2 gene sequence for c.1738G>T site"
      /organism="Artificial Sequence"

<400> 323
gaagaagccc aggatgccat tgttaaa                                          27


<210> 324
<211> 41
<212> DNA
<213> Artificial Sequence

<220>
<221> source
<222> 1..41

<223> /mol_type="unassigned DNA"
      /note="Oligonucleotide antisense probe designed on
      the basis of MSH2 gene sequence for c.1738G>T site"
      /organism="Artificial Sequence"

<400> 324
attagttcta ttaagtttac ctgaagaaat attgacaatt t                          41


<210> 325
<211> 26
<212> DNA
<213> Artificial Sequence

<220>
<221> source
<222> 1..26
<223> /mol_type="unassigned DNA"
      /note="Oligonucleotide sense probe designed on
      the basis of MSH2 gene sequence for c.1786delAAT site"
      /organism="Artificial Sequence"

<400> 325
gctatgtaga accaatgcag acactc                                           26


<210> 326
<211> 27
<212> DNA
<213> Artificial Sequence

<220>
<221> source
<222> 1..27
<223> /mol_type="unassigned DNA"
      /note="Oligonucleotide antisense probe designed on
      the basis of MSH2 gene sequence for c.1786delAAT site"
      /organism="Artificial Sequence"

<400> 326
aacaccatct agctgagcta acacatc                                          27


<210> 327
<211> 27
<212> DNA
<213> Artificial Sequence

<220>
<221> source
<222> 1..27
<223> /mol_type="unassigned DNA"
      /note="Oligonucleotide sense probe designed on
      the basis of MSH2 gene sequence for c.1787A>G site"
      /organism="Artificial Sequence"

<400> 327
gctatgtaga accaatgcag acactca                                          27

<210> 328
<211> 27
<212> DNA
<213> Artificial Sequence

<220>
<221> source
<222> 1..27
<223> /mol_type="unassigned DNA"
      /note="Oligonucleotide antisense probe designed on
      the basis of MSH2 gene sequence for c.1787A>G site"
      /organism="Artificial Sequence"

<400> 328
acaccatcta gctgagctaa cacatca                                          27


<210> 329
<211> 28
<212> DNA
<213> Artificial Sequence

<220>
<221> source
<222> 1..28
<223> /mol_type="unassigned DNA"
      /note="Oligonucleotide sense probe designed on
      the basis of MSH2 gene sequence for c.1799C>T site"
      /organism="Artificial Sequence"

<400> 329
ccaatgcaga gactcaatga tgtgttag                                         28


<210> 330
<211> 26
<212> DNA
<213> Artificial Sequence

<220>
<221> source
<222> 1..26
<223> /mol_type="unassigned DNA"
      /note="Oligonucleotide antisense probe designed on
      the basis of MSH2 gene sequence for c.1799C>T site"
      /organism="Artificial Sequence"

<400> 330
caaagctgac aacaccatct agctga                                           26


<210> 331
<211> 28
<212> DNA
<213> Artificial Sequence

<220>
<221> source
<222> 1..28
<223> /mol_type="unassigned DNA"
      /note="Oligonucleotide sense probe designed on
      the basis of MSH2 gene sequence for c.1807G>A site"
      /organism="Artificial Sequence"

<400> 331
gacactcaat gatgtgttag gtcagcta                                          28


<210> 332
<211> 25
<212> DNA
<213> Artificial Sequence

<220>
<221> source
<222> 1..25
<223> /mol_type="unassigned DNA"
      /note="Oligonucleotide antisense probe designed on
      the basis of MSH2 gene sequence for c.1807G>A site"
      /organism="Artificial Sequence"

<400> 332
acgtgagcaa aggtgacaac agcat                                             25


<210> 333
<211> 28
<212> DNA
<213> Artificial Sequence

<220>
<221> source
<222> 1..28
<223> /mol_type="unassigned DNA"
      /note="Oligonucleotide sense probe designed on
      the basis of MSH2 gene sequence for c.1861C>T site"
      /organism="Artificial Sequence"

<400> 333
gtcaaatcga gcacctgttc catatgta                                          28


<210> 334
<211> 26
<212> DNA
<213> Artificial Sequence

<220>
<221> source
<222> 1..26
<223> /mol_type="unassigned DNA"
      /note="Oligonucleotide antisense probe designed on
      the basis of MSH2 gene sequence for c.1861C>T site"
      /organism="Artificial Sequence"

<400> 334
ttgtcctttc tccaaaatgg ctggtc                                          26


<210> 335
<211> 25
<212> DNA
<213> Artificial Sequence

<220>
<221> source
<222> 1..25
<223> /mol_type="unassigned DNA"
      /note="Oligonucleotide sense probe designed on
      the basis of MSH2 gene sequence for c.1865C>T site"
      /organism="Artificial Sequence"

<400> 335
cgagcacctg ttccatatgt acgac                                           25


<210> 336
<211> 29
<212> DNA
<213> Artificial Sequence

<220>
<221> source
<222> 1..29
<223> /mol_type="unassigned DNA"
      /note="Oligonucleotide antisense probe designed on
      the basis of MSH2 gene sequence for c.1865C>T site"
      /organism="Artificial Sequence"

<400> 336
ttcttccttg tcctttctcc aaaatggct                                       29


<210> 337
<211> 37
<212> DNA
<213> Artificial Sequence

<220>
<221> source
<222> 1..37
<223> /mol_type="unassigned DNA"
      /note="Oligonucleotide sense probe designed on
      the basis of MSH2 gene sequence for c.1906G>C site"
      /organism="Artificial Sequence"

<400> 337
cattttggag aaaggacaag gaagaattat attaaaa                              37


<210> 338
<211> 26

<212> DNA
<213> Artificial Sequence

<220>
<221> source
<222> 1..26
<223> /mol_type="unassigned DNA"
      /note="Oligonucleotide antisense probe designed on
      the basis of MSH2 gene sequence for c.1906G>C site"
      /organism="Artificial Sequence"

<400> 338
aacttcaaca caagcatgcc tggatg                                          26


<210> 339
<211> 41
<212> DNA
<213> Artificial Sequence

<220>
<221> source
<222> 1..41
<223> /mol_type="unassigned DNA"
      /note="Oligonucleotide sense probe designed on
      the basis of MSH2 gene sequence for c.2006-6T>C site"
      /organism="Artificial Sequence"

<400> 339
cacaaagaag tttaaaatct tgctttctga tataatttgt t                         41


<210> 340
<211> 26
<212> DNA
<213> Artificial Sequence

<220>
<221> source
<222> 1..26
<223> /mol_type="unassigned DNA"
      /note="Oligonucleotide antisense probe designed on
      the basis of MSH2 gene sequence for c.2006-6T>C site"
      /organism="Artificial Sequence"

<400> 340
atttacctcg catattgggg cctaca                                          26


<210> 341
<211> 28
<212> DNA
<213> Artificial Sequence

<220>
<221> source
<222> 1..28
<223> /mol_type="unassigned DNA"

/note="Oligonucleotide sense probe designed on
the basis of MSH2 gene sequence for c.2064G>A site"
/organism="Artificial Sequence"

<400> 341
ttcgacaaac tggggtgata gtactcat                                          28


<210> 342
<211> 26
<212> DNA
<213> Artificial Sequence

<220>
<221> source
<222> 1..26
<223> /mol_type="unassigned DNA"
/note="Oligonucleotide antisense probe designed on
the basis of MSH2 gene sequence for c.2064G>A site"
/organism="Artificial Sequence"

<400> 342
catggcacaa aacacgcaat ttgggc                                            26


<210> 343
<211> 25
<212> DNA
<213> Artificial Sequence

<220>
<221> source
<222> 1..25
<223> /mol_type="unassigned DNA"
/note="Oligonucleotide sense probe designed on
the basis of MSH2 gene sequence for c.2089T>C site"
/organism="Artificial Sequence"

<400> 343
ggccgaaatt gggtgttttg tgcca                                             25


<210> 344
<211> 25
<212> DNA
<213> Artificial Sequence

<220>
<221> source
<222> 1..25
<223> /mol_type="unassigned DNA"
/note="Oligonucleotide antisense probe designed on
the basis of MSH2 gene sequence for c.2089T>C site"
/organism="Artificial Sequence"

<400> 344
acaatggaca cttctgctga ctcac                                             25

<210> 345
<211> 25
<212> DNA
<213> Artificial Sequence

<220>
<221> source
<222> 1..25
<223> /mol_type="unassigned DNA"
      /note="Oligonucleotide sense probe designed on
      the basis of MSH2 gene sequence for c.2090G>T site"
      /organism="Artificial Sequence"

<400> 345
gccgaaattg ggtgttttgt gccat                                          25


<210> 346
<211> 25
<212> DNA
<213> Artificial Sequence

<220>
<221> source
<222> 1..25
<223> /mol_type="unassigned DNA"
      /note="Oligonucleotide antisense probe designed on
      the basis of MSH2 gene sequence for c.2090G>T site"
      /organism="Artificial Sequence"

<400> 346
cacaatggac acttctgctg actca                                          25


<210> 347
<211> 25
<212> DNA
<213> Artificial Sequence

<220>
<221> source
<222> 1..25
<223> /mol_type="unassigned DNA"
      /note="Oligonucleotide sense probe designed on
      the basis of MSH2 gene sequence for c.2131C>T site"
      /organism="Artificial Sequence"

<400> 347
gtcgattgtg gactgcatct tagcc                                          25


<210> 348
<211> 23
<212> DNA
<213> Artificial Sequence

<220>

<221> source
<222> 1..23
<223> /mol_type="unassigned DNA"
      /note="Oligonucleotide antisense probe designed on
      the basis of MSH2 gene sequence for c.2131C>T site"
      /organism="Artificial Sequence"

<400> 348
ttgactgtca ccagccccta ctc                                              23


<210> 349
<211> 27
<212> DNA
<213> Artificial Sequence

<220>
<221> source
<222> 1..27
<223> /mol_type="unassigned DNA"
      /note="Oligonucleotide sense probe designed on
      the basis of MSH2 gene sequence for c.2168C>T site"
      /organism="Artificial Sequence"

<400> 349
ctggtgagag tcaattgaaa ggagtct                                          27


<210> 350
<211> 28
<212> DNA
<213> Artificial Sequence

<220>
<221> source
<222> 1..28
<223> /mol_type="unassigned DNA"
      /note="Oligonucleotide antisense probe designed on
      the basis of MSH2 gene sequence for c.2168C>T site"
      /organism="Artificial Sequence"

<400> 350
agtttccaac atttcagcca tgaacgtg                                         28


<210> 351
<211> 34
<212> DNA
<213> Artificial Sequence

<220>
<221> source
<222> 1..34
<223> /mol_type="unassigned DNA"
      /note="Oligonucleotide sense probe designed on
      the basis of MSH2 gene sequence for c.2245G>A site"
      /organism="Artificial Sequence"

<400> 351
gtctgcaacc aaagattcat taataatcat agat                                34

<210> 352
<211> 28
<212> DNA
<213> Artificial Sequence

<220>
<221> source
<222> 1..28
<223> /mol_type="unassigned DNA"
      /note="Oligonucleotide antisense probe designed on
      the basis of MSH2 gene sequence for c.2245G>A site"
      /organism="Artificial Sequence"

<400> 352
tcgtaggtac aagttcctct tcccaatt                                       28

<210> 353
<211> 36
<212> DNA
<213> Artificial Sequence

<220>
<221> source
<222> 1..36
<223> /mol_type="unassigned DNA"
      /note="Oligonucleotide sense probe designed on
      the basis of MSH2 gene sequence for c.2251G>A site"
      /organism="Artificial Sequence"

<400> 353
gcaaccaaag atacattaat aatcatagat gaattg                              36

<210> 354
<211> 28
<212> DNA
<213> Artificial Sequence

<220>
<221> source
<222> 1..28
<223> /mol_type="unassigned DNA"
      /note="Oligonucleotide antisense probe designed on
      the basis of MSH2 gene sequence for c.2251G>A site"
      /organism="Artificial Sequence"

<400> 354
gaacttctac ctacgatgga tttgggtt                                       28

<210> 355
<211> 30
<212> DNA

<213> Artificial Sequence

<220>
<221> source
<222> 1..30
<223> /mol_type="unassigned DNA"
      /note="Oligonucleotide sense probe designed on
      the basis of MSH2 gene sequence for c.2500G>A site"
      /organism="Artificial Sequence"

<400> 355
caaagttttg ggattcatgt tgcagagctt                                        30


<210> 356
<211> 30
<212> DNA
<213> Artificial Sequence

<220>
<221> source
<222> 1..30
<223> /mol_type="unassigned DNA"
      /note="Oligonucleotide antisense probe designed on
      the basis of MSH2 gene sequence for c.2500G>A site"
      /organism="Artificial Sequence"

<400> 356
cacactctat tacatgctta gggaaattag                                        30


<210> 357
<211> 27
<212> DNA
<213> Artificial Sequence

<220>
<221> source
<222> 1..27
<223> /mol_type="unassigned DNA"
      /note="Oligonucleotide sense probe designed on
      the basis of MSH2 gene sequence for c.2633delAG site"
      /organism="Artificial Sequence"

<400> 357
caccaaagaa gtgctatctg gaaagag                                           27


<210> 358
<211> 34
<212> DNA
<213> Artificial Sequence

<220>
<221> source
<222> 1..34
<223> /mol_type="unassigned DNA"
      /note="Oligonucleotide antisense probe designed on

the basis of MSH2 gene sequence for c.2633delAG site"
      /organism="Artificial Sequence"

<400> 358
gtttgtcagt ttgttttcat agtttaactt agct                                   34


<210> 359
<211> 31
<212> DNA
<213> Artificial Sequence

<220>
<221> source
<222> 1..31
<223> /mol_type="unassigned DNA"
      /note="Oligonucleotide sense probe designed on
      the basis of MSH2 gene sequence for c.2714C>G site"
      /organism="Artificial Sequence"

<400> 359
ccctttactg aaatgtcaga agaaaacatc a                                      31


<210> 360
<211> 33
<212> DNA
<213> Artificial Sequence

<220>
<221> source
<222> 1..33
<223> /mol_type="unassigned DNA"
      /note="Oligonucleotide antisense probe designed on
      the basis of MSH2 gene sequence for c.2714C>G site"
      /organism="Artificial Sequence"

<400> 360
gctattactt ctgcttttag ctgttttaac ttt                                    33


<210> 361
<211> 25
<212> DNA
<213> Artificial Sequence

<220>
<221> source
<222> 1..25
<223> /mol_type="unassigned DNA"
      /note="Oligonucleotide sense probe designed on
      the basis of MSH6 gene sequence for c.276A>G site"
      /organism="Artificial Sequence"

<400> 361
ttggcaacag ttgtcacttc tcacc                                             25

<210> 362
<211> 24
<212> DNA
<213> Artificial Sequence

<220>
<221> source
<222> 1..24
<223> /mol_type="unassigned DNA"
      /note="Oligonucleotide antisense probe designed on
      the basis of MSH6 gene sequence for c.276A>G site"
      /organism="Artificial Sequence"

<400> 362
catctaggcc caaaccaaat ctcc                                              24


<210> 363
<211> 22
<212> DNA
<213> Artificial Sequence

<220>
<221> source
<222> 1..22
<223> /mol_type="unassigned DNA"
      /note="Oligonucleotide sense probe designed on
      the basis of MSH6 gene sequence for c.431G>T site"
      /organism="Artificial Sequence"

<400> 363
agcgcaacaa ggggctgggt ta                                                22


<210> 364
<211> 33
<212> DNA
<213> Artificial Sequence

<220>
<221> source
<222> 1..33
<223> /mol_type="unassigned DNA"
      /note="Oligonucleotide antisense probe designed on
      the basis of MSH6 gene sequence for c.431G>T site"
      /organism="Artificial Sequence"

<400> 364
tttgaacctg tatatgcctt taaaagcctt ttg                                   33


<210> 365
<211> 26
<212> DNA
<213> Artificial Sequence

<220>
<221> source

<222> 1..26
<223> /mol_type="unassigned DNA"
      /note="Oligonucleotide sense probe designed on
      the basis of MSH6 gene sequence for c.457+52T>A site"
      /organism="Artificial Sequence"

<400> 365
tgtgtttgtg tgtgtgtgtg tgtgtg                                          26


<210> 366
<211> 25
<212> DNA
<213> Artificial Sequence

<220>
<221> source
<222> 1..25
<223> /mol_type="unassigned DNA"
      /note="Oligonucleotide antisense probe designed on
      the basis of MSH6 gene sequence for c.457+52T>A site"
      /organism="Artificial Sequence"

<400> 366
gtctgcctgt ctgtctgttt ctctc                                            25


<210> 367
<211> 41
<212> DNA
<213> Artificial Sequence

<220>
<221> source
<222> 1..41
<223> /mol_type="unassigned DNA"
      /note="Oligonucleotide sense probe designed on
      the basis of MSH6 gene sequence for c.467C>G site"
      /organism="Artificial Sequence"

<400> 367
cccttattgt ttataaatac atttctttct aggttcaaaa t                          41


<210> 368
<211> 23
<212> DNA
<213> Artificial Sequence

<220>
<221> source
<222> 1..23
<223> /mol_type="unassigned DNA"
      /note="Oligonucleotide antisense probe designed on
      the basis of MSH6 gene sequence for c.467C>G site"
      /organism="Artificial Sequence"

<400> 368

gacctcgctt ctgggcttcc ttt                                                23

<210> 369
<211> 25
<212> DNA
<213> Artificial Sequence

<220>
<221> source
<222> 1..25
<223> /mol_type="unassigned DNA"
      /note="Oligonucleotide sense probe designed on
      the basis of MSH6 gene sequence for c.540T>C site"
      /organism="Artificial Sequence"

<400> 369
gtctgcctgt ctgtctgttt ctctc                                              25

<210> 370
<211> 33
<212> DNA
<213> Artificial Sequence

<220>
<221> source
<222> 1..33
<223> /mol_type="unassigned DNA"
      /note=" Oligonucleotide antisense probe designed on
      the basis of MSH6 gene sequence for c.540T>C site"
      /organism="Artificial Sequence"

<400> 370
aaccctctta atcttgtctt tatttaaggc ttc                                     33

<210> 371
<211> 23
<212> DNA
<213> Artificial Sequence

<220>
<221> source
<222> 1..23
<223> /mol_type="unassigned DNA"
      /note="Oligonucleotide sense probe designed on
      the basis of MSH6 gene sequence for c.642C>T site"
      /organism="Artificial Sequence"

<400> 371
ggctggcagg taggcacaac tta                                                23

<210> 372
<211> 36
<212> DNA
<213> Artificial Sequence

<220>
<221> source
<222> 1..36
<223> /mol_type="unassigned DNA"
      /note="Oligonucleotide antisense probe designed on
      the basis of MSH6 gene sequence for c.642C>T site"
      /organism="Artificial Sequence"

<400> 372
ctcaatttca ttatcttctt cactcttatc tgttac                                36


<210> 373
<211> 27
<212> DNA
<213> Artificial Sequence

<220>
<221> source
<222> 1..27
<223> /mol_type="unassigned DNA"
      /note="Oligonucleotide sense probe designed on
      the basis of MSH6 gene sequence for c.1186C>G site"
      /organism="Artificial Sequence"

<400> 373
gatcaccccg attttgatgc atctaca                                          27


<210> 374
<211> 29
<212> DNA
<213> Artificial Sequence

<220>
<221> source
<222> 1..29
<223> /mol_type="unassigned DNA"
      /note="Oligonucleotide antisense probe designed on
      the basis of MSH6 gene sequence for c.1186C>G site"
      /organism="Artificial Sequence"

<400> 374
agaattcagg aaatcctcag gcacataga                                        29


<210> 375
<211> 29
<212> DNA
<213> Artificial Sequence

<220>
<221> source
<222> 1..29
<223> /mol_type="unassigned DNA"
      /note="Oligonucleotide sense probe designed on
      the basis of MSH6 gene sequence for c.1508C>G site"

/organism="Artificial Sequence"

<400> 375
ggcacgatgt agaaagatgg cacatatat                                          29


<210> 376
<211> 25
<212> DNA
<213> Artificial Sequence

<220>
<221> source
<222> 1..25
<223> /mol_type="unassigned DNA"
      /note="Oligonucleotide antisense probe designed on
      the basis of MSH6 gene sequence for c.1508C>G site"
      /organism="Artificial Sequence"

<400> 376
cctcctcacc actctatcat acttg                                              25

<210> 377
<211> 25
<212> DNA
<213> Artificial Sequence

<220>
<221> source
<222> 1..25
<223> /mol_type="unassigned DNA"
      /note="Oligonucleotide sense probe designed on
      the basis of MSH6 gene sequence for c.1784delT site"
      /organism="Artificial Sequence"

<400> 377
gcacactatc ccccagtaca agttt                                              25


<210> 378
<211> 39
<212> DNA
<213> Artificial Sequence

<220>
<221> source
<222> 1..39
<223> /mol_type="unassigned DNA"
      /note="Oligonucleotide antisense probe designed on
      the basis of MSH6 gene sequence for c.1784delT site"
      /organism="Artificial Sequence"

<400> 378
gttttagttt cctttgagag atttcctttt tcaaataaa                               39


<210> 379
<211> 29

<212> DNA
<213> Artificial Sequence

<220>
<221> source
<222> 1..29
<223> /mol_type="unassigned DNA"
      /note="Oligonucleotide sense probe designed on
      the basis of MSH6 gene sequence for c.1787delT site"
      /organism="Artificial Sequence"

<400> 379
gcacactatc ccccagtaca agttttatt                                         29


<210> 380
<211> 34
<212> DNA
<213> Artificial Sequence

<220>
<221> source
<222> 1..34
<223> /mol_type="unassigned DNA"
      /note="Oligonucleotide antisense probe designed on
      the basis of MSH6 gene sequence for c.1787delT site"
      /organism="Artificial Sequence"

<400> 380
gttttagttt cctttgagag atttcctttt tcaa                                   34


<210> 381
<211> 20
<212> DNA
<213> Artificial Sequence

<220>
<221> source
<222> 1..20
<223> /mol_type="unassigned DNA"
      /note="Oligonucleotide sense probe designed on
      the basis of MSH6 gene sequence for c.3261delC site"
      /organism="Artificial Sequence"

<400> 381
ttgccggaag ataccccccc                                                   20


<210> 382
<211> 24
<212> DNA
<213> Artificial Sequence

<220>
<221> source
<222> 1..24
<223> /mol_type="unassigned DNA"

/note="Oligonucleotide antisense probe designed on
      the basis of MSH6 gene sequence for c.3261delC site"
      /organism="Artificial Sequence"

<400> 382
cgtttaagct ctaagaaggg gggg                                              24


<210> 383
<211> 30
<212> DNA
<213> Artificial Sequence

<220>
<221> source
<222> 1..30
<223> /mol_type="unassigned DNA"
      /note="Oligonucleotide sense probe designed on
      the basis of MSH6 gene sequence for c.3438+14A>T site"
      /organism="Artificial Sequence"

<400> 383
ctacgcttat gagactggta actgattctt                                        30


<210> 384
<211> 36
<212> DNA
<213> Artificial Sequence

<220>
<221> source
<222> 1..36
<223> /mol_type="unassigned DNA"
      /note="Oligonucleotide antisense probe designed on
      the basis of MSH6 gene sequence for c.3438+14A>T site"
      /organism="Artificial Sequence"

<400> 384
cctaatgaca caaatgactt tctgataaca aaactt                                 36


<210> 385
<211> 27
<212> DNA
<213> Artificial Sequence

<220>
<221> source
<222> 1..27
<223> /mol_type="unassigned DNA"
      /note="Oligonucleotide sense probe designed on
      the basis of MSH6 gene sequence for c.3514dupA site"
      /organism="Artificial Sequence"

<400> 385
aagtctgcag gctcacacca attgata                                           27

<210> 386
<211> 27
<212> DNA
<213> Artificial Sequence

<220>
<221> source
<222> 1..27
<223> /mol_type="unassigned DNA"
      /note="Oligonucleotide antisense probe designed on
      the basis of MSH6 gene sequence for c.3514dupA site"
      /organism="Artificial Sequence"

<400> 386
tcaggcacca agtctagtaa acactct                                          27


<210> 387
<211> 27
<212> DNA
<213> Artificial Sequence

<220>
<221> source
<222> 1..27
<223> /mol_type="unassigned DNA"
      /note="Oligonucleotide sense probe designed on
      the basis of MSH6 gene sequence for c.3838C>T site"
      /organism="Artificial Sequence"

<400> 387
gtagaaaatg aatgtgaaga ccccagc                                          27


<210> 388
<211> 35
<212> DNA
<213> Artificial Sequence

<220>
<221> source
<222> 1..35
<223> /mol_type="unassigned DNA"
      /note="Oligonucleotide antisense probe designed on
      the basis of MSH6 gene sequence for c.3838C>T site"
      /organism="Artificial Sequence"

<400> 388
cttaatgaat ttatagagca acgtaatagt ctcct                                 35


<210> 389
<211> 20
<212> DNA
<213> Artificial Sequence

<220>

<221> source
<222> 1..20
<223> /mol_type="unassigned DNA"
      /note="Oligonucleotide sense probe designed on
      the basis of NOD2 gene sequence for c.3020insC site"
      /organism="Artificial Sequence"

<400> 389
agaacccctc ctgcaggccc                                                    20


<210> 390
<211> 27
<212> DNA
<213> Artificial Sequence

<220>
<221> source
<222> 1..27
<223> /mol_type="unassigned DNA"
      /note="Oligonucleotide antisense probe designed on
      the basis of NOD2 gene sequence for c.3020insC site"
      /organism="Artificial Sequence"

<400> 390
cttccaggat ggtgtcattc ctttcaa                                            27


<210> 391
<211> 23
<212> DNA
<213> Artificial Sequence

<220>
<221> source
<222> 1..23
<223> /mol_type="unassigned DNA"
      /note="Oligonucleotide sense probe designed on
      the basis of CDKN2A gene sequence for p.A148T site"
      /organism="Artificial Sequence"

<400> 391
gtaaccatgc ccgcatagat gcc                                                23


<210> 392
<211> 20
<212> DNA
<213> Artificial Sequence

<220>
<221> source
<222> 1..20
<223> /mol_type="unassigned DNA"
      /note="Oligonucleotide antisense probe designed on
      the basis of CDKN2A gene sequence for p.A148T site"
      /organism="Artificial Sequence"

<400> 392
ctcacctgag ggaccttccg                                                   20


<210> 393
<211> 18
<212> DNA
<213> Artificial Sequence

<220>
<221> source
<222> 1..18
<223> /mol_type="unassigned DNA"
      /note="Oligonucleotide sense probe designed on
      the basis of CYP1B1 gene sequence for p.A119S site"
      /organism="Artificial Sequence"

<400> 393
gccttcgccg accggccg                                                     18


<210> 394
<211> 22
<212> DNA
<213> Artificial Sequence

<220>
<221> source
<222> 1..22
<223> /mol_type="unassigned DNA"
      /note="Oligonucleotide antisense probe designed on
      the basis of CYP1B1 gene sequence for p.A119S site"
      /organism="Artificial Sequence"

<400> 394
accacacgga aggaggcgaa gg                                                22


<210> 395
<211> 40
<212> DNA
<213> Artificial Sequence

<220>
<221> source
<222> 1..40
<223> /mol_type="unassigned DNA"
      /note="Oligonucleotide sense probe designed on
      the basis of CHEK2 gene sequence for c.1100delC site"
      /organism="Artificial Sequence"

<400> 395
aagttcaaca ttattccctt ttgtactgaa ttttagatta                             40


<210> 396
<211> 28
<212> DNA

<213> Artificial Sequence

<220>
<221> source
<222> 1..28
<223> /mol_type="unassigned DNA"
      /note="Oligonucleotide antisense probe designed on
      the basis of CHEK2 gene sequence for c.1100delC site"
      /organism="Artificial Sequence"

<400> 396
tcgcaaaatc ttggagtgcc caaaatca                                          28


<210> 397
<211> 28
<212> DNA
<213> Artificial Sequence

<220>
<221> source
<222> 1..28
<223> /mol_type="unassigned DNA"
      /note="Oligonucleotide sense probe designed on
      the basis of CHEK2 gene sequence for p.IVS2+1G>A site"
      /organism="Artificial Sequence"

<400> 397
cagcaagaaa cactttcgga ttttcagg                                          28


<210> 398
<211> 31
<212> DNA
<213> Artificial Sequence

<220>
<221> source
<222> 1..31
<223> /mol_type="unassigned DNA"
      /note="Oligonucleotide antisense probe designed on
      the basis of CHEK2 gene sequence for p.IVS2+1G>A site"
      /organism="Artificial Sequence"

<400> 398
ctctcctaga tacatggcta ttcattacct a                                      31


<210> 399
<211> 21
<212> DNA
<213> Artificial Sequence

<220>
<221> source
<222> 1..21
<223> /mol_type="unassigned DNA"
      /note="Oligonucleotide forward primer designed on

the basis of MLH1 gene sequence enabling amplification of a region
      comprising genetic mutation site c.-93G>A"
      /organism="Artificial Sequence"

<400> 399
cagcaaccca cagagttgag a                                                21


<210> 400
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH1 gene sequence enabling amplification
      of a region comprising genetic mutation site c.-93G>A"
      /organism="Artificial Sequence"

<400> 400
atgcgctgta catgcctctg                                                  20


<210> 401
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.37delG"
      /organism="Artificial Sequence"

<400> 401
cagcaaccca cagagttgag a                                                21


<210> 402
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH1 gene sequence enabling amplification
      of a region comprising genetic mutation site c.37delG"
      /organism="Artificial Sequence"

<400> 402
atgcgctgta catgcctctg                                                    20


<210> 403
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.65G>C"
      /organism="Artificial Sequence"

<400> 403
cagcaaccca cagagttgag a                                                  21


<210> 404
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH1 gene sequence enabling amplification
      of a region comprising genetic mutation site c.65G>C"
      /organism="Artificial Sequence"

<400> 404
atgcgctgta catgcctctg                                                    20


<210> 405
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.66delG"
      /organism="Artificial Sequence"

<400> 405
cagcaaccca cagagttgag a                                                  21

<210> 406
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH1 gene sequence enabling amplification
      of a region comprising genetic mutation site c.66delG"
      /organism="Artificial Sequence"

<400> 406
atgcgctgta catgcctctg                                                   20


<210> 407
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.69A>T"
      /organism="Artificial Sequence"

<400> 407
cagcaaccca cagagttgag a                                                 21


<210> 408
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH1 gene sequence enabling amplification
      of a region comprising genetic mutation site c.69A>T"
      /organism="Artificial Sequence"

<400> 408
atgcgctgta catgcctctg                                                   20


<210> 409
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.74T>C"
      /organism="Artificial Sequence"

<400> 409
cagcaaccca cagagttgag a                                                21


<210> 410
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH1 gene sequence enabling amplification
      of a region comprising genetic mutation site c.74T>C"
      /organism="Artificial Sequence"

<400> 410
atgcgctgta catgcctctg                                                  20


<210> 411
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.83C>T"
      /organism="Artificial Sequence"

<400> 411
cagcaaccca cagagttgag a                                                21


<210> 412
<211> 20
<212> DNA
<213> Artificial Sequence

<220>
<221> source
<222> 1..20

<223> /mol_type="unassigned DNA"
      /note="Oligonucleotide reverse primer designed
      on the basis of MLH1 gene sequence enabling amplification
      of a region comprising genetic mutation site c.83C>T"
      /organism="Artificial Sequence"

<400> 412
atgcgctgta catgcctctg                                                   20


<210> 413
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.85G>T"
      /organism="Artificial Sequence"

<400> 413
cagcaaccca cagagttgag a                                                 21


<210> 414
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH1 gene sequence enabling amplification
      of a region comprising genetic mutation site c.85G>T"
      /organism="Artificial Sequence"

<400> 414
atgcgctgta catgcctctg                                                   20


<210> 415
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.104T>G"

/organism="Artificial Sequence"

<400> 415
cagcaaccca cagagttgag a                                                21


<210> 416
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH1 gene sequence enabling amplification
      of a region comprising genetic mutation site c.104T>G"
      /organism="Artificial Sequence"

<400> 416
atgcgctgta catgcctctg                                                  20


<210> 417
<211> 22
<212> DNA
<213> Artificial Sequence

<220>
<221> source
<222> 1..22
<223> /mol_type="unassigned DNA"
      /note="Oligonucleotide forward primer designed on
      the basis of MLH1 gene sequence enabling amplification of a region
      comprising genetic mutation site c.131C>T"
      /organism="Artificial Sequence"

<400> 417
tttgttatca ttgcttggct ca                                               22


<210> 418
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH1 gene sequence enabling amplification
      of a region comprising genetic mutation site c.131C>T"
      /organism="Artificial Sequence"

<400> 418
cttccatgaa gcgcacaaac                                                  20

<210> 419
<211> 22
<212> DNA
<213> Artificial Sequence

<220>
<221> source
<222> 1..22
<223> /mol_type="unassigned DNA"
      /note="Oligonucleotide forward primer designed on
      the basis of MLH1 gene sequence enabling amplification of a region
      comprising genetic mutation site c.137G>T"
      /organism="Artificial Sequence"

<400> 419
tttgttatca ttgcttggct ca                                                22


<210> 420
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH1 gene sequence enabling amplification
      of a region comprising genetic mutation site c.137G>T"
      /organism="Artificial Sequence"

<400> 420
cttccatgaa gcgcacaaac                                                   20


<210> 421
<211> 22
<212> DNA
<213> Artificial Sequence

<220>
<221> source
<222> 1..22
<223> /mol_type="unassigned DNA"
      /note="Oligonucleotide forward primer designed on
      the basis of MLH1 gene sequence enabling amplification of a region
      comprising genetic mutation site c.161G>A"
      /organism="Artificial Sequence"

<400> 421
tttgttatca ttgcttggct ca                                                22


<210> 422
<211> 20

<212> DNA
<213> Artificial Sequence

<220>
<221> source
<222> 1..20
<223> /mol_type="unassigned DNA"
      /note="Oligonucleotide reverse primer designed
      on the basis of MLH1 gene sequence enabling amplification
      of a region comprising genetic mutation site c.161G>A"
      /organism="Artificial Sequence"

<400> 422
cttccatgaa gcgcacaaac                                                   20


<210> 423
<211> 22
<212> DNA
<213> Artificial Sequence

<220>
<221> source
<222> 1..22
<223> /mol_type="unassigned DNA"
      /note="Oligonucleotide forward primer designed on
      the basis of MLH1 gene sequence enabling amplification of a region
      comprising genetic mutation site c.161delG"
      /organism="Artificial Sequence"

<400> 423
tttgttatca ttgcttggct ca                                                22


<210> 424
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH1 gene sequence enabling amplification
      of a region comprising genetic mutation site c.161delG"
      /organism="Artificial Sequence"

<400> 424
cttccatgaa gcgcacaaac                                                   20


<210> 425
<211> 22
<212> DNA
<213> Artificial Sequence

<220>

<221> source
<222> 1..22
<223> /mol_type="unassigned DNA"
      /note="Oligonucleotide forward primer designed on
      the basis of MLH1 gene sequence enabling amplification of a region
      comprising genetic mutation site c.184C>A/T"
      /organism="Artificial Sequence"

<400> 425
tttgttatca ttgcttggct ca                                                22


<210> 426
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH1 gene sequence enabling amplification
      of a region comprising genetic mutation site c.184C>A/T"
      /organism="Artificial Sequence"

<400> 426
cttccatgaa gcgcacaaac                                                   20


<210> 427
<211> 22
<212> DNA
<213> Artificial Sequence

<220>
<221> source
<222> 1..22
<223> /mol_type="unassigned DNA"
      /note="Oligonucleotide forward primer designed on
      the basis of MLH1 gene sequence enabling amplification of a region
      comprising genetic mutation site c.191A>G"
      /organism="Artificial Sequence"

<400> 427
tttgttatca ttgcttggct ca                                                22


<210> 428
<211> 20
<212> DNA
<213> Artificial Sequence

<220>
<221> source
<222> 1..20
<223> /mol_type="unassigned DNA"
      /note="Oligonucleotide reverse primer designed

on the basis of MLH1 gene sequence enabling amplification
      of a region comprising genetic mutation site c.191A>G"
      /organism="Artificial Sequence"

<400> 428
cttccatgaa gcgcacaaac                                                   20


<210> 429
<211> 22
<212> DNA
<213> Artificial Sequence

<220>
<221> source
<222> 1..22
<223> /mol_type="unassigned DNA"
      /note="Oligonucleotide forward primer designed on
      the basis of MLH1 gene sequence enabling amplification of a region
      comprising genetic mutation site c.194G>A"
      /organism="Artificial Sequence"

<400> 429
tttgttatca ttgcttggct ca                                                22


<210> 430
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH1 gene sequence enabling amplification
      of a region comprising genetic mutation site c.194G>A"
      /organism="Artificial Sequence"

<400> 430
cttccatgaa gcgcacaaac                                                   20


<210> 431
<211> 22
<212> DNA
<213> Artificial Sequence

<220>
<221> source
<222> 1..22
<223> /mol_type="unassigned DNA"
      /note="Oligonucleotide forward primer designed on
      the basis of MLH1 gene sequence enabling amplification of a region
      comprising genetic mutation site c.199G>A/T"
      /organism="Artificial Sequence"

<400> 431
tttgttatca ttgcttggct ca                                                22


<210> 432
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH1 gene sequence enabling amplification
      of a region comprising genetic mutation site c.199G>A/T"
      /organism="Artificial Sequence"

<400> 432
cttccatgaa gcgcacaaac                                                   20


<210> 433
<211> 22
<212> DNA
<213> Artificial Sequence

<220>
<221> source
<222> 1..22
<223> /mol_type="unassigned DNA"
      /note="Oligonucleotide forward primer designed on
      the basis of MLH1 gene sequence enabling amplification of a region
      comprising genetic mutation site c.200G>A"
      /organism="Artificial Sequence"

<400> 433
tttgttatca ttgcttggct ca                                                22


<210> 434
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH1 gene sequence enabling amplification
      of a region comprising genetic mutation site c.200G>A"
      /organism="Artificial Sequence"

<400> 434
cttccatgaa gcgcacaaac                                                   20

<210> 435
<211> 22
<212> DNA
<213> Artificial Sequence

<220>
<221> source
<222> 1..22
<223> /mol_type="unassigned DNA"
      /note="Oligonucleotide forward primer designed on
      the basis of MLH1 gene sequence enabling amplification of a region
      comprising genetic mutation site c.203T>A"
      /organism="Artificial Sequence"

<400> 435
tttgttatca ttgcttggct ca                                                22


<210> 436
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH1 gene sequence enabling amplification
      of a region comprising genetic mutation site c.203T>A"
      /organism="Artificial Sequence"

<400> 436
cttccatgaa gcgcacaaac                                                   20


<210> 437
<211> 22
<212> DNA
<213> Artificial Sequence

<220>
<221> source
<222> 1..22
<223> /mol_type="unassigned DNA"
      /note="Oligonucleotide forward primer designed on
      the basis of MLH1 gene sequence enabling amplification of a region
      comprising genetic mutation site c.206G>A"
      /organism="Artificial Sequence"

<400> 437
tttgttatca ttgcttggct ca                                                22


<210> 438
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH1 gene sequence enabling amplification
      of a region comprising genetic mutation site c.206G>A"
      /organism="Artificial Sequence"

<400> 438
cttccatgaa gcgcacaaac                                                   20


<210> 439
<211> 24
<212> DNA
<213> Artificial Sequence

<220>
<221> source
<222> 1..24
<223> /mol_type="unassigned DNA"
      /note="Oligonucleotide forward primer designed on
      the basis of MLH1 gene sequence enabling amplification of a region
      comprising genetic mutation site c.229T>C"
      /organism="Artificial Sequence"

<400> 439
tcaagaaaat gggaattcaa agag                                              24


<210> 440
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH1 gene sequence enabling amplification
      of a region comprising genetic mutation site c.229T>C"
      /organism="Artificial Sequence"

<400> 440
cagacaatgt catcacagga gga                                               23


<210> 441
<211> 24
<212> DNA
<213> Artificial Sequence

<220>
<221> source
<222> 1..24

<223> /mol_type="unassigned DNA"
      /note="Oligonucleotide forward primer designed on
      the basis of MLH1 gene sequence enabling amplification of a region
      comprising genetic mutation site c.230G>A"
      /organism="Artificial Sequence"

<400> 441
tcaagaaaat gggaattcaa agag                                              24


<210> 442
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH1 gene sequence enabling amplification
      of a region comprising genetic mutation site c.230G>A"
      /organism="Artificial Sequence"

<400> 442
cagacaatgt catcacagga gga                                               23


<210> 443
<211> 24
<212> DNA
<213> Artificial Sequence

<220>
<221> source
<222> 1..24
<223> /mol_type="unassigned DNA"
      /note="Oligonucleotide forward primer designed on
      the basis of MLH1 gene sequence enabling amplification of a region
      comprising genetic mutation site c.238T>G"
      /organism="Artificial Sequence"

<400> 443
tcaagaaaat gggaattcaa agag                                              24


<210> 444
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH1 gene sequence enabling amplification
      of a region comprising genetic mutation site c.238T>G"

/organism="Artificial Sequence"

<400> 444
cagacaatgt catcacagga gga                                              23


<210> 445
<211> 24
<212> DNA
<213> Artificial Sequence

<220>
<221> source
<222> 1..24
<223> /mol_type="unassigned DNA"
      /note="Oligonucleotide forward primer designed on
      the basis of MLH1 gene sequence enabling amplification of a region
      comprising genetic mutation site c.250A>G"
      /organism="Artificial Sequence"

<400> 445
tcaagaaaat gggaattcaa agag                                             24


<210> 446
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH1 gene sequence enabling amplification
      of a region comprising genetic mutation site c.250A>G"
      /organism="Artificial Sequence"

<400> 446
cagacaatgt catcacagga gga                                              23


<210> 447
<211> 24
<212> DNA
<213> Artificial Sequence

<220>
<221> source
<222> 1..24
<223> /mol_type="unassigned DNA"
      /note="Oligonucleotide forward primer designed on
      the basis of MLH1 gene sequence enabling amplification of a region
      comprising genetic mutation site c.256C>T"
      /organism="Artificial Sequence"

<400> 447
tcaagaaaat gggaattcaa agag                                             24

<210> 448
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH1 gene sequence enabling amplification
      of a region comprising genetic mutation site c.256C>T"
      /organism="Artificial Sequence"

<400> 448
cagacaatgt catcacagga gga                                              23


<210> 449
<211> 24
<212> DNA
<213> Artificial Sequence

<220>
<221> source
<222> 1..24
<223> /mol_type="unassigned DNA"
      /note="Oligonucleotide forward primer designed on
      the basis of MLH1 gene sequence enabling amplification of a region
      comprising genetic mutation site c.277A>G"
      /organism="Artificial Sequence"

<400> 449
tcaagaaaat gggaattcaa agag                                             24


<210> 450
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH1 gene sequence enabling amplification
      of a region comprising genetic mutation site c.277A>G"
      /organism="Artificial Sequence"

<400> 450
cagacaatgt catcacagga gga                                              23


<210> 451
<211> 24

<212> DNA
<213> Artificial Sequence

<220>
<221> source
<222> 1..24
<223> /mol_type="unassigned DNA"
      /note="Oligonucleotide forward primer designed on
      the basis of MLH1 gene sequence enabling amplification of a region
      comprising genetic mutation site c.298C>T"
      /organism="Artificial Sequence"

<400> 451
tcaagaaaat gggaattcaa agag                                              24


<210> 452
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH1 gene sequence enabling amplification
      of a region comprising genetic mutation site c.298C>T"
      /organism="Artificial Sequence"

<400> 452
cagacaatgt catcacagga gga                                               23


<210> 453
<211> 24
<212> DNA
<213> Artificial Sequence

<220>
<221> source
<222> 1..24
<223> /mol_type="unassigned DNA"
      /note="Oligonucleotide forward primer designed on
      the basis of MLH1 gene sequence enabling amplification of a region
      comprising genetic mutation site c.299G>C "
      /organism="Artificial Sequence"

<400> 453
tcaagaaaat gggaattcaa agag                                              24


<210> 454
<211> 23
<212> DNA
<213> Artificial Sequence

<220>

<221> source
<222> 1..23
<223> /mol_type="unassigned DNA"
      /note="Oligonucleotide reverse primer designed
      on the basis of MLH1 gene sequence enabling amplification
      of a region comprising genetic mutation site c.299G>C "
      /organism="Artificial Sequence"

<400> 454
cagacaatgt catcacagga gga                                              23


<210> 455
<211> 24
<212> DNA
<213> Artificial Sequence

<220>
<221> source
<222> 1..24
<223> /mol_type="unassigned DNA"
      /note="Oligonucleotide forward primer designed on
      the basis of MLH1 gene sequence enabling amplification of a region
      comprising genetic mutation site c.304G>A "
      /organism="Artificial Sequence"

<400> 455
tcaagaaaat gggaattcaa agag                                             24


<210> 456
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH1 gene sequence enabling amplification
      of a region comprising genetic mutation site c.304G>A"
      /organism="Artificial Sequence"

<400> 456
cagacaatgt catcacagga gga                                              23


<210> 457
<211> 24
<212> DNA
<213> Artificial Sequence

<220>
<221> source
<222> 1..24
<223> /mol_type="unassigned DNA"
      /note="Oligonucleotide forward primer designed on

the basis of MLH1 gene sequence enabling amplification of a region
      comprising genetic mutation site c.306G>T"
      /organism="Artificial Sequence"

<400> 457
tcaagaaaat gggaattcaa agag                                              24


<210> 458
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH1 gene sequence enabling amplification
      of a region comprising genetic mutation site c.306G>T"
      /organism="Artificial Sequence"

<400> 458
cagacaatgt catcacagga gga                                               23


<210> 459
<211> 24
<212> DNA
<213> Artificial Sequence

<220>
<221> source
<222> 1..24
<223> /mol_type="unassigned DNA"
      /note="Oligonucleotide forward primer designed on
      the basis of MLH1 gene sequence enabling amplification of a region
      comprising genetic mutation site c.306+1G>A"
      /organism="Artificial Sequence"

<400> 459
tcaagaaaat gggaattcaa agag                                              24


<210> 460
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH1 gene sequence enabling amplification
      of a region comprising genetic mutation site c.306+1G>A"
      /organism="Artificial Sequence"

<400> 460
cagacaatgt catcacagga gga                                              23


<210> 461
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.320T>G"
      /organism="Artificial Sequence"

<400> 461
ttccctttgg tgaggtgaca g                                                21


<210> 462
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH1 gene sequence enabling amplification
      of a region comprising genetic mutation site c.320T>G"
      /organism="Artificial Sequence"

<400> 462
tggtgttgag acaggattac tctga                                            25


<210> 463
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.332C>T"
      /organism="Artificial Sequence"

<400> 463
ttccctttgg tgaggtgaca g                                                21

<210> 464
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH1 gene sequence enabling amplification
      of a region comprising genetic mutation site c.332C>T"
      /organism="Artificial Sequence"

<400> 464
tggtgttgag acaggattac tctga                                          25


<210> 465
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.350C>G/T"
      /organism="Artificial Sequence"

<400> 465
ttccctttgg tgaggtgaca g                                              21


<210> 466
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH1 gene sequence enabling amplification
      of a region comprising genetic mutation site c.350C>G/T"
      /organism="Artificial Sequence"

<400> 466
tggtgttgag acaggattac tctga                                          25


<210> 467
<211> 23
<212> DNA
<213> Artificial Sequence

<220>
<221> source
<222> 1..23
<223> /mol_type="unassigned DNA"
      /note="Oligonucleotide forward primer designed on
      the basis of MLH1 gene sequence enabling amplification of a region
      comprising genetic mutation site c.382G>C"
      /organism="Artificial Sequence"

<400> 467
tctcttttcc ccttgggatt agt                                              23


<210> 468
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH1 gene sequence enabling amplification
      of a region comprising genetic mutation site c.382G>C"
      /organism="Artificial Sequence"

<400> 468
gacctccatt aactagtgca aattc                                            25


<210> 469
<211> 23
<212> DNA
<213> Artificial Sequence

<220>
<221> source
<222> 1..23
<223> /mol_type="unassigned DNA"
      /note="Oligonucleotide forward primer designed on
      the basis of MLH1 gene sequence enabling amplification of a region
      comprising genetic mutation site c.392C>A"
      /organism="Artificial Sequence"

<400> 469
tctcttttcc ccttgggatt agt                                              23


<210> 470
<211> 25
<212> DNA
<213> Artificial Sequence

<220>
<221> source
<222> 1..25

<223> /mol_type="unassigned DNA"
      /note="Oligonucleotide reverse primer designed
      on the basis of MLH1 gene sequence enabling amplification
      of a region comprising genetic mutation site c.392C>A"
      /organism="Artificial Sequence"

<400> 470
gacctccatt aactagtgca aattc                                          25


<210> 471
<211> 23
<212> DNA
<213> Artificial Sequence

<220>
<221> source
<222> 1..23
<223> /mol_type="unassigned DNA"
      /note="Oligonucleotide forward primer designed on
      the basis of MLH1 gene sequence enabling amplification of a region
      comprising genetic mutation site c.394G>C"
      /organism="Artificial Sequence"

<400> 471
tctcttttcc ccttgggatt agt                                            23


<210> 472
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH1 gene sequence enabling amplification
      of a region comprising genetic mutation site c.394G>C"
      /organism="Artificial Sequence"

<400> 472
gacctccatt aactagtgca aattc                                          25


<210> 473
<211> 23
<212> DNA
<213> Artificial Sequence

<220>
<221> source
<222> 1..23
<223> /mol_type="unassigned DNA"
      /note="Oligonucleotide forward primer designed on
      the basis of MLH1 gene sequence enabling amplification of a region
      comprising genetic mutation site c.453+79A>G"

/organism="Artificial Sequence"

<400> 473
tctcttttcc ccttgggatt agt                                              23


<210> 474
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH1 gene sequence enabling amplification
      of a region comprising genetic mutation site c.453+79A>G"
      /organism="Artificial Sequence"

<400> 474
gacctccatt aactagtgca aattc                                            25


<210> 475
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.454-1G>A"
      /organism="Artificial Sequence"

<400> 475
ctcgcttttg ccaggacatc t                                                21


<210> 476
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH1 gene sequence enabling amplification
      of a region comprising genetic mutation site c.454-1G>A"
      /organism="Artificial Sequence"

<400> 476
gacaaatctc agagacccac tcc                                              23

<210> 477
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.464T>G"
      /organism="Artificial Sequence"

<400> 477
ctcgcttttg ccaggacatc t                                                21


<210> 478
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH1 gene sequence enabling amplification
      of a region comprising genetic mutation site c.464T>G"
      /organism="Artificial Sequence"

<400> 478
gacaaatctc agagacccac tcc                                              23


<210> 479
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.479C>T"
      /organism="Artificial Sequence"

<400> 479
ctcgcttttg ccaggacatc t                                                21


<210> 480
<211> 23

<212> DNA
<213> Artificial Sequence

<220>
<221> source
<222> 1..23
<223> /mol_type="unassigned DNA"
      /note="Oligonucleotide reverse primer designed
      on the basis of MLH1 gene sequence enabling amplification
      of a region comprising genetic mutation site c.479C>T"
      /organism="Artificial Sequence"

<400> 480
gacaaatctc agagacccac tcc                                              23


<210> 481
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.544A>G"
      /organism="Artificial Sequence"

<400> 481
ctcgcttttg ccaggacatc t                                                21


<210> 482
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH1 gene sequence enabling amplification
      of a region comprising genetic mutation site c.544A>G"
      /organism="Artificial Sequence"

<400> 482
gacaaatctc agagacccac tcc                                              23


<210> 483
<211> 23
<212> DNA
<213> Artificial Sequence

<220>

<221> source
<222> 1..23
<223> /mol_type="unassigned DNA"
      /note="Oligonucleotide forward primer designed on
      the basis of MLH1 gene sequence enabling amplification of a region
      comprising genetic mutation site c.546-2A>G"
      /organism="Artificial Sequence"

<400> 483
   ttaactaaaa gggggctctg aca                                              23


<210> 484
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH1 gene sequence enabling amplification
      of a region comprising genetic mutation site c.546-2A>G"
      /organism="Artificial Sequence"

<400> 484
ggttatcgac ataccgacta acagc                                           25


<210> 485
<211> 23
<212> DNA
<213> Artificial Sequence

<220>
<221> source
<222> 1..23
<223> /mol_type="unassigned DNA"
      /note="Oligonucleotide forward primer designed on
      the basis of MLH1 gene sequence enabling amplification of a region
      comprising genetic mutation site c.577T>C"
      /organism="Artificial Sequence"

<400> 485
   ttaactaaaa gggggctctg aca                                              23


<210> 486
<211> 25
<212> DNA
<213> Artificial Sequence

<220>
<221> source
<222> 1..25
<223> /mol_type="unassigned DNA"
      /note="Oligonucleotide reverse primer designed

on the basis of MLH1 gene sequence enabling amplification
      of a region comprising genetic mutation site c.577T>C"
      /organism="Artificial Sequence"

<400> 486
ggttatcgac ataccgacta acagc                                          25


<210> 487
<211> 23
<212> DNA
<213> Artificial Sequence

<220>
<221> source
<222> 1..23
<223> /mol_type="unassigned DNA"
      /note="Oligonucleotide forward primer designed on
      the basis of MLH1 gene sequence enabling amplification of a region
      comprising genetic mutation site c.589-2A>G"
      /organism="Artificial Sequence"

<400> 487
ttaactaaaa gggggctctg aca                                            23


<210> 488
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH1 gene sequence enabling amplification
      of a region comprising genetic mutation site c.589-2A>G"
      /organism="Artificial Sequence"

<400> 488
ggttatcgac ataccgacta acagc                                          25


<210> 489
<211> 25
<212> DNA
<213> Artificial Sequence

<220>
<221> source
<222> 1..25
<223> /mol_type="unassigned DNA"
      /note="Oligonucleotide forward primer designed on
      the basis of MLH1 gene sequence enabling amplification of a region
      comprising genetic mutation site c.595G>C"
      /organism="Artificial Sequence"

<400> 489
ggttatcgac ataccgacta acagc                                          25


<210> 490
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH1 gene sequence enabling amplification
      of a region comprising genetic mutation site c.595G>C"
      /organism="Artificial Sequence"

<400> 490
ggttatcgac ataccgacta acagc                                          25


<210> 491
<211> 23
<212> DNA
<213> Artificial Sequence

<220>
<221> source
<222> 1..23
<223> /mol_type="unassigned DNA"
      /note="Oligonucleotide forward primer designed on
      the basis of MLH1 gene sequence enabling amplification of a region
      comprising genetic mutation site c.637G>A"
      /organism="Artificial Sequence"

<400> 491
ttaactaaaa gggggctctg aca                                            23


<210> 492
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH1 gene sequence enabling amplification
      of a region comprising genetic mutation site c.637G>A"
      /organism="Artificial Sequence"

<400> 492
ggttatcgac ataccgacta acagc                                          25

<210> 493
<211> 23
<212> DNA
<213> Artificial Sequence

<220>
<221> source
<222> 1..23
<223> /mol_type="unassigned DNA"
      /note="Oligonucleotide forward primer designed on
      the basis of MLH1 gene sequence enabling amplification of a region
      comprising genetic mutation site c.649C>T"
      /organism="Artificial Sequence"

<400> 493
ttaactaaaa gggggctctg aca                                              23


<210> 494
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH1 gene sequence enabling amplification
      of a region comprising genetic mutation site c.649C>T"
      /organism="Artificial Sequence"

<400> 494
ggttatcgac ataccgacta acagc                                            25


<210> 495
<211> 23
<212> DNA
<213> Artificial Sequence

<220>
<221> source
<222> 1..23
<223> /mol_type="unassigned DNA"
      /note="Oligonucleotide forward primer designed on
      the basis of MLH1 gene sequence enabling amplification of a region
      comprising genetic mutation site c.655A>C/G"
      /organism="Artificial Sequence"

<400> 495
ttaactaaaa gggggctctg aca                                              23


<210> 496
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH1 gene sequence enabling amplification
      of a region comprising genetic mutation site c.655A>C/G"
      /organism="Artificial Sequence"

<400> 496
ggttatcgac ataccgacta acagc                                          25


<210> 497
<211> 23
<212> DNA
<213> Artificial Sequence

<220>
<221> source
<222> 1..23
<223> /mol_type="unassigned DNA"
      /note="Oligonucleotide forward primer designed on
      the basis of MLH1 gene sequence enabling amplification of a region
      comprising genetic mutation site c.676C>T"
      /organism="Artificial Sequence"

<400> 497
ttaactaaaa gggggctctg aca                                            23


<210> 498
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH1 gene sequence enabling amplification
      of a region comprising genetic mutation site c.676C>T"
      /organism="Artificial Sequence"

<400> 498
ggttatcgac ataccgacta acagc                                          25


<210> 499
<211> 23
<212> DNA
<213> Artificial Sequence

<220>
<221> source
<222> 1..23

<223> /mol_type="unassigned DNA"
      /note="Oligonucleotide forward primer designed on
      the basis of MLH1 gene sequence enabling amplification of a region
      comprising genetic mutation site c.677G>A/T"
      /organism="Artificial Sequence"

<400> 499
ttaactaaaa gggggctctg aca                                              23


<210> 500
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH1 gene sequence enabling amplification
      of a region comprising genetic mutation site c.677G>A/T"
      /organism="Artificial Sequence"

<400> 500
ggttatcgac ataccgacta acagc                                            25


<210> 501
<211> 23
<212> DNA
<213> Artificial Sequence

<220>
<221> source
<222> 1..23
<223> /mol_type="unassigned DNA"
      /note="Oligonucleotide forward primer designed on
      the basis of MLH1 gene sequence enabling amplification of a region
      comprising genetic mutation site c.677+3A>G"
      /organism="Artificial Sequence"

<400> 501
ttaactaaaa gggggctctg aca                                              23


<210> 502
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH1 gene sequence enabling amplification
      of a region comprising genetic mutation site c.677+3A>G"

/organism="Artificial Sequence"

<400> 502
ggttatcgac ataccgacta acagc                                          25


<210> 503
<211> 26
<212> DNA
<213> Artificial Sequence

<220>
<221> source
<222> 1..26
<223> /mol_type="unassigned DNA"
      /note="Oligonucleotide forward primer designed on
      the basis of MLH1 gene sequence enabling amplification of a region
      comprising genetic mutation site c.731G>A"
      /organism="Artificial Sequence"

<400> 503
gagaactgat agaaattgga tgtgag                                         26


<210> 504
<211> 26
<212> DNA
<213> Artificial Sequence

<220>
<221> source
<222> 1..26
<223> /mol_type="unassigned DNA"
      /note="Oligonucleotide reverse primer designed
      on the basis of MLH1 gene sequence enabling amplification
      of a region comprising genetic mutation site c.731G>A"
      /organism="Artificial Sequence"

<400> 504
ccatgtttaa tcaaacctat ggatca                                         26


<210> 505
<211> 26
<212> DNA
<213> Artificial Sequence

<220>
<221> source
<222> 1..26
<223> /mol_type="unassigned DNA"
      /note="Oligonucleotide forward primer designed on
      the basis of MLH1 gene sequence enabling amplification of a region
      comprising genetic mutation site c.739T>C"
      /organism="Artificial Sequence"

<400> 505
gagaactgat agaaattgga tgtgag                                         26

<210> 506
<211> 26
<212> DNA
<213> Artificial Sequence

<220>
<221> source
<222> 1..26
<223> /mol_type="unassigned DNA"
      /note="Oligonucleotide reverse primer designed
      on the basis of MLH1 gene sequence enabling amplification
      of a region comprising genetic mutation site c.739T>C"
      /organism="Artificial Sequence"

<400> 506
ccatgtttaa tcaaacctat ggatca                                          26


<210> 507
<211> 26
<212> DNA
<213> Artificial Sequence

<220>
<221> source
<222> 1..26
<223> /mol_type="unassigned DNA"
      /note="Oligonucleotide forward primer designed on
      the basis of MLH1 gene sequence enabling amplification of a region
      comprising genetic mutation site c.778C>T"
      /organism="Artificial Sequence"

<400> 507
gagaactgat agaaattgga tgtgag                                          26


<210> 508
<211> 26
<212> DNA
<213> Artificial Sequence

<220>
<221> source
<222> 1..26
<223> /mol_type="unassigned DNA"
      /note="Oligonucleotide reverse primer designed
      on the basis of MLH1 gene sequence enabling amplification
      of a region comprising genetic mutation site c.778C>T"
      /organism="Artificial Sequence"

<400> 508
ccatgtttaa tcaaacctat ggatca                                          26


<210> 509
<211> 26

<212> DNA
<213> Artificial Sequence

<220>
<221> source
<222> 1..26
<223> /mol_type="unassigned DNA"
      /note="Oligonucleotide forward primer designed on
      the basis of MLH1 gene sequence enabling amplification of a region
      comprising genetic mutation site c.790+1G>A"
      /organism="Artificial Sequence"

<400> 509
gagaactgat agaaattgga tgtgag                                          26


<210> 510
<211> 26
<212> DNA
<213> Artificial Sequence

<220>
<221> source
<222> 1..26
<223> /mol_type="unassigned DNA"
      /note="Oligonucleotide reverse primer designed
      on the basis of MLH1 gene sequence enabling amplification
      of a region comprising genetic mutation site c.790+1G>A"
      /organism="Artificial Sequence"

<400> 510
ccatgtttaa tcaaacctat ggatca                                          26


<210> 511
<211> 24
<212> DNA
<213> Artificial Sequence

<220>
<221> source
<222> 1..24
<223> /mol_type="unassigned DNA"
      /note="Oligonucleotide forward primer designed on
      the basis of MLH1 gene sequence enabling amplification of a region
      comprising genetic mutation site c.793C>T"
      /organism="Artificial Sequence"

<400> 511
acagaaatgt ctttcctgag gtga                                            24


<210> 512
<211> 22
<212> DNA
<213> Artificial Sequence

<220>

<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.793C>T"
      /organism="Artificial Sequence"

<400> 512
tccttgtgag tcttggttga gg                                              22


<210> 513
<211> 24
<212> DNA
<213> Artificial Sequence

<220>
<221> source
<222> 1..24
<223> /mol_type="unassigned DNA"
      /note="Oligonucleotide forward primer designed on
      the basis of MLH1 gene sequence enabling amplification of a region
      comprising genetic mutation site c.794G>A"
      /organism="Artificial Sequence"

<400> 513
acagaaatgt ctttcctgag gtga                                            24


<210> 514
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.794G>A"
      /organism="Artificial Sequence"

<400> 514
tccttgtgag tcttggttga gg                                              22


<210> 515
<211> 24
<212> DNA
<213> Artificial Sequence

<220>
<221> source
<222> 1..24
<223> /mol_type="unassigned DNA"
      /note="Oligonucleotide forward primer designed on

the basis of MLH1 gene sequence enabling amplification of a region
      comprising genetic mutation site c.803A>G"
      /organism="Artificial Sequence"

<400> 515
acagaaatgt ctttcctgag gtga                                              24


<210> 516
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.803A>G"
      /organism="Artificial Sequence"

<400> 516
tccttgtgag tcttggttga gg                                                22


<210> 517
<211> 24
<212> DNA
<213> Artificial Sequence

<220>
<221> source
<222> 1..24
<223> /mol_type="unassigned DNA"
      /note="Oligonucleotide forward primer designed on
      the basis of MLH1 gene sequence enabling amplification of a region
      comprising genetic mutation site c.814T>G"
      /organism="Artificial Sequence"

<400> 517
acagaaatgt ctttcctgag gtga                                              24


<210> 518
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.814T>G"
      /organism="Artificial Sequence"

<400> 518
tccttgtgag tcttggttga gg                                                22


<210> 519
<211> 24
<212> DNA
<213> Artificial Sequence

<220>
<221> source
<222> 1..24
<223> /mol_type="unassigned DNA"
      /note="Oligonucleotide forward primer designed on
      the basis of MLH1 gene sequence enabling amplification of a region
      comprising genetic mutation site c.842C>T"
      /organism="Artificial Sequence"

<400> 519
acagaaatgt ctttcctgag gtga                                              24


<210> 520
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.842C>T"
      /organism="Artificial Sequence"

<400> 520
tccttgtgag tcttggttga gg                                                22


<210> 521
<211> 24
<212> DNA
<213> Artificial Sequence

<220>
<221> source
<222> 1..24
<223> /mol_type="unassigned DNA"
      /note="Oligonucleotide forward primer designed on
      the basis of MLH1 gene sequence enabling amplification of a region
      comprising genetic mutation site c.875T>C"
      /organism="Artificial Sequence"

<400> 521
acagaaatgt ctttcctgag gtga                                              24

<210> 522
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.875T>C"
      /organism="Artificial Sequence"

<400> 522
tccttgtgag tcttggttga gg                                              22


<210> 523
<211> 24
<212> DNA
<213> Artificial Sequence

<220>
<221> source
<222> 1..24
<223> /mol_type="unassigned DNA"
      /note="Oligonucleotide forward primer designed on
      the basis of MLH1 gene sequence enabling amplification of a region
      comprising genetic mutation site c.882C>T"
      /organism="Artificial Sequence"

<400> 523
acagaaatgt ctttcctgag gtga                                            24


<210> 524
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.882C>T"
      /organism="Artificial Sequence"

<400> 524
tccttgtgag tcttggttga gg                                              22

<210> 525
<211> 24
<212> DNA
<213> Artificial Sequence

<220>
<221> source
<222> 1..24
<223> /mol_type="unassigned DNA"
      /note="Oligonucleotide forward primer designed on
      the basis of MLH1 gene sequence enabling amplification of a region
      comprising genetic mutation site c.883delAGgt"
      /organism="Artificial Sequence"

<400> 525
acagaaatgt ctttcctgag gtga                                              24


<210> 526
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.883delAGgt"
      /organism="Artificial Sequence"

<400> 526
tccttgtgag tcttggttga gg                                                22


<210> 527
<211> 24
<212> DNA
<213> Artificial Sequence

<220>
<221> source
<222> 1..24
<223> /mol_type="unassigned DNA"
      /note="Oligonucleotide forward primer designed on
      the basis of MLH1 gene sequence enabling amplification of a region
      comprising genetic mutation site c.883A>C"
      /organism="Artificial Sequence"

<400> 527
acagaaatgt ctttcctgag gtga                                              24


<210> 528
<211> 22
<212> DNA
<213> Artificial Sequence

<220>
<221> source
<222> 1..22
<223> /mol_type="unassigned DNA"

/note="Oligonucleotide reverse primer designed
      on the basis of MLH1 gene sequence enabling amplification
      of a region comprising genetic mutation site c.883A>C"
      /organism="Artificial Sequence"

<400> 528
tccttgtgag tcttggttga gg                                              22


<210> 529
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.884-2A>C"
      /organism="Artificial Sequence"

<400> 529
tccccctccc actatctaag g                                               21


<210> 530
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH1 gene sequence enabling amplification
      of a region comprising genetic mutation site c.884-2A>C"
      /organism="Artificial Sequence"

<400> 530
aaatctgggc tctcacgtct g                                               21


<210> 531
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1013A>G"
      /organism="Artificial Sequence"

<400> 531
tccccctccc actatctaag g                                                21

<210> 532
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH1 gene sequence enabling amplification
      of a region comprising genetic mutation site c.1013A>G"
      /organism="Artificial Sequence"

<400> 532
aaatctgggc tctcacgtct g                                                21

<210> 533
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1038G>C"
      /organism="Artificial Sequence"

<400> 533
tccccctccc actatctaag g                                                21

<210> 534
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH1 gene sequence enabling amplification
      of a region comprising genetic mutation site c.1038G>C"
      /organism="Artificial Sequence"

<400> 534
aaatctgggc tctcacgtct g                                                21

<210> 535
<211> 22
<212> DNA
<213> Artificial Sequence

<220>
<221> source
<222> 1..22
<223> /mol_type="unassigned DNA"
      /note="Oligonucleotide forward primer designed on
      the basis of MLH1 gene sequence enabling amplification of a region
      comprising genetic mutation site c.1151T>A"
      /organism="Artificial Sequence"

<400> 535
gactttgcta ccaggacttg ct                                                22


<210> 536
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.1151T>A"
      /organism="Artificial Sequence"

<400> 536
ggcagagaga agatgcaagt ga                                                22


<210> 537
<211> 22
<212> DNA
<213> Artificial Sequence

<220>
<221> source
<222> 1..22
<223> /mol_type="unassigned DNA"
      /note="Oligonucleotide forward primer designed on
      the basis of MLH1 gene sequence enabling amplification of a region
      comprising genetic mutation site c.1166G>A"
      /organism="Artificial Sequence"

<400> 537
gactttgcta ccaggacttg ct                                                22


<210> 538
<211> 22
<212> DNA

<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.1166G>A"
      /organism="Artificial Sequence"

<400> 538
ggcagagaga agatgcaagt ga                                                22


<210> 539
<211> 22
<212> DNA
<213> Artificial Sequence

<220>
<221> source
<222> 1..22
<223> /mol_type="unassigned DNA"
      /note="Oligonucleotide forward primer designed on
      the basis of MLH1 gene sequence enabling amplification of a region
      comprising genetic mutation site c.1217G>A"
      /organism="Artificial Sequence"

<400> 539
gactttgcta ccaggacttg ct                                                22


<210> 540
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.1217G>A"
      /organism="Artificial Sequence"

<400> 540
ggcagagaga agatgcaagt ga                                                22


<210> 541
<211> 22
<212> DNA
<213> Artificial Sequence

<220>
<221> source

<222> 1..22
<223> /mol_type="unassigned DNA"
      /note="Oligonucleotide forward primer designed on
      the basis of MLH1 gene sequence enabling amplification of a region
      comprising genetic mutation site c.1252delGA"
      /organism="Artificial Sequence"

<400> 541
gactttgcta ccaggacttg ct                                              22


<210> 542
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.1252delGA"
      /organism="Artificial Sequence"

<400> 542
ggcagagaga agatgcaagt ga                                              22


<210> 543
<211> 22
<212> DNA
<213> Artificial Sequence

<220>
<221> source
<222> 1..22
<223> /mol_type="unassigned DNA"
      /note="Oligonucleotide forward primer designed on
      the basis of MLH1 gene sequence enabling amplification of a region
      comprising genetic mutation site c.1321G>A"
      /organism="Artificial Sequence"

<400> 543
gactttgcta ccaggacttg ct                                              22


<210> 544
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification

of a region comprising genetic mutation site c.1321G>A"
/organism="Artificial Sequence"

<400> 544
ggcagagaga agatgcaagt ga                                                22


<210> 545
<211> 22
<212> DNA
<213> Artificial Sequence

<220>
<221> source
<222> 1..22
<223> /mol_type="unassigned DNA"
/note="Oligonucleotide forward primer designed on
the basis of MLH1 gene sequence enabling amplification of a region
comprising genetic mutation site c.1409+1G>C"
/organism="Artificial Sequence"

<400> 545
gactttgcta ccaggacttg ct                                                22


<210> 546
<211> 22
<212> DNA
<213> Artificial Sequence

<220>
<221> source
<222> 1..22
<223> /mol_type="unassigned DNA"
/note="Oligonucleotide reverse primer designed
on the basis of MLH1 gene sequence enabling amplification
of a region comprising genetic mutation site c.1409+1G>C"
/organism="Artificial Sequence"

<400> 546
ggcagagaga agatgcaagt ga                                                22


<210> 547
<211> 21
<212> DNA
<213> Artificial Sequence

<220>
<221> source
<222> 1..21
<223> /mol_type="unassigned DNA"
/note="Oligonucleotide forward primer designed on
the basis of MLH1 gene sequence enabling amplification of a region
comprising genetic mutation site c.1421G>A"
/organism="Artificial Sequence"

<400> 547

gcaacccaca aaatttggct a                                                21

<210> 548
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH1 gene sequence enabling amplification
      of a region comprising genetic mutation site c.1421G>A"
      /organism="Artificial Sequence"

<400> 548
tccaaaacct tggcagttga g                                                21

<210> 549
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1474G>A"
      /organism="Artificial Sequence"

<400> 549
gcaacccaca aaatttggct a                                                21

<210> 550
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH1 gene sequence enabling amplification
      of a region comprising genetic mutation site c.1474G>A"
      /organism="Artificial Sequence"

<400> 550
tccaaaacct tggcagttga g                                                21

<210> 551

<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1489dupC"
      /organism="Artificial Sequence"

<400> 551
gcaacccaca aaatttggct a                                                21


<210> 552
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH1 gene sequence enabling amplification
      of a region comprising genetic mutation site c.1489dupC"
      /organism="Artificial Sequence"

<400> 552
tccaaaacct tggcagttga g                                                21


<210> 553
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1490insC"
      /organism="Artificial Sequence"

<400> 553
gcaacccaca aaatttggct a                                                21


<210> 554
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH1 gene sequence enabling amplification
      of a region comprising genetic mutation site c.1490insC"
      /organism="Artificial Sequence"

<400> 554
tccaaaacct tggcagttga g                                                21


<210> 555
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1517T>C"
      /organism="Artificial Sequence"

<400> 555
gcaacccaca aaatttggct a                                                21


<210> 556
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH1 gene sequence enabling amplification
      of a region comprising genetic mutation site c.1517T>C"
      /organism="Artificial Sequence"

<400> 556
tccaaaacct tggcagttga g                                                21


<210> 557
<211> 21
<212> DNA
<213> Artificial Sequence

<220>
<221> source
<222> 1..21
<223> /mol_type="unassigned DNA"

/note="Oligonucleotide forward primer designed on
      the basis of MLH1 gene sequence enabling amplification of a region
      comprising genetic mutation site c.1528C>T"
      /organism="Artificial Sequence"

<400> 557
gcaacccaca aaatttggct a                                                21


<210> 558
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH1 gene sequence enabling amplification
      of a region comprising genetic mutation site c.1528C>T"
      /organism="Artificial Sequence"

<400> 558
tccaaaacct tggcagttga g                                                21


<210> 559
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1558+14G>A"
      /organism="Artificial Sequence"

<400> 559
gcaacccaca aaatttggct a                                                21


<210> 560
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH1 gene sequence enabling amplification
      of a region comprising genetic mutation site c.1558+14G>A"
      /organism="Artificial Sequence"

<400> 560
tccaaaacct tggcagttga g                                                21

<210> 561
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1569G>T"
      /organism="Artificial Sequence"

<400> 561
tagttctggt gcctggtgct t                                                21

<210> 562
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH1 gene sequence enabling amplification
      of a region comprising genetic mutation site c.1569G>T"
      /organism="Artificial Sequence"

<400> 562
cctcccaact acctcatgct g                                                21

<210> 563
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1625A>T"
      /organism="Artificial Sequence"

<400> 563
tagttctggt gcctggtgct t                                                21

<210> 564
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH1 gene sequence enabling amplification
      of a region comprising genetic mutation site c.1625A>T"
      /organism="Artificial Sequence"

<400> 564
cctcccaact acctcatgct g                                              21


<210> 565
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1646T>C"
      /organism="Artificial Sequence"

<400> 565
tagttctggt gcctggtgct t                                              21


<210> 566
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH1 gene sequence enabling amplification
      of a region comprising genetic mutation site c.1646T>C"
      /organism="Artificial Sequence"

<400> 566
cctcccaact acctcatgct g                                              21


<210> 567
<211> 21
<212> DNA

<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1649T>C"
      /organism="Artificial Sequence"

<400> 567
tagttctggt gcctggtgct t                                                21


<210> 568
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH1 gene sequence enabling amplification
      of a region comprising genetic mutation site c.1649T>C"
      /organism="Artificial Sequence"

<400> 568
cctcccaact acctcatgct g                                                21


<210> 569
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1652A>C"
      /organism="Artificial Sequence"

<400> 569
tagttctggt gcctggtgct t                                                21


<210> 570
<211> 21
<212> DNA
<213> Artificial Sequence

<220>
<221> source

<222> 1..21
<223> /mol_type="unassigned DNA"
      /note="Oligonucleotide reverse primer designed
      on the basis of MLH1 gene sequence enabling amplification
      of a region comprising genetic mutation site c.1652A>C"
      /organism="Artificial Sequence"

<400> 570
cctcccaact acctcatgct g                                              21


<210> 571
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1658delCCA"
      /organism="Artificial Sequence"

<400> 571
tagttctggt gcctggtgct t                                              21


<210> 572
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH1 gene sequence enabling amplification
      of a region comprising genetic mutation site c.1658delCCA"
      /organism="Artificial Sequence"

<400> 572
cctcccaact acctcatgct g                                              21


<210> 573
<211> 22
<212> DNA
<213> Artificial Sequence

<220>
<221> source
<222> 1..22
<223> /mol_type="unassigned DNA"
      /note="Oligonucleotide forward primer designed on
      the basis of MLH1 gene sequence enabling amplification of a region

comprising genetic mutation site c.1668-19A>G"
/organism="Artificial Sequence"

<400> 573
cccaactggt tgtatctcaa gc                                              22


<210> 574
<211> 25
<212> DNA
<213> Artificial Sequence

<220>
<221> source
<222> 1..25
<223> /mol_type="unassigned DNA"
/note="Oligonucleotide reverse primer designed
on the basis of MLH1 gene sequence enabling amplification
of a region comprising genetic mutation site c.1668-19A>G"
/organism="Artificial Sequence"

<400> 574
tggagagcta ctattttcag aaacg                                           25


<210> 575
<211> 22
<212> DNA
<213> Artificial Sequence

<220>
<221> source
<222> 1..22
<223> /mol_type="unassigned DNA"
/note="Oligonucleotide forward primer designed on
the basis of MLH1 gene sequence enabling amplification of a region
comprising genetic mutation site c.1672G>T"
/organism="Artificial Sequence"

<400> 575
cccaactggt tgtatctcaa gc                                              22


<210> 576
<211> 25
<212> DNA
<213> Artificial Sequence

<220>
<221> source
<222> 1..25
<223> /mol_type="unassigned DNA"
/note="Oligonucleotide reverse primer designed
on the basis of MLH1 gene sequence enabling amplification
of a region comprising genetic mutation site c.1672G>T"
/organism="Artificial Sequence"

<400> 576

tggagagcta ctattttcag aaacg                                          25

<210> 577
<211> 22
<212> DNA
<213> Artificial Sequence

<220>
<221> source
<222> 1..22
<223> /mol_type="unassigned DNA"
      /note="Oligonucleotide forward primer designed on
      the basis of MLH1 gene sequence enabling amplification of a region
      comprising genetic mutation site c.1693A>T"
      /organism="Artificial Sequence"

<400> 577
cccaactggt tgtatctcaa gc                                             22

<210> 578
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH1 gene sequence enabling amplification
      of a region comprising genetic mutation site c.1693A>T"
      /organism="Artificial Sequence"

<400> 578
tggagagcta ctattttcag aaacg                                          25

<210> 579
<211> 22
<212> DNA
<213> Artificial Sequence

<220>
<221> source
<222> 1..22
<223> /mol_type="unassigned DNA"
      /note="Oligonucleotide forward primer designed on
      the basis of MLH1 gene sequence enabling amplification of a region
      comprising genetic mutation site c.1721T>C"
      /organism="Artificial Sequence"

<400> 579
cccaactggt tgtatctcaa gc                                             22

<210> 580

<211> 25
<212> DNA
<213> Artificial Sequence

<220>
<221> source
<222> 1..25
<223> /mol_type="unassigned DNA"
      /note="Oligonucleotide reverse primer designed
      on the basis of MLH1 gene sequence enabling amplification
      of a region comprising genetic mutation site c.1721T>C"
      /organism="Artificial Sequence"

<400> 580
tggagagcta ctattttcag aaacg                                          25


<210> 581
<211> 22
<212> DNA
<213> Artificial Sequence

<220>
<221> source
<222> 1..22
<223> /mol_type="unassigned DNA"
      /note="Oligonucleotide forward primer designed on
      the basis of MLH1 gene sequence enabling amplification of a region
      comprising genetic mutation site c.1731G>A"
      /organism="Artificial Sequence"

<400> 581
cccaactggt tgtatctcaa gc                                             22


<210> 582
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH1 gene sequence enabling amplification
      of a region comprising genetic mutation site c.1731G>A"
      /organism="Artificial Sequence"

<400> 582
tggagagcta ctattttcag aaacg                                          25


<210> 583
<211> 26
<212> DNA
<213> Artificial Sequence

<220>
<221> source
<222> 1..26
<223> /mol_type="unassigned DNA"
      /note="Oligonucleotide forward primer designed on
      the basis of MLH1 gene sequence enabling amplification of a region
      comprising genetic mutation site c.1733A>G"
      /organism="Artificial Sequence"

<400> 583
gggatgagtg ttacatttat ggtttc                                          26


<210> 584
<211> 24
<212> DNA
<213> Artificial Sequence

<220>
<221> source
<222> 1..24
<223> /mol_type="unassigned DNA"
      /note="Oligonucleotide reverse primer designed
      on the basis of MLH1 gene sequence enabling amplification
      of a region comprising genetic mutation site c.1733A>G"
      /organism="Artificial Sequence"

<400> 584
gtataagaat ggctgtcaca cctc                                            24


<210> 585
<211> 26
<212> DNA
<213> Artificial Sequence

<220>
<221> source
<222> 1..26
<223> /mol_type="unassigned DNA"
      /note="Oligonucleotide forward primer designed on
      the basis of MLH1 gene sequence enabling amplification of a region
      comprising genetic mutation site c.1744C>G"
      /organism="Artificial Sequence"

<400> 585
gggatgagtg ttacatttat ggtttc                                          26


<210> 586
<211> 24
<212> DNA
<213> Artificial Sequence

<220>
<221> source
<222> 1..24
<223> /mol_type="unassigned DNA"

/note="Oligonucleotide reverse primer designed
     on the basis of MLH1 gene sequence enabling amplification
     of a region comprising genetic mutation site c.1744C>G"
     /organism="Artificial Sequence"

<400> 586
gtataagaat ggctgtcaca cctc                                              24


<210> 587
<211> 26
<212> DNA
<213> Artificial Sequence

<220>
<221> source
<222> 1..26
<223> /mol_type="unassigned DNA"
     /note="Oligonucleotide forward primer designed on
     the basis of MLH1 gene sequence enabling amplification of a region
     comprising genetic mutation site c.1756G>C"
     /organism="Artificial Sequence"

<400> 587
gggatgagtg ttacatttat ggtttc                                            26


<210> 588
<211> 24
<212> DNA
<213> Artificial Sequence

<220>
<221> source
<222> 1..24
<223> /mol_type="unassigned DNA"
     /note="Oligonucleotide reverse primer designed
     on the basis of MLH1 gene sequence enabling amplification
     of a region comprising genetic mutation site c.1756G>C"
     /organism="Artificial Sequence"

<400> 588
gtataagaat ggctgtcaca cctc                                              24


<210> 589
<211> 26
<212> DNA
<213> Artificial Sequence

<220>
<221> source
<222> 1..26
<223> /mol_type="unassigned DNA"
     /note="Oligonucleotide forward primer designed on
     the basis of MLH1 gene sequence enabling amplification of a region
     comprising genetic mutation site c.1766C>A"
     /organism="Artificial Sequence"

<400> 589
gggatgagtg ttacatttat ggtttc                                          26


<210> 590
<211> 24
<212> DNA
<213> Artificial Sequence

<220>
<221> source
<222> 1..24
<223> /mol_type="unassigned DNA"
      /note="Oligonucleotide reverse primer designed
      on the basis of MLH1 gene sequence enabling amplification
      of a region comprising genetic mutation site c.1766C>A"
      /organism="Artificial Sequence"

<400> 590
gtataagaat ggctgtcaca cctc                                            24


<210> 591
<211> 26
<212> DNA
<213> Artificial Sequence

<220>
<221> source
<222> 1..26
<223> /mol_type="unassigned DNA"
      /note="Oligonucleotide forward primer designed on
      the basis of MLH1 gene sequence enabling amplification of a region
      comprising genetic mutation site c.1783delAG"
      /organism="Artificial Sequence"

<400> 591
gggatgagtg ttacatttat ggtttc                                          26


<210> 592
<211> 24
<212> DNA
<213> Artificial Sequence

<220>
<221> source
<222> 1..24
<223> /mol_type="unassigned DNA"
      /note="Oligonucleotide reverse primer designed
      on the basis of MLH1 gene sequence enabling amplification
      of a region comprising genetic mutation site c.1783delAG"
      /organism="Artificial Sequence"

<400> 592
gtataagaat ggctgtcaca cctc                                            24

<210> 593
<211> 26
<212> DNA
<213> Artificial Sequence

<220>
<221> source
<222> 1..26
<223> /mol_type="unassigned DNA"
      /note="Oligonucleotide forward primer designed on
      the basis of MLH1 gene sequence enabling amplification of a region
      comprising genetic mutation site c.1808C>G"
      /organism="Artificial Sequence"

<400> 593
gggatgagtg ttacatttat ggtttc                                          26


<210> 594
<211> 24
<212> DNA
<213> Artificial Sequence

<220>
<221> source
<222> 1..24
<223> /mol_type="unassigned DNA"
      /note="Oligonucleotide reverse primer designed
      on the basis of MLH1 gene sequence enabling amplification
      of a region comprising genetic mutation site c.1808C>G"
      /organism="Artificial Sequence"

<400> 594
gtataagaat ggctgtcaca cctc                                            24


<210> 595
<211> 26
<212> DNA
<213> Artificial Sequence

<220>
<221> source
<222> 1..26
<223> /mol_type="unassigned DNA"
      /note="Oligonucleotide forward primer designed on
      the basis of MLH1 gene sequence enabling amplification of a region
      comprising genetic mutation site c.1820T>A"
      /organism="Artificial Sequence"

<400> 595
gggatgagtg ttacatttat ggtttc                                          26


<210> 596
<211> 24
<212> DNA

<213> Artificial Sequence

<220>
<221> source
<222> 1..24
<223> /mol_type="unassigned DNA"
      /note="Oligonucleotide reverse primer designed
      on the basis of MLH1 gene sequence enabling amplification
      of a region comprising genetic mutation site c.1820T>A"
      /organism="Artificial Sequence"

<400> 596
gtataagaat ggctgtcaca cctc                                              24


<210> 597
<211> 26
<212> DNA
<213> Artificial Sequence

<220>
<221> source
<222> 1..26
<223> /mol_type="unassigned DNA"
      /note="Oligonucleotide forward primer designed on
      the basis of MLH1 gene sequence enabling amplification of a region
      comprising genetic mutation site c.1846delAAG"
      /organism="Artificial Sequence"

<400> 597
gggatgagtg ttacatttat ggtttc                                            26


<210> 598
<211> 24
<212> DNA
<213> Artificial Sequence

<220>
<221> source
<222> 1..24
<223> /mol_type="unassigned DNA"
      /note="Oligonucleotide reverse primer designed
      on the basis of MLH1 gene sequence enabling amplification
      of a region comprising genetic mutation site c.1846delAAG"
      /organism="Artificial Sequence"

<400> 598
gtataagaat ggctgtcaca cctc                                              24


<210> 599
<211> 26
<212> DNA
<213> Artificial Sequence

<220>
<221> source

<222> 1..26
<223> /mol_type="unassigned DNA"
      /note="Oligonucleotide forward primer designed on
      the basis of MLH1 gene sequence enabling amplification of a region
      comprising genetic mutation site c.1852_1853delAAinsGC"
      /organism="Artificial Sequence"

<400> 599
gggatgagtg ttacatttat ggtttc                                          26


<210> 600
<211> 24
<212> DNA
<213> Artificial Sequence

<220>
<221> source
<222> 1..24
<223> /mol_type="unassigned DNA"
      /note="Oligonucleotide reverse primer designed
      on the basis of MLH1 gene sequence enabling amplification
      of a region comprising genetic mutation site
      c.1852_1853delAAinsGC"
      /organism="Artificial Sequence"

<400> 600
gtataagaat ggctgtcaca cctc                                            24


<210> 601
<211> 26
<212> DNA
<213> Artificial Sequence

<220>
<221> source
<222> 1..26
<223> /mol_type="unassigned DNA"
      /note="Oligonucleotide forward primer designed on
      the basis of MLH1 gene sequence enabling amplification of a region
      comprising genetic mutation site c.1852delAAG"
      /organism="Artificial Sequence"

<400> 601
gggatgagtg ttacatttat ggtttc                                          26


<210> 602
<211> 24
<212> DNA
<213> Artificial Sequence

<220>
<221> source
<222> 1..24
<223> /mol_type="unassigned DNA"
      /note="Oligonucleotide reverse primer designed

on the basis of MLH1 gene sequence enabling amplification
      of a region comprising genetic mutation site c.1852delAAG"
      /organism="Artificial Sequence"

<400> 602
gtataagaat ggctgtcaca cctc                                              24


<210> 603
<211> 26
<212> DNA
<213> Artificial Sequence

<220>
<221> source
<222> 1..26
<223> /mol_type="unassigned DNA"
      /note="Oligonucleotide forward primer designed on
      the basis of MLH1 gene sequence enabling amplification of a region
      comprising genetic mutation site c.1853A>C/G"
      /organism="Artificial Sequence"

<400> 603
gggatgagtg ttacatttat ggtttc                                            26


<210> 604
<211> 24
<212> DNA
<213> Artificial Sequence

<220>
<221> source
<222> 1..24
<223> /mol_type="unassigned DNA"
      /note="Oligonucleotide reverse primer designed
      on the basis of MLH1 gene sequence enabling amplification
      of a region comprising genetic mutation site c.1853A>C/G"
      /organism="Artificial Sequence"

<400> 604
gtataagaat ggctgtcaca cctc                                              24


<210> 605
<211> 26
<212> DNA
<213> Artificial Sequence

<220>
<221> source
<222> 1..26
<223> /mol_type="unassigned DNA"
      /note="Oligonucleotide forward primer designed on
      the basis of MLH1 gene sequence enabling amplification of a region
      comprising genetic mutation site c.1865T>A"
      /organism="Artificial Sequence"

<400> 605
gggatgagtg ttacatttat ggtttc                                            26


<210> 606
<211> 24
<212> DNA
<213> Artificial Sequence

<220>
<221> source
<222> 1..24
<223> /mol_type="unassigned DNA"
      /note="Oligonucleotide reverse primer designed
      on the basis of MLH1 gene sequence enabling amplification
      of a region comprising genetic mutation site c.1865T>A"
      /organism="Artificial Sequence"

<400> 606
gtataagaat ggctgtcaca cctc                                              24


<210> 607
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1918C>T"
      /organism="Artificial Sequence"

<400> 607
gtggcagata ggagcacaag g                                                 21


<210> 608
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.1918C>T"
      /organism="Artificial Sequence"

<400> 608
gagatgggca agtttcatct cc                                                22

<210> 609
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1937A>G"
      /organism="Artificial Sequence"

<400> 609
gtggcagata ggagcacaag g                                                21


<210> 610
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.1937A>G"
      /organism="Artificial Sequence"

<400> 610
gagatgggca agtttcatct cc                                               22


<210> 611
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1942C>T"
      /organism="Artificial Sequence"

<400> 611
gtggcagata ggagcacaag g                                                21


<210> 612
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.1942C>T"
      /organism="Artificial Sequence"

<400> 612
gagatgggca agtttcatct cc                                              22


<210> 613
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1943C>T"
      /organism="Artificial Sequence"

<400> 613
gtggcagata ggagcacaag g                                               21


<210> 614
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.1943C>T"
      /organism="Artificial Sequence"

<400> 614
gagatgggca agtttcatct cc                                              22


<210> 615
<211> 21
<212> DNA
<213> Artificial Sequence

<220>
<221> source
<222> 1..21

<223> /mol_type="unassigned DNA"
      /note="Oligonucleotide forward primer designed on
      the basis of MLH1 gene sequence enabling amplification of a region
      comprising genetic mutation site c.1958T>G"
      /organism="Artificial Sequence"

<400> 615
gtggcagata ggagcacaag g                                                21


<210> 616
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.1958T>G"
      /organism="Artificial Sequence"

<400> 616
gagatgggca agtttcatct cc                                               22


<210> 617
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1959G>T"
      /organism="Artificial Sequence"

<400> 617
gtggcagata ggagcacaag g                                                21


<210> 618
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.1959G>T"

/organism="Artificial Sequence"

<400> 618
gagatgggca agtttcatct cc                                                22


<210> 619
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1961C>T"
      /organism="Artificial Sequence"

<400> 619
gtggcagata ggagcacaag g                                                 21


<210> 620
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.1961C>T"
      /organism="Artificial Sequence"

<400> 620
gagatgggca agtttcatct cc                                                22


<210> 621
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1963A>G"
      /organism="Artificial Sequence"

<400> 621
gtggcagata ggagcacaag g                                                 21

<210> 622
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.1963A>G"
      /organism="Artificial Sequence"

<400> 622
gagatgggca agtttcatct cc                                              22


<210> 623
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1964T>C"
      /organism="Artificial Sequence"

<400> 623
gtggcagata ggagcacaag g                                               21


<210> 624
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.1964T>C"
      /organism="Artificial Sequence"

<400> 624
gagatgggca agtttcatct cc                                              22


<210> 625
<211> 21

<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1976G>A/C"
      /organism="Artificial Sequence"

<400> 625
gtggcagata ggagcacaag g                                                21


<210> 626
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.1976G>A/C"
      /organism="Artificial Sequence"

<400> 626
gagatgggca agtttcatct cc                                               22


<210> 627
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1984A>C"
      /organism="Artificial Sequence"

<400> 627
gtggcagata ggagcacaag g                                                21


<210> 628
<211> 22
<212> DNA
<213> Artificial Sequence

<220>

<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.1984A>C"
      /organism="Artificial Sequence"

<400> 628
gagatgggca agtttcatct cc                                                22


<210> 629
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.1989G>T"
      /organism="Artificial Sequence"

<400> 629
gtggcagata ggagcacaag g                                                 21


<210> 630
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.1989G>T"
      /organism="Artificial Sequence"

<400> 630
gagatgggca agtttcatct cc                                                22


<210> 631
<211> 21
<212> DNA
<213> Artificial Sequence

<220>
<221> source
<222> 1..21
<223> /mol_type="unassigned DNA"
      /note="Oligonucleotide forward primer designed on

the basis of MLH1 gene sequence enabling amplification of a region
      comprising genetic mutation site c.2027T>G"
      /organism="Artificial Sequence"

<400> 631
gtggcagata ggagcacaag g                                                21


<210> 632
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.2027T>G"
      /organism="Artificial Sequence"

<400> 632
gagatgggca agtttcatct cc                                               22


<210> 633
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.2040C>A"
      /organism="Artificial Sequence"

<400> 633
gtggcagata ggagcacaag g                                                21


<210> 634
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.2040C>A"
      /organism="Artificial Sequence"

<400> 634
gagatgggca agtttcatct cc                                                22

<210> 635
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.2041G>A"
      /organism="Artificial Sequence"

<400> 635
gtggcagata ggagcacaag g                                                 21

<210> 636
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.2041G>A"
      /organism="Artificial Sequence"

<400> 636
gagatgggca agtttcatct cc                                                22

<210> 637
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.2059C>T"
      /organism="Artificial Sequence"

<400> 637
gtggcagata ggagcacaag g                                                 21

<210> 638
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.2059C>T"
      /organism="Artificial Sequence"

<400> 638
gagatgggca agtttcatct cc                                              22


<210> 639
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.2066A>G"
      /organism="Artificial Sequence"

<400> 639
gtggcagata ggagcacaag g                                               21


<210> 640
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.2066A>G"
      /organism="Artificial Sequence"

<400> 640
gagatgggca agtttcatct cc                                              22


<210> 641
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.2103G>C"
      /organism="Artificial Sequence"

<400> 641
gtggcagata ggagcacaag g                                                21


<210> 642
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH1 gene sequence enabling amplification
      of a region comprising genetic mutation site c.2103G>C"
      /organism="Artificial Sequence"

<400> 642
gagatgggca agtttcatct cc                                               22


<210> 643
<211> 21
<212> DNA
<213> Artificial Sequence

<220>
<221> source
<222> 1..21
<223> /mol_type="unassigned DNA"
      /note="Oligonucleotide forward primer designed on
      the basis of MLH1 gene sequence enabling amplification of a region
      comprising genetic mutation site c.2103+1G>A"
      /organism="Artificial Sequence"

<400> 643
gtggcagata ggagcacaag g                                                21


<210> 644
<211> 22
<212> DNA
<213> Artificial Sequence

<220>
<221> source
<222> 1..22

<223> /mol_type="unassigned DNA"
      /note="Oligonucleotide reverse primer designed
      on the basis of MLH1 gene sequence enabling amplification
      of a region comprising genetic mutation site c.2103+1G>A"
      /organism="Artificial Sequence"

<400> 644
gagatgggca agtttcatct cc                                                22


<210> 645
<211> 19
<212> DNA
<213> Artificial Sequence

<220>
<221> source
<222> 1..19
<223> /mol_type="unassigned DNA"
      /note="Oligonucleotide forward primer designed on
      the basis of MLH1 gene sequence enabling amplification of a region
      comprising genetic mutation site c.2146G>A"
      /organism="Artificial Sequence"

<400> 645
acccagtgca catcccatc                                                    19


<210> 646
<211> 27
<212> DNA
<213> Artificial Sequence

<220>
<221> source
<222> 1..27
<223> /mol_type="unassigned DNA"
      /note="Oligonucleotide reverse primer designed
      on the basis of MLH1 gene sequence enabling amplification
      of a region comprising genetic mutation site c.2146G>A"
      /organism="Artificial Sequence"

<400> 646
aggcaagtat aagtcttaag tgctacc                                           27


<210> 647
<211> 19
<212> DNA
<213> Artificial Sequence

<220>
<221> source
<222> 1..19
<223> /mol_type="unassigned DNA"
      /note="Oligonucleotide forward primer designed on
      the basis of MLH1 gene sequence enabling amplification of a region
      comprising genetic mutation site c.2152C>T"

/organism="Artificial Sequence"

<400> 647
acccagtgca catcccatc                                                    19


<210> 648
<211> 27
<212> DNA
<213> Artificial Sequence

<220>
<221> source
<222> 1..27
<223> /mol_type="unassigned DNA"
      /note="Oligonucleotide reverse primer designed
      on the basis of MLH1 gene sequence enabling amplification
      of a region comprising genetic mutation site c.2152C>T"
      /organism="Artificial Sequence"

<400> 648
aggcaagtat aagtcttaag tgctacc                                           27

<210> 649
<211> 19
<212> DNA
<213> Artificial Sequence

<220>
<221> source
<222> 1..19
<223> /mol_type="unassigned DNA"
      /note="Oligonucleotide forward primer designed on
      the basis of MLH1 gene sequence enabling amplification of a region
      comprising genetic mutation site c.2223del11"
      /organism="Artificial Sequence"

<400> 649
acccagtgca catcccatc                                                    19


<210> 650
<211> 27
<212> DNA
<213> Artificial Sequence

<220>
<221> source
<222> 1..27
<223> /mol_type="unassigned DNA"
      /note="Oligonucleotide reverse primer designed
      on the basis of MLH1 gene sequence enabling amplification
      of a region comprising genetic mutation site c.2223del11"
      /organism="Artificial Sequence"

<400> 650
aggcaagtat aagtcttaag tgctacc                                           27

<210> 651
<211> 19
<212> DNA
<213> Artificial Sequence

<220>
<221> source
<222> 1..19
<223> /mol_type="unassigned DNA"
      /note="Oligonucleotide forward primer designed on
      the basis of MLH1 gene sequence enabling amplification of a region
      comprising genetic mutation site c.2252A>G"
      /organism="Artificial Sequence"

<400> 651
acccagtgca catcccatc                                                    19


<210> 652
<211> 27
<212> DNA
<213> Artificial Sequence

<220>
<221> source
<222> 1..27
<223> /mol_type="unassigned DNA"
      /note="Oligonucleotide reverse primer designed
      on the basis of MLH1 gene sequence enabling amplification
      of a region comprising genetic mutation site c.2252A>G"
      /organism="Artificial Sequence"

<400> 652
aggcaagtat aagtcttaag tgctacc                                           27


<210> 653
<211> 20
<212> DNA
<213> Artificial Sequence

<220>
<221> source
<222> 1..20
<223> /mol_type="unassigned DNA"
      /note="Oligonucleotide forward primer designed on
      the basis of MSH2 gene sequence enabling amplification of a region
      comprising genetic mutation site c.-118T>C"
      /organism="Artificial Sequence"

<400> 653
agactcccac ccaccgaaac                                                   20


<210> 654
<211> 21
<212> DNA

<213> Artificial Sequence

<220>
<221> source
<222> 1..21
<223> /mol_type="unassigned DNA"
      /note="Oligonucleotide reverse primer designed
      on the basis of MLH2 gene sequence enabling amplification
      of a region comprising genetic mutation site c.-118T>C"
      /organism="Artificial Sequence"

<400> 654
ggccccatgt acttgatcac c                                                21


<210> 655
<211> 20
<212> DNA
<213> Artificial Sequence

<220>
<221> source
<222> 1..20
<223> /mol_type="unassigned DNA"
      /note="Oligonucleotide forward primer designed on
      the basis of MSH2 gene sequence enabling amplification of a region
      comprising genetic mutation site c.4G>A"
      /organism="Artificial Sequence"

<400> 655
agactcccac ccaccgaaac                                                  20


<210> 656
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH2 gene sequence enabling amplification
      of a region comprising genetic mutation site c.4G>A"
      /organism="Artificial Sequence"

<400> 656
ggccccatgt acttgatcac c                                                21


<210> 657
<211> 23
<212> DNA
<213> Artificial Sequence

<220>
<221> source

<222> 1..23
<223> /mol_type="unassigned DNA"
      /note="Oligonucleotide forward primer designed on
      the basis of MSH2 gene sequence enabling amplification of a region
      comprising genetic mutation site c.226C>T"
      /organism="Artificial Sequence"

<400> 657
ctttatcagc agcatgaagt cca                                              23


<210> 658
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH2 gene sequence enabling amplification
      of a region comprising genetic mutation site c.226C>T"
      /organism="Artificial Sequence"

<400> 658
ttttaaaaac cacatgctaa ttgct                                            25


<210> 659
<211> 23
<212> DNA
<213> Artificial Sequence

<220>
<221> source
<222> 1..23
<223> /mol_type="unassigned DNA"
      /note="Oligonucleotide forward primer designed on
      the basis of MSH2 gene sequence enabling amplification of a region
      comprising genetic mutation site c.339G>A"
      /organism="Artificial Sequence"

<400> 659
ctttatcagc agcatgaagt cca                                              23


<210> 660
<211> 25
<212> DNA
<213> Artificial Sequence

<220>
<221> source
<222> 1..25
<223> /mol_type="unassigned DNA"
      /note="Oligonucleotide reverse primer designed
      on the basis of MLH2 gene sequence enabling amplification

of a region comprising genetic mutation site c.339G>A"
/organism="Artificial Sequence"

<400> 660
ttttaaaaac cacatgctaa ttgct 25

<210> 661
<211> 21
<212> DNA
<213> Artificial Sequence

<220>
<221> source
<222> 1..21
<223> /mol_type="unassigned DNA"
/note="Oligonucleotide forward primer designed on
the basis of MSH2 gene sequence enabling amplification of a region
comprising genetic mutation site c.380A>G"
/organism="Artificial Sequence"

<400> 661
gaggcagagg ttgcagtgag t 21

<210> 662
<211> 21
<212> DNA
<213> Artificial Sequence

<220>
<221> source
<222> 1..21
<223> /mol_type="unassigned DNA"
/note="Oligonucleotide reverse primer designed
on the basis of MLH2 gene sequence enabling amplification
of a region comprising genetic mutation site c.380A>G"
/organism="Artificial Sequence"

<400> 662
tcctaggcct ggaatctcct c 21

<210> 663
<211> 21
<212> DNA
<213> Artificial Sequence

<220>
<221> source
<222> 1..21
<223> /mol_type="unassigned DNA"
/note="Oligonucleotide forward primer designed on
the basis of MSH2 gene sequence enabling amplification of a region
comprising genetic mutation site c.435T>G"
/organism="Artificial Sequence"

<400> 663

gaggcagagg ttgcagtgag t                                                21

<210> 664
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH2 gene sequence enabling amplification
      of a region comprising genetic mutation site c.435T>G"
      /organism="Artificial Sequence"

<400> 664
tcctaggcct ggaatctcct c                                                21

<210> 665
<211> 21
<212> DNA
<213> Artificial Sequence

<220>
<221> source
<222> 1..21
<223> /mol_type="unassigned DNA"
      /note="Oligonucleotide forward primer designed on
      the basis of MSH2 gene sequence enabling amplification of a region
      comprising genetic mutation site c.499G>C"
      /organism="Artificial Sequence"

<400> 665
gaggcagagg ttgcagtgag t                                                21

<210> 666
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH2 gene sequence enabling amplification
      of a region comprising genetic mutation site c.499G>C"
      /organism="Artificial Sequence"

<400> 666
tcctaggcct ggaatctcct c                                                21

<210> 667

<211> 21
<212> DNA
<213> Artificial Sequence

<220>
<221> source
<222> 1..21
<223> /mol_type="unassigned DNA"
      /note="Oligonucleotide forward primer designed on
      the basis of MSH2 gene sequence enabling amplification of a region
      comprising genetic mutation site c.505A>G"
      /organism="Artificial Sequence"

<400> 667
gaggcagagg ttgcagtgag t                                                21


<210> 668
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH2 gene sequence enabling amplification
      of a region comprising genetic mutation site c.505A>G"
      /organism="Artificial Sequence"

<400> 668
tcctaggcct ggaatctcct c                                                21


<210> 669
<211> 21
<212> DNA
<213> Artificial Sequence

<220>
<221> source
<222> 1..21
<223> /mol_type="unassigned DNA"
      /note="Oligonucleotide forward primer designed on
      the basis of MSH2 gene sequence enabling amplification of a region
      comprising genetic mutation site c.518T>C"
      /organism="Artificial Sequence"

<400> 669
gaggcagagg ttgcagtgag t                                                21


<210> 670
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH2 gene sequence enabling amplification
      of a region comprising genetic mutation site c.518T>C"
      /organism="Artificial Sequence"

<400> 670
tcctaggcct ggaatctcct c                                                21


<210> 671
<211> 21
<212> DNA
<213> Artificial Sequence

<220>
<221> source
<222> 1..21
<223> /mol_type="unassigned DNA"
      /note="Oligonucleotide forward primer designed on
      the basis of MSH2 gene sequence enabling amplification of a region
      comprising genetic mutation site c.560T>C"
      /organism="Artificial Sequence"

<400> 671
gaggcagagg ttgcagtgag t                                                21


<210> 672
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH2 gene sequence enabling amplification
      of a region comprising genetic mutation site c.560T>C"
      /organism="Artificial Sequence"

<400> 672
tcctaggcct ggaatctcct c                                                21


<210> 673
<211> 21
<212> DNA
<213> Artificial Sequence

<220>
<221> source
<222> 1..21
<223> /mol_type="unassigned DNA"

/note="Oligonucleotide forward primer designed on
      the basis of MSH2 gene sequence enabling amplification of a region
      comprising genetic mutation site c.593A>G"
      /organism="Artificial Sequence"

<400> 673
gaggcagagg ttgcagtgag t                                                21


<210> 674
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH2 gene sequence enabling amplification
      of a region comprising genetic mutation site c.593A>G"
      /organism="Artificial Sequence"

<400> 674
tcctaggcct ggaatctcct c                                                21


<210> 675
<211> 21
<212> DNA
<213> Artificial Sequence

<220>
<221> source
<222> 1..21
<223> /mol_type="unassigned DNA"
      /note="Oligonucleotide forward primer designed on
      the basis of MSH2 gene sequence enabling amplification of a region
      comprising genetic mutation site c.595T>C"
      /organism="Artificial Sequence"

<400> 675
gaggcagagg ttgcagtgag t                                                21


<210> 676
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH2 gene sequence enabling amplification
      of a region comprising genetic mutation site c.595T>C"
      /organism="Artificial Sequence"

<400> 676
tcctaggcct ggaatctcct c                                                21

<210> 677
<211> 27
<212> DNA
<213> Artificial Sequence

<220>
<221> source
<222> 1..27
<223> /mol_type="unassigned DNA"
      /note="Oligonucleotide forward primer designed on
      the basis of MSH2 gene sequence enabling amplification of a region
      comprising genetic mutation site c.687delA"
      /organism="Artificial Sequence"

<400> 677
tcagtacatc atatcagtgt cttgcac                                          27

<210> 678
<211> 27
<212> DNA
<213> Artificial Sequence

<220>
<221> source
<222> 1..27
<223> /mol_type="unassigned DNA"
      /note="Oligonucleotide reverse primer designed
      on the basis of MLH2 gene sequence enabling amplification
      of a region comprising genetic mutation site c.687delA"
      /organism="Artificial Sequence"

<400> 678
tcattgatac acagtttagg ttttgag                                          27

<210> 679
<211> 22
<212> DNA
<213> Artificial Sequence

<220>
<221> source
<222> 1..22
<223> /mol_type="unassigned DNA"
      /note="Oligonucleotide forward primer designed on
      the basis of MSH2 gene sequence enabling amplification of a region
      comprising genetic mutation site c.806C>T"
      /organism="Artificial Sequence"

<400> 679
ggaaggaaca ccaaggaaaa tg                                               22

<210> 680
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH2 gene sequence enabling amplification
      of a region comprising genetic mutation site c.806C>T"
      /organism="Artificial Sequence"

<400> 680
aggaggggag agaaaaatac agc                                              23


<210> 681
<211> 22
<212> DNA
<213> Artificial Sequence

<220>
<221> source
<222> 1..22
<223> /mol_type="unassigned DNA"
      /note="Oligonucleotide forward primer designed on
      the basis of MSH2 gene sequence enabling amplification of a region
      comprising genetic mutation site c.862C>T"
      /organism="Artificial Sequence"

<400> 681
ggaaggaaca ccaaggaaaa tg                                               22


<210> 682
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH2 gene sequence enabling amplification
      of a region comprising genetic mutation site c.862C>T"
      /organism="Artificial Sequence"

<400> 682
aggaggggag agaaaaatac agc                                              23


<210> 683
<211> 22
<212> DNA

<213> Artificial Sequence

<220>
<221> source
<222> 1..22
<223> /mol_type="unassigned DNA"
      /note="Oligonucleotide forward primer designed on
      the basis of MSH2 gene sequence enabling amplification of a region
      comprising genetic mutation site c.892C>T"
      /organism="Artificial Sequence"

<400> 683
ggaaggaaca ccaaggaaaa tg                                                22


<210> 684
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH2 gene sequence enabling amplification
      of a region comprising genetic mutation site c.892C>T"
      /organism="Artificial Sequence"

<400> 684
aggaggggag agaaaaatac agc                                               23


<210> 685
<211> 22
<212> DNA
<213> Artificial Sequence

<220>
<221> source
<222> 1..22
<223> /mol_type="unassigned DNA"
      /note="Oligonucleotide forward primer designed on
      the basis of MSH2 gene sequence enabling amplification of a region
      comprising genetic mutation site c.942+3A>T"
      /organism="Artificial Sequence"

<400> 685
tcctctgttt ttcatggcgt ag                                                22


<210> 686
<211> 24
<212> DNA
<213> Artificial Sequence

<220>
<221> source

<222> 1..24
<223> /mol_type="unassigned DNA"
      /note="Oligonucleotide reverse primer designed
      on the basis of MLH2 gene sequence enabling amplification
      of a region comprising genetic mutation site c.942+3A>T"
      /organism="Artificial Sequence"

<400> 686
tggtataatc atgtgggtaa ctgc                                              24


<210> 687
<211> 22
<212> DNA
<213> Artificial Sequence

<220>
<221> source
<222> 1..22
<223> /mol_type="unassigned DNA"
      /note="Oligonucleotide forward primer designed on
      the basis of MSH2 gene sequence enabling amplification of a region
      comprising genetic mutation site c.965G>A"
      /organism="Artificial Sequence"

<400> 687
tcctctgttt ttcatggcgt ag                                                22


<210> 688
<211> 24
<212> DNA
<213> Artificial Sequence

<220>
<221> source
<222> 1..24
<223> /mol_type="unassigned DNA"
      /note="Oligonucleotide reverse primer designed
      on the basis of MLH2 gene sequence enabling amplification
      of a region comprising genetic mutation site c.965G>A"
      /organism="Artificial Sequence"

<400> 688
tggtataatc atgtgggtaa ctgc                                              24


<210> 689
<211> 22
<212> DNA
<213> Artificial Sequence

<220>
<221> source
<222> 1..22
<223> /mol_type="unassigned DNA"
      /note="Oligonucleotide forward primer designed on
      the basis of MSH2 gene sequence enabling amplification of a region

comprising genetic mutation site c.984C>T"
      /organism="Artificial Sequence"

<400> 689
tcctctgttt ttcatggcgt ag                                              22


<210> 690
<211> 24
<212> DNA
<213> Artificial Sequence

<220>
<221> source
<222> 1..24
<223> /mol_type="unassigned DNA"
      /note="Oligonucleotide reverse primer designed
      on the basis of MLH2 gene sequence enabling amplification
      of a region comprising genetic mutation site c.984C>T"
      /organism="Artificial Sequence"

<400> 690
tggtataatc atgtgggtaa ctgc                                            24


<210> 691
<211> 22
<212> DNA
<213> Artificial Sequence

<220>
<221> source
<222> 1..22
<223> /mol_type="unassigned DNA"
      /note="Oligonucleotide forward primer designed on
      the basis of MSH2 gene sequence enabling amplification of a region
      comprising genetic mutation site c.998G>A"
      /organism="Artificial Sequence"

<400> 691
tcctctgttt ttcatggcgt ag                                              22


<210> 692
<211> 24
<212> DNA
<213> Artificial Sequence

<220>
<221> source
<222> 1..24
<223> /mol_type="unassigned DNA"
      /note="Oligonucleotide reverse primer designed
      on the basis of MLH2 gene sequence enabling amplification
      of a region comprising genetic mutation site c.998G>A"
      /organism="Artificial Sequence"

<400> 692

tggtataatc atgtgggtaa ctgc                                              24


<210> 693
<211> 25
<212> DNA
<213> Artificial Sequence

<220>
<221> source
<222> 1..25
<223> /mol_type="unassigned DNA"
      /note="Oligonucleotide forward primer designed on
      the basis of MSH2 gene sequence enabling amplification of a region
      comprising genetic mutation site c.1077-10T>C"
      /organism="Artificial Sequence"

<400> 693
tggaattttg agctgattta gttga                                             25


<210> 694
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH2 gene sequence enabling amplification
      of a region comprising genetic mutation site c.1077-10T>C"
      /organism="Artificial Sequence"

<400> 694
gaggacagca cattgccaag                                                   20


<210> 695
<211> 25
<212> DNA
<213> Artificial Sequence

<220>
<221> source
<222> 1..25
<223> /mol_type="unassigned DNA"
      /note="Oligonucleotide forward primer designed on
      the basis of MSH2 gene sequence enabling amplification of a region
      comprising genetic mutation site c.1077A>T"
      /organism="Artificial Sequence"

<400> 695
tggaattttg agctgattta gttga                                             25


<210> 696

<211> 20
<212> DNA
<213> Artificial Sequence

<220>
<221> source
<222> 1..20
<223> /mol_type="unassigned DNA"
      /note="Oligonucleotide reverse primer designed
      on the basis of MLH2 gene sequence enabling amplification
      of a region comprising genetic mutation site c.1077A>T"
      /organism="Artificial Sequence"

<400> 696
gaggacagca cattgccaag                                                   20


<210> 697
<211> 25
<212> DNA
<213> Artificial Sequence

<220>
<221> source
<222> 1..25
<223> /mol_type="unassigned DNA"
      /note="Oligonucleotide forward primer designed on
      the basis of MSH2 gene sequence enabling amplification of a region
      comprising genetic mutation site c.1147C>T"
      /organism="Artificial Sequence"

<400> 697
tggaattttg agctgattta gttga                                             25


<210> 698
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH2 gene sequence enabling amplification
      of a region comprising genetic mutation site c.1147C>T"
      /organism="Artificial Sequence"

<400> 698
gaggacagca cattgccaag                                                   20


<210> 699
<211> 25
<212> DNA
<213> Artificial Sequence

<220>
<221> source
<222> 1..25
<223> /mol_type="unassigned DNA"
      /note="Oligonucleotide forward primer designed on
      the basis of MSH2 gene sequence enabling amplification of a region
      comprising genetic mutation site c.1165C>T"
      /organism="Artificial Sequence"

<400> 699
tggaattttg agctgattta gttga                                          25


<210> 700
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH2 gene sequence enabling amplification
      of a region comprising genetic mutation site c.1165C>T"
      /organism="Artificial Sequence"

<400> 700
gaggacagca cattgccaag                                                20


<210> 701
<211> 25
<212> DNA
<213> Artificial Sequence

<220>
<221> source
<222> 1..25
<223> /mol_type="unassigned DNA"
      /note="Oligonucleotide forward primer designed on
      the basis of MSH2 gene sequence enabling amplification of a region
      comprising genetic mutation site c.1168C>T"
      /organism="Artificial Sequence"

<400> 701
tggaattttg agctgattta gttga                                          25


<210> 702
<211> 20
<212> DNA
<213> Artificial Sequence

<220>
<221> source
<222> 1..20
<223> /mol_type="unassigned DNA"

/note="Oligonucleotide reverse primer designed
on the basis of MLH2 gene sequence enabling amplification
of a region comprising genetic mutation site c.1168C>T"
/organism="Artificial Sequence"

<400> 702
gaggacagca cattgccaag                                                    20


<210> 703
<211> 25
<212> DNA
<213> Artificial Sequence

<220>
<221> source
<222> 1..25
<223> /mol_type="unassigned DNA"
/note="Oligonucleotide forward primer designed on
the basis of MSH2 gene sequence enabling amplification of a region
comprising genetic mutation site c.1216C>T"
/organism="Artificial Sequence"

<400> 703
tggaattttg agctgattta gttga                                              25


<210> 704
<211> 20
<212> DNA
<213> Artificial Sequence

<220>
<221> source
<222> 1..20
<223> /mol_type="unassigned DNA"
/note="Oligonucleotide reverse primer designed
on the basis of MLH2 gene sequence enabling amplification
of a region comprising genetic mutation site c.1216C>T"
/organism="Artificial Sequence"

<400> 704
gaggacagca cattgccaag                                                    20


<210> 705
<211> 25
<212> DNA
<213> Artificial Sequence

<220>
<221> source
<222> 1..25
<223> /mol_type="unassigned DNA"
/note="Oligonucleotide forward primer designed on
the basis of MSH2 gene sequence enabling amplification of a region
comprising genetic mutation site c.1226delAG"
/organism="Artificial Sequence"

<400> 705
tggaattttg agctgattta gttga                                          25


<210> 706
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH2 gene sequence enabling amplification
      of a region comprising genetic mutation site c.1226delAG"
      /organism="Artificial Sequence"

<400> 706
gaggacagca cattgccaag                                                20


<210> 707
<211> 25
<212> DNA
<213> Artificial Sequence

<220>
<221> source
<222> 1..25
<223> /mol_type="unassigned DNA"
      /note="Oligonucleotide forward primer designed on
      the basis of MSH2 gene sequence enabling amplification of a region
      comprising genetic mutation site c.1255C>A"
      /organism="Artificial Sequence"

<400> 707
tggaattttg agctgattta gttga                                          25


<210> 708
<211> 20
<212> DNA
<213> Artificial Sequence

<220>
<221> source
<222> 1..20
<223> /mol_type="unassigned DNA"
      /note="Oligonucleotide reverse primer designed
      on the basis of MLH2 gene sequence enabling amplification
      of a region comprising genetic mutation site c.1255C>A"
      /organism="Artificial Sequence"

<400> 708
gaggacagca cattgccaag                                                20

<210> 709
<211> 27
<212> DNA
<213> Artificial Sequence

<220>
<221> source
<222> 1..27
<223> /mol_type="unassigned DNA"
      /note="Oligonucleotide forward primer designed on
      the basis of MSH2 gene sequence enabling amplification of a region
      comprising genetic mutation site c.1373T>G"
      /organism="Artificial Sequence"

<400> 709
actactttct tttaggaaaa caccaga                                          27


<210> 710
<211> 24
<212> DNA
<213> Artificial Sequence

<220>
<221> source
<222> 1..24
<223> /mol_type="unassigned DNA"
      /note="Oligonucleotide reverse primer designed
      on the basis of MLH2 gene sequence enabling amplification
      of a region comprising genetic mutation site c.1373T>G"
      /organism="Artificial Sequence"

<400> 710
caaactttct taaagtggcc tttg                                             24


<210> 711
<211> 27
<212> DNA
<213> Artificial Sequence

<220>
<221> source
<222> 1..27
<223> /mol_type="unassigned DNA"
      /note="Oligonucleotide forward primer designed on
      the basis of MSH2 gene sequence enabling amplification of a region
      comprising genetic mutation site c.1571G>C"
      /organism="Artificial Sequence"

<400> 711
atggaatact ttttcttttc ttcttga                                          27


<210> 712
<211> 24
<212> DNA

<213> Artificial Sequence

<220>
<221> source
<222> 1..24
<223> /mol_type="unassigned DNA"
      /note="Oligonucleotide reverse primer designed
      on the basis of MLH2 gene sequence enabling amplification
      of a region comprising genetic mutation site c.1571G>C"
      /organism="Artificial Sequence"

<400> 712
cacatcatgt tagagcattt aggg                                              24


<210> 713
<211> 27
<212> DNA
<213> Artificial Sequence

<220>
<221> source
<222> 1..27
<223> /mol_type="unassigned DNA"
      /note="Oligonucleotide forward primer designed on
      the basis of MSH2 gene sequence enabling amplification of a region
      comprising genetic mutation site c.1654A>C"
      /organism="Artificial Sequence"

<400> 713
   atggaatact ttttcttttc ttcttga                                            27


<210> 714
<211> 24
<212> DNA
<213> Artificial Sequence

<220>
<221> source
<222> 1..24
<223> /mol_type="unassigned DNA"
      /note="Oligonucleotide reverse primer designed
      on the basis of MLH2 gene sequence enabling amplification
      of a region comprising genetic mutation site c.1654A>C"
      /organism="Artificial Sequence"

<400> 714
cacatcatgt tagagcattt aggg                                              24


<210> 715
<211> 27
<212> DNA
<213> Artificial Sequence

<220>
<221> source

<222> 1..27
<223> /mol_type="unassigned DNA"
      /note="Oligonucleotide forward primer designed on
      the basis of MSH2 gene sequence enabling amplification of a region
      comprising genetic mutation site c.1661+12G>A"
      /organism="Artificial Sequence"

<400> 715
atggaatact ttttcttttc ttcttga                                          27


<210> 716
<211> 24
<212> DNA
<213> Artificial Sequence

<220>
<221> source
<222> 1..24
<223> /mol_type="unassigned DNA"
      /note="Oligonucleotide reverse primer designed
      on the basis of MLH2 gene sequence enabling amplification
      of a region comprising genetic mutation site c.1661+12G>A"
      /organism="Artificial Sequence"

<400> 716
cacatcatgt tagagcattt aggg                                             24


<210> 717
<211> 25
<212> DNA
<213> Artificial Sequence

<220>
<221> source
<222> 1..25
<223> /mol_type="unassigned DNA"
      /note="Oligonucleotide forward primer designed on
      the basis of MSH2 gene sequence enabling amplification of a region
      comprising genetic mutation site c.1666T>C"
      /organism="Artificial Sequence"

<400> 717
tgtttcacgt agtacacatt gcttc                                            25


<210> 718
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH2 gene sequence enabling amplification

of a region comprising genetic mutation site c.1666T>C"
      /organism="Artificial Sequence"

<400> 718
caaaagccag gtgacattca ga                                              22


<210> 719
<211> 25
<212> DNA
<213> Artificial Sequence

<220>
<221> source
<222> 1..25
<223> /mol_type="unassigned DNA"
      /note="Oligonucleotide forward primer designed on
      the basis of MSH2 gene sequence enabling amplification of a region
      comprising genetic mutation site c.1737A>G"
      /organism="Artificial Sequence"

<400> 719
tgtttcacgt agtacacatt gcttc                                           25


<210> 720
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH2 gene sequence enabling amplification
      of a region comprising genetic mutation site c.1737A>G"
      /organism="Artificial Sequence"

<400> 720
caaaagccag gtgacattca ga                                              22


<210> 721
<211> 25
<212> DNA
<213> Artificial Sequence

<220>
<221> source
<222> 1..25
<223> /mol_type="unassigned DNA"
      /note="Oligonucleotide forward primer designed on
      the basis of MSH2 gene sequence enabling amplification of a region
      comprising genetic mutation site c.1738G>T"
      /organism="Artificial Sequence"

<400> 721

tgtttcacgt agtacacatt gcttc                                          25

<210> 722
<211> 22
<212> DNA
<213> Artificial Sequence

<220>
<221> source
<222> 1..22
<223> /mol_type="unassigned DNA"
      /note="Oligonucleotide reverse primer designed
      on the basis of MLH2 gene sequence enabling amplification
      of a region comprising genetic mutation site c.1738G>T"
      /organism="Artificial Sequence"

<400> 722
caaaagccag gtgacattca ga                                             22

<210> 723
<211> 24
<212> DNA
<213> Artificial Sequence

<220>
<221> source
<222> 1..24
<223> /mol_type="unassigned DNA"
      /note="Oligonucleotide forward primer designed on
      the basis of MSH2 gene sequence enabling amplification of a region
      comprising genetic mutation site c.1786delAAT"
      /organism="Artificial Sequence"

<400> 723
gggggattaa atgtattttt acgg                                           24

<210> 724
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH2 gene sequence enabling amplification
      of a region comprising genetic mutation site c.1786delAAT"
      /organism="Artificial Sequence"

<400> 724
caaaacgtta cccccacaaa g                                              21

<210> 725

<211> 24
<212> DNA
<213> Artificial Sequence

<220>
<221> source
<222> 1..24
<223> /mol_type="unassigned DNA"
      /note="Oligonucleotide forward primer designed on
      the basis of MSH2 gene sequence enabling amplification of a region
      comprising genetic mutation site c.1787A>G"
      /organism="Artificial Sequence"

<400> 725
gggggattaa atgtattttt acgg                                              24


<210> 726
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH2 gene sequence enabling amplification
      of a region comprising genetic mutation site c.1787A>G"
      /organism="Artificial Sequence"

<400> 726
caaaacgtta cccccacaaa g                                                 21


<210> 727
<211> 24
<212> DNA
<213> Artificial Sequence

<220>
<221> source
<222> 1..24
<223> /mol_type="unassigned DNA"
      /note="Oligonucleotide forward primer designed on
      the basis of MSH2 gene sequence enabling amplification of a region
      comprising genetic mutation site c.1799C>T"
      /organism="Artificial Sequence"

<400> 727
gggggattaa atgtattttt acgg                                              24


<210> 728
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH2 gene sequence enabling amplification
      of a region comprising genetic mutation site c.1799C>T"
      /organism="Artificial Sequence"

<400> 728
caaaacgtta cccccacaaa g                                                21


<210> 729
<211> 24
<212> DNA
<213> Artificial Sequence

<220>
<221> source
<222> 1..24
<223> /mol_type="unassigned DNA"
      /note="Oligonucleotide forward primer designed on
      the basis of MSH2 gene sequence enabling amplification of a region
      comprising genetic mutation site c.1807G>A"
      /organism="Artificial Sequence"

<400> 729
gggggattaa atgtattttt acgg                                             24


<210> 730
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH2 gene sequence enabling amplification
      of a region comprising genetic mutation site c.1807G>A"
      /organism="Artificial Sequence"

<400> 730
caaaacgtta cccccacaaa g                                                21


<210> 731
<211> 24
<212> DNA
<213> Artificial Sequence

<220>
<221> source
<222> 1..24
<223> /mol_type="unassigned DNA"

/note="Oligonucleotide forward primer designed on
      the basis of MSH2 gene sequence enabling amplification of a region
      comprising genetic mutation site c.1861C>T"
      /organism="Artificial Sequence"

<400> 731
gggggattaa atgtattttt acgg                                              24


<210> 732
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH2 gene sequence enabling amplification
      of a region comprising genetic mutation site c.1861C>T"
      /organism="Artificial Sequence"

<400> 732
caaaacgtta cccccacaaa g                                                 21


<210> 733
<211> 24
<212> DNA
<213> Artificial Sequence

<220>
<221> source
<222> 1..24
<223> /mol_type="unassigned DNA"
      /note="Oligonucleotide forward primer designed on
      the basis of MSH2 gene sequence enabling amplification of a region
      comprising genetic mutation site c.1865C>T"
      /organism="Artificial Sequence"

<400> 733
gggggattaa atgtattttt acgg                                              24


<210> 734
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH2 gene sequence enabling amplification
      of a region comprising genetic mutation site c.1865C>T"
      /organism="Artificial Sequence"

<400> 734
caaaacgtta cccccacaaa g                                                21


<210> 735
<211> 24
<212> DNA
<213> Artificial Sequence

<220>
<221> source
<222> 1..24
<223> /mol_type="unassigned DNA"
      /note="Oligonucleotide forward primer designed on
      the basis of MSH2 gene sequence enabling amplification of a region
      comprising genetic mutation site c.1906G>C"
      /organism="Artificial Sequence"

<400> 735
gggggattaa atgtattttt acgg                                             24


<210> 736
<211> 21
<212> DNA
<213> Artificial Sequence

<220>
<221> source
<222> 1..21
<223> /mol_type="unassigned DNA"
      /note="Oligonucleotide reverse primer designed
      on the basis of MLH2 gene sequence enabling amplification
      of a region comprising genetic mutation site c.1906G>C"
      /organism="Artificial Sequence"

<400> 736
caaaacgtta cccccacaaa g                                                21


<210> 737
<211> 24
<212> DNA
<213> Artificial Sequence

<220>
<221> source
<222> 1..24
<223> /mol_type="unassigned DNA"
      /note="Oligonucleotide forward primer designed on
      the basis of MSH2 gene sequence enabling amplification of a region
      comprising genetic mutation site c.2006-6T>C"
      /organism="Artificial Sequence"

<400> 737
aacctacgcg attaatcatc agtg                                             24

<210> 738
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH2 gene sequence enabling amplification
      of a region comprising genetic mutation site c.2006-6T>C"
      /organism="Artificial Sequence"

<400> 738
ttccttctca caggacagag aca                                              23


<210> 739
<211> 24
<212> DNA
<213> Artificial Sequence

<220>
<221> source
<222> 1..24
<223> /mol_type="unassigned DNA"
      /note="Oligonucleotide forward primer designed on
      the basis of MSH2 gene sequence enabling amplification of a region
      comprising genetic mutation site c.2064G>A"
      /organism="Artificial Sequence"

<400> 739
aacctacgcg attaatcatc agtg                                             24


<210> 740
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH2 gene sequence enabling amplification
      of a region comprising genetic mutation site c.2064G>A"
      /organism="Artificial Sequence"

<400> 740
ttccttctca caggacagag aca                                              23


<210> 741
<211> 24
<212> DNA

<213> Artificial Sequence

<220>
<221> source
<222> 1..24
<223> /mol_type="unassigned DNA"
      /note="Oligonucleotide forward primer designed on
      the basis of MSH2 gene sequence enabling amplification of a region
      comprising genetic mutation site c.2089T>C"
      /organism="Artificial Sequence"

<400> 741
aacctacgcg attaatcatc agtg                                              24


<210> 742
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH2 gene sequence enabling amplification
      of a region comprising genetic mutation site c.2089T>C"
      /organism="Artificial Sequence"

<400> 742
ttccttctca caggacagag aca                                               23


<210> 743
<211> 24
<212> DNA
<213> Artificial Sequence

<220>
<221> source
<222> 1..24
<223> /mol_type="unassigned DNA"
      /note="Oligonucleotide forward primer designed on
      the basis of MSH2 gene sequence enabling amplification of a region
      comprising genetic mutation site c.2090G>T"
      /organism="Artificial Sequence"

<400> 743
aacctacgcg attaatcatc agtg                                              24


<210> 744
<211> 23
<212> DNA
<213> Artificial Sequence

<220>
<221> source

<222> 1..23
<223> /mol_type="unassigned DNA"
      /note="Oligonucleotide reverse primer designed
      on the basis of MLH2 gene sequence enabling amplification
      of a region comprising genetic mutation site c.2090G>T"
      /organism="Artificial Sequence"

<400> 744
ttccttctca caggacagag aca                                               23


<210> 745
<211> 24
<212> DNA
<213> Artificial Sequence

<220>
<221> source
<222> 1..24
<223> /mol_type="unassigned DNA"
      /note="Oligonucleotide forward primer designed on
      the basis of MSH2 gene sequence enabling amplification of a region
      comprising genetic mutation site c.2131C>T"
      /organism="Artificial Sequence"

<400> 745
aacctacgcg attaatcatc agtg                                              24


<210> 746
<211> 23
<212> DNA
<213> Artificial Sequence

<220>
<221> source
<222> 1..23
<223> /mol_type="unassigned DNA"
      /note="Oligonucleotide reverse primer designed
      on the basis of MLH2 gene sequence enabling amplification
      of a region comprising genetic mutation site c.2131C>T"
      /organism="Artificial Sequence"

<400> 746
ttccttctca caggacagag aca                                               23


<210> 747
<211> 24
<212> DNA
<213> Artificial Sequence

<220>
<221> source
<222> 1..24
<223> /mol_type="unassigned DNA"
      /note="Oligonucleotide forward primer designed on
      the basis of MSH2 gene sequence enabling amplification of a region

comprising genetic mutation site c.2168C>T"
/organism="Artificial Sequence"

<400> 747
aacctacgcg attaatcatc agtg                                              24


<210> 748
<211> 23
<212> DNA
<213> Artificial Sequence

<220>
<221> source
<222> 1..23
<223> /mol_type="unassigned DNA"
/note="Oligonucleotide reverse primer designed
on the basis of MLH2 gene sequence enabling amplification
of a region comprising genetic mutation site c.2168C>T"
/organism="Artificial Sequence"

<400> 748
ttccttctca caggacagag aca                                               23


<210> 749
<211> 21
<212> DNA
<213> Artificial Sequence

<220>
<221> source
<222> 1..21
<223> /mol_type="unassigned DNA"
/note="Oligonucleotide forward primer designed on
the basis of MSH2 gene sequence enabling amplification of a region
comprising genetic mutation site c.2245G>A"
/organism="Artificial Sequence"

<400> 749
tgtggcatat ccttcccaat g                                                 21


<210> 750
<211> 24
<212> DNA
<213> Artificial Sequence

<220>
<221> source
<222> 1..24
<223> /mol_type="unassigned DNA"
/note="Oligonucleotide reverse primer designed
on the basis of MLH2 gene sequence enabling amplification
of a region comprising genetic mutation site c.2245G>A"
/organism="Artificial Sequence"

<400> 750

aagtttccca ttaccaagtt ctga                                          24

<210> 751
<211> 21
<212> DNA
<213> Artificial Sequence

<220>
<221> source
<222> 1..21
<223> /mol_type="unassigned DNA"
      /note="Oligonucleotide forward primer designed on
      the basis of MSH2 gene sequence enabling amplification of a region
      comprising genetic mutation site c.2251G>A"
      /organism="Artificial Sequence"

<400> 751
tgtggcatat ccttcccaat g                                               21

<210> 752
<211> 24
<212> DNA
<213> Artificial Sequence

<220>
<221> source
<222> 1..24
<223> /mol_type="unassigned DNA"
      /note="Oligonucleotide reverse primer designed
      on the basis of MLH2 gene sequence enabling amplification
      of a region comprising genetic mutation site c.2251G>A"
      /organism="Artificial Sequence"

<400> 752
aagtttccca ttaccaagtt ctga                                          24

<210> 753
<211> 25
<212> DNA
<213> Artificial Sequence

<220>
<221> source
<222> 1..25
<223> /mol_type="unassigned DNA"
      /note="Oligonucleotide forward primer designed on
      the basis of MSH2 gene sequence enabling amplification of a region
      comprising genetic mutation site c.2500G>A"
      /organism="Artificial Sequence"

<400> 753
tgacaaggtg agaaggataa attcc                                         25

<210> 754

<211> 24
<212> DNA
<213> Artificial Sequence

<220>
<221> source
<222> 1..24
<223> /mol_type="unassigned DNA"
      /note="Oligonucleotide reverse primer designed
      on the basis of MLH2 gene sequence enabling amplification
      of a region comprising genetic mutation site c.2500G>A"
      /organism="Artificial Sequence"

<400> 754
cacagaggaa aacaaacaac aaca                                              24


<210> 755
<211> 25
<212> DNA
<213> Artificial Sequence

<220>
<221> source
<222> 1..25
<223> /mol_type="unassigned DNA"
      /note="Oligonucleotide forward primer designed on
      the basis of MSH2 gene sequence enabling amplification of a region
      comprising genetic mutation site c.2633delAG"
      /organism="Artificial Sequence"

<400> 755
tgacaaggtg agaaggataa attcc                                             25


<210> 756
<211> 24
<212> DNA
<213> Artificial Sequence

<220>
<221> source
<222> 1..24
<223> /mol_type="unassigned DNA"
      /note="Oligonucleotide reverse primer designed
      on the basis of MLH2 gene sequence enabling amplification
      of a region comprising genetic mutation site c.2633delAG"
      /organism="Artificial Sequence"

<400> 756
cacagaggaa aacaaacaac aaca                                              24


<210> 757
<211> 26
<212> DNA
<213> Artificial Sequence

<220>
<221> source
<222> 1..26
<223> /mol_type="unassigned DNA"
      /note="Oligonucleotide forward primer designed on
      the basis of MSH2 gene sequence enabling amplification of a region
      comprising genetic mutation site c.2714C>G"
      /organism="Artificial Sequence"

<400> 757
tgtgtgatat gtttagatgg aaatga                                          26


<210> 758
<211> 26
<212> DNA
<213> Artificial Sequence

<220>
<221> source
<222> 1..26
<223> /mol_type="unassigned DNA"
      /note="Oligonucleotide reverse primer designed
      on the basis of MLH2 gene sequence enabling amplification
      of a region comprising genetic mutation site c.2714C>G"
      /organism="Artificial Sequence"

<400> 758
ccctccagtt taagatattt tacgag                                          26


<210> 759
<211> 23
<212> DNA
<213> Artificial Sequence

<220>
<221> source
<222> 1..23
<223> /mol_type="unassigned DNA"
      /note="Oligonucleotide forward primer designed on
      the basis of MSH6 gene sequence enabling amplification of a region
      comprising genetic mutation site c.276A>G"
      /organism="Artificial Sequence"

<400> 759
ctgcctttaa ggaaacttga cca                                             23


<210> 760
<211> 21
<212> DNA
<213> Artificial Sequence

<220>
<221> source
<222> 1..21
<223> /mol_type="unassigned DNA"

      /note="Oligonucleotide reverse primer designed
      on the basis of MSH6 gene sequence enabling amplification
      of a region comprising genetic mutation site c.276A>G"
      /organism="Artificial Sequence"

<400> 760
ccaccccttg caatttactg g                                                21


<210> 761
<211> 23
<212> DNA
<213> Artificial Sequence

<220>
<221> source
<222> 1..23
<223> /mol_type="unassigned DNA"
      /note="Oligonucleotide forward primer designed on
      the basis of MSH6 gene sequence enabling amplification of a region
      comprising genetic mutation site c.431G>T"
      /organism="Artificial Sequence"

<400> 761
ctgcctttaa ggaaacttga cca                                              23


<210> 762
<211> 21
<212> RNA
<213> Artificial Sequence

<220>
<221> source
<222> 1..21
<223> /mol_type="unassigned RNA"
      /note="Oligonucleotide reverse primer designed
      on the basis of MSH6 gene sequence enabling amplification
      of a region comprising genetic mutation site c.431G>T"
      /organism="Artificial Sequence"

<400> 762
ccaccccttg caatttactg g                                                21


<210> 763
<211> 23
<212> DNA
<213> Artificial Sequence

<220>
<221> source
<222> 1..23
<223> /mol_type="unassigned DNA"
      /note="Oligonucleotide forward primer designed on
      the basis of MSH6 gene sequence enabling amplification of a region
      comprising genetic mutation site c.457+52T>A"
      /organism="Artificial Sequence"

<400> 763
ctgcctttaa ggaaacttga cca                                              23


<210> 764
<211> 21
<212> DNA
<213> Artificial Sequence

<220>
<221> source
<222> 1..21
<223> /mol_type="unassigned DNA"
      /note="Oligonucleotide reverse primer designed
      on the basis of MSH6 gene sequence enabling amplification
      of a region comprising genetic mutation site c.457+52T>A"
      /organism="Artificial Sequence"

<400> 764
ccaccccttg caatttactg g                                                21


<210> 765
<211> 21
<212> DNA
<213> Artificial Sequence

<220>
<221> source
<222> 1..21
<223> /mol_type="unassigned DNA"
      /note="Oligonucleotide forward primer designed on
      the basis of MSH6 gene sequence enabling amplification of a region
      comprising genetic mutation site c.467C>G"
      /organism="Artificial Sequence"

<400> 765
agatggggtt tgctatgttg c                                                21


<210> 766
<211> 21
<212> DNA
<213> Artificial Sequence

<220>
<221> source
<222> 1..21
<223> /mol_type="unassigned DNA"
      /note="Oligonucleotide reverse primer designed
      on the basis of MSH6 gene sequence enabling amplification
      of a region comprising genetic mutation site c.467C>G"
      /organism="Artificial Sequence"

<400> 766
cccacctcca tctcttcttc c                                                21

<210> 767
<211> 21
<212> DNA
<213> Artificial Sequence

<220>
<221> source
<222> 1..21
<223> /mol_type="unassigned DNA"
      /note="Oligonucleotide forward primer designed on
      the basis of MSH6 gene sequence enabling amplification of a region
      comprising genetic mutation site c.540T>C"
      /organism="Artificial Sequence"

<400> 767
agatggggtt tgctatgttg c                                                21


<210> 768
<211> 21
<212> DNA
<213> Artificial Sequence

<220>
<221> source
<222> 1..21
<223> /mol_type="unassigned DNA"
      /note="Oligonucleotide reverse primer designed
      on the basis of MSH6 gene sequence enabling amplification
      of a region comprising genetic mutation site c.540T>C"
      /organism="Artificial Sequence"

<400> 768
cccacctcca tctcttcttc c                                                21


<210> 769
<211> 21
<212> DNA
<213> Artificial Sequence

<220>
<221> source
<222> 1..21
<223> /mol_type="unassigned DNA"
      /note="Oligonucleotide forward primer designed on
      the basis of MSH6 gene sequence enabling amplification of a region
      comprising genetic mutation site c.642C>T"
      /organism="Artificial Sequence"

<400> 769
gaacagccct gtcaaagttg c                                                21


<210> 770
<211> 21
<212> DNA

<213> Artificial Sequence

<220>
<221> source
<222> 1..21
<223> /mol_type="unassigned DNA"
      /note="Oligonucleotide reverse primer designed
      on the basis of MSH6 gene sequence enabling amplification
      of a region comprising genetic mutation site c.642C>T"
      /organism="Artificial Sequence"

<400> 770
gcatccatgt ggtacagctc a                                                21


<210> 771
<211> 21
<212> DNA
<213> Artificial Sequence

<220>
<221> source
<222> 1..21
<223> /mol_type="unassigned DNA"
      /note="Oligonucleotide forward primer designed on
      the basis of MSH6 gene sequence enabling amplification of a region
      comprising genetic mutation site c.1186C>G"
      /organism="Artificial Sequence"

<400> 771
gaacagccct gtcaaagttg c                                                21


<210> 772
<211> 21
<212> DNA
<213> Artificial Sequence

<220>
<221> source
<222> 1..21
<223> /mol_type="unassigned DNA"
      /note="Oligonucleotide reverse primer designed
      on the basis of MSH6 gene sequence enabling amplification
      of a region comprising genetic mutation site c.1186C>G"
      /organism="Artificial Sequence"

<400> 772
gcatccatgt ggtacagctc a                                                21


<210> 773
<211> 21
<212> DNA
<213> Artificial Sequence

<220>
<221> source

<222> 1..21
<223> /mol_type="unassigned DNA"
      /note="Oligonucleotide forward primer designed on
      the basis of MSH6 gene sequence enabling amplification of a region
      comprising genetic mutation site c.1508C>G"
      /organism="Artificial Sequence"

<400> 773
tgagctgtac cacatggatg c                                                21


<210> 774
<211> 21
<212> DNA
<213> Artificial Sequence

<220>
<221> source
<222> 1..21
<223> /mol_type="unassigned DNA"
      /note="Oligonucleotide reverse primer designed
      on the basis of MSH6 gene sequence enabling amplification
      of a region comprising genetic mutation site c.1508C>G"
      /organism="Artificial Sequence"

<400> 774
caccccaatg ccatcactta g                                                21

<210> 775
<211> 21
<212> DNA
<213> Artificial Sequence

<220>
<221> source
<222> 1..21
<223> /mol_type="unassigned DNA"
      /note="Oligonucleotide forward primer designed on
      the basis of MSH6 gene sequence enabling amplification of a region
      comprising genetic mutation site c.1784delT"
      /organism="Artificial Sequence"

<400> 775
tgagctgtac cacatggatg c                                                21


<210> 776
<211> 21
<212> DNA
<213> Artificial Sequence

<220>
<221> source
<222> 1..21
<223> /mol_type="unassigned DNA"
      /note="Oligonucleotide reverse primer designed
      on the basis of MSH6 gene sequence enabling amplification
      of a region comprising genetic mutation site c.1784delT"

/organism="Artificial Sequence"

<400> 776
caccccaatg ccatcactta g                                                21


<210> 777
<211> 21
<212> DNA
<213> Artificial Sequence

<220>
<221> source
<222> 1..21
<223> /mol_type="unassigned DNA"
      /note="Oligonucleotide forward primer designed on
      the basis of MSH6 gene sequence enabling amplification of a region
      comprising genetic mutation site c.1787delT"
      /organism="Artificial Sequence"

<400> 777
tgagctgtac cacatggatg c                                                21


<210> 778
<211> 21
<212> DNA
<213> Artificial Sequence

<220>
<221> source
<222> 1..21
<223> /mol_type="unassigned DNA"
      /note="Oligonucleotide reverse primer designed
      on the basis of MSH6 gene sequence enabling amplification
      of a region comprising genetic mutation site c.1787delT"
      /organism="Artificial Sequence"

<400> 778
caccccaatg ccatcactta g                                                21


<210> 779
<211> 21
<212> DNA
<213> Artificial Sequence

<220>
<221> source
<222> 1..21
<223> /mol_type="unassigned DNA"
      /note="Oligonucleotide forward primer designed on
      the basis of MSH6 gene sequence enabling amplification of a region
      comprising genetic mutation site c.3261delC"
      /organism="Artificial Sequence"

<400> 779
tgataaaacc cccaaacgat g                                                21

<210> 780
<211> 27
<212> DNA
<213> Artificial Sequence

<220>
<221> source
<222> 1..27
<223> /mol_type="unassigned DNA"
      /note="Oligonucleotide reverse primer designed
      on the basis of MSH6 gene sequence enabling amplification
      of a region comprising genetic mutation site c.3261delC"
      /organism="Artificial Sequence"

<400> 780
tgtttggaaa atgatcacct aagtatg                                          27


<210> 781
<211> 21
<212> DNA
<213> Artificial Sequence

<220>
<221> source
<222> 1..21
<223> /mol_type="unassigned DNA"
      /note="Oligonucleotide forward primer designed on
      the basis of MSH6 gene sequence enabling amplification of a region
      comprising genetic mutation site c.3438+14A>T"
      /organism="Artificial Sequence"

<400> 781
tgataaaacc cccaaacgat g                                                21


<210> 782
<211> 27
<212> DNA
<213> Artificial Sequence

<220>
<221> source
<222> 1..27
<223> /mol_type="unassigned DNA"
      /note="Oligonucleotide reverse primer designed
      on the basis of MSH6 gene sequence enabling amplification
      of a region comprising genetic mutation site c.3438+14A>T"
      /organism="Artificial Sequence"

<400> 782
tgtttggaaa atgatcacct aagtatg                                          27


<210> 783
<211> 24

<212> DNA
<213> Artificial Sequence

<220>
<221> source
<222> 1..24
<223> /mol_type="unassigned DNA"
      /note="Oligonucleotide forward primer designed on
      the basis of MSH6 gene sequence enabling amplification of a region
      comprising genetic mutation site c.3514dupA"
      /organism="Artificial Sequence"

<400> 783
ccagtcataa aagacctttt cctc                                              24


<210> 784
<211> 27
<212> DNA
<213> Artificial Sequence

<220>
<221> source
<222> 1..27
<223> /mol_type="unassigned DNA"
      /note="Oligonucleotide reverse primer designed
      on the basis of MSH6 gene sequence enabling amplification
      of a region comprising genetic mutation site c.3514dupA"
      /organism="Artificial Sequence"

<400> 784
tcacatctaa atgactgaat gagaact                                           27


<210> 785
<211> 21
<212> DNA
<213> Artificial Sequence

<220>
<221> source
<222> 1..21
<223> /mol_type="unassigned DNA"
      /note="Oligonucleotide forward primer designed on
      the basis of MSH6 gene sequence enabling amplification of a region
      comprising genetic mutation site c.3838C>T"
      /organism="Artificial Sequence"

<400> 785
accgatgttg cttttctgtc c                                                 21


<210> 786
<211> 22
<212> DNA
<213> Artificial Sequence

<220>

<221> source
<222> 1..22
<223> /mol_type="unassigned DNA"
      /note="Oligonucleotide reverse primer designed
      on the basis of MSH6 gene sequence enabling amplification
      of a region comprising genetic mutation site c.3838C>T"
      /organism="Artificial Sequence"

<400> 786
   tcatcccttc cccttttact gt                                                22


<210> 787
<211> 21
<212> DNA
<213> Artificial Sequence

<220>
<221> source
<222> 1..21
<223> /mol_type="unassigned DNA"
      /note="Oligonucleotide forward primer designed on
      the basis of NOD2 gene sequence enabling amplification of a region
      comprising genetic mutation site c.3020insC"
      /organism="Artificial Sequence"

<400> 787
tctaagggac aggtgggctt c                                                21


<210> 788
<211> 21
<212> DNA
<213> Artificial Sequence

<220>
<221> source
<222> 1..21
<223> /mol_type="unassigned DNA"
      /note="Oligonucleotide reverse primer designed
      on the basis of NOD2 gene sequence enabling amplification
      of a region comprising genetic mutation site c.3020insC"
      /organism="Artificial Sequence"

<400> 788
ccagacttcc aggatggtgt c                                                21


<210> 789
<211> 19
<212> DNA
<213> Artificial Sequence

<220>
<221> source
<222> 1..19
<223> /mol_type="unassigned DNA"
      /note="Oligonucleotide forward primer designed on

on the basis of CDKN2A gene sequence enabling amplification
of a region comprising genetic mutation site p.A148T"
/organism="Artificial Sequence"

<400> 789
cttcctggac acgctggtg                                                    19


<210> 790
<211> 19
<212> DNA
<213> Artificial Sequence

<220>
<221> source
<222> 1..19
<223> /mol_type="unassigned DNA"
/note="Oligonucleotide reverse primer designed
na on the basis of CDKN2A gene sequence enabling amplification
of a region comprising genetic mutation site p.A148T"
/organism="Artificial Sequence"

<400> 790
acgtgcgcaa gtccatttc                                                    19


<210> 791
<211> 19
<212> DNA
<213> Artificial Sequence

<220>
<221> source
<222> 1..19
<223> /mol_type="unassigned DNA"
/note="Oligonucleotide forward primer designed on
on the basis of CYP1B1 gene sequence enabling amplification
of a region comprising genetic mutation site p.A119S"
/organism="Artificial Sequence"

<400> 791
gtggccactg atcggaaac                                                    19


<210> 792
<211> 19
<212> DNA
<213> Artificial Sequence

<220>
<221> source
<222> 1..19
<223> /mol_type="unassigned DNA"
/note="Oligonucleotide reverse primer designed
na on the basis of CYP1B1 gene sequence enabling amplification
of a region comprising genetic mutation site p.A119S"
/organism="Artificial Sequence"

<400> 792
agggcatcac gtccaccag                                                    19


<210> 793
<211> 24
<212> DNA
<213> Artificial Sequence

<220>
<221> source
<222> 1..24
<223> /mol_type="unassigned DNA"
      /note="Oligonucleotide forward primer designed on
      the basis of CHEK2 gene sequence enabling amplification of a region
      comprising genetic mutation site c.1100delC"
      /organism="Artificial Sequence"

<400> 793
gattgccttc ttaggctatt ttcc                                              24


<210> 794
<211> 21
<212> DNA
<213> Artificial Sequence

<220>
<221> source
<222> 1..21
<223> /mol_type="unassigned DNA"
      /note="Oligonucleotide reverse primer designed
      on the basis of CHEK2 gene sequence enabling amplification
      of a region comprising genetic mutation site c.1100delC"
      /organism="Artificial Sequence"

<400> 794
tattcaaagg acggcgtttt c                                                 21


<210> 795
<211> 26
<212> DNA
<213> Artificial Sequence

<220>
<221> source
<222> 1..26
<223> /mol_type="unassigned DNA"
      /note="Oligonucleotide forward primer designed on
      the basis of CHEK2 gene sequence enabling amplification of a region
      comprising genetic mutation site p.IVS2+1G>A"
      /organism="Artificial Sequence"

<400> 795
caatagaaac tgatctagcc tacgtg                                            26

<210> 796
<211> 24
<212> DNA
<213> Artificial Sequence

<220>
<221> source
<222> 1..24
<223> /mol_type="unassigned DNA"
      /note="Oligonucleotide reverse primer designed
      on the basis of CHEK2 gene sequence enabling amplification
      of a region comprising genetic mutation site p.IVS2+1G>A"
      /organism="Artificial Sequence"

<400> 796
tcccaacaga aacaagaact tcag                                              24